(19) Europäisches Patentamt
European Patent Office
Office européen des brevets

(11) EP 1 266 888 A1

(12) **EUROPEAN PATENT APPLICATION**
published in accordance with Art. 158(3) EPC

(43) Date of publication:
**18.12.2002 Bulletin 2002/51**

(21) Application number: **01914178.7**

(22) Date of filing: **19.03.2001**

(51) Int Cl.[7]: **C07D 215/22**, C07D 215/14, A61K 31/47, A61P 43/00, A61P 3/10, A61P 3/06, A61P 9/10

(86) International application number:
**PCT/JP01/02168**

(87) International publication number:
**WO 01/070698 (27.09.2001 Gazette 2001/39)**

(84) Designated Contracting States:
**AT BE CH CY DE DK ES FI FR GB GR IE IT LI LU MC NL PT SE TR**

(30) Priority: **21.03.2000 JP 2000079146**

(71) Applicant: **Mitsui Chemicals, Inc.**
**Tokyo 100-6070 (JP)**

(72) Inventors:
- **TSUNODA, Hidetoshi**
  **Mobara-shi Chiba 297-0017 (JP)**
- **FUKAZAWA, Nobuyuki**
  **Chiyoda-ku Tokyo 100-6070 (JP)**
- **NAGASE, Hiroshi**
  **Mobara-shi Chiba 297-0017 (JP)**
- **CHIBA, Kyoko**
  **Mobara-shi Chiba 297-0017 (JP)**
- **NAKAO, Toshifumi**
  **Mobara-shi Chiba 297-0017 (JP)**
- **ASADA, Noriaki**
  **Mobara-shi Chiba 297-0017 (JP)**
- **YAMAKI, Toshifumi**
  **Mobara-shi Chiba 297-0017 (JP)**
- **KIBAYASHI, Kenji**
  **Suita-shi, Osaka 565-0836 (JP)**
- **MIGITA, Hideyuki**
  **Mobara-shi, Chiba 297-0017 (JP)**
- **MORIKAWA, Maki**
  **Mobara-shi, Chiba 297-0012 (JP)**

(74) Representative: **Stuart, Ian Alexander et al**
**MEWBURN ELLIS**
**York House**
**23 Kingsway**
**London WC2B 6HP (GB)**

(54) **QUINOLINE DERIVATIVES AND MEDICINAL USE THEREOF**

(57) A quinoline derivative represented by the following formula (1):

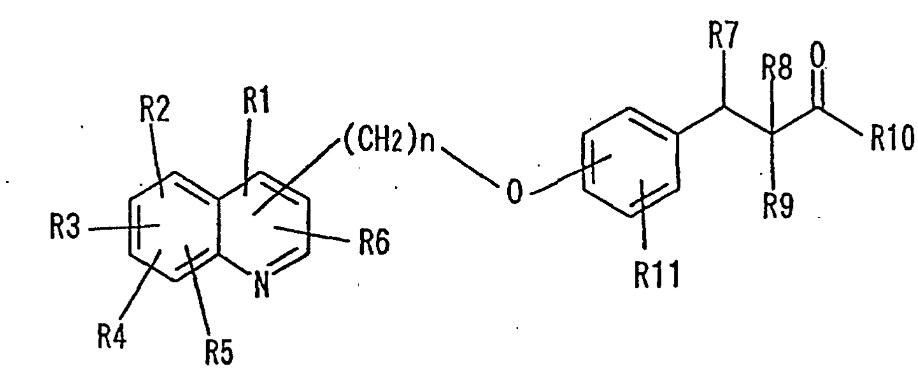

(1)

allows PPARα or γ which is an intranuclear transcription factor, to function strongly and is low in toxicity. By using this compound as an active ingredient, there can be provided a preventive or therapeutic agent for various diseases related to PPARα or γ.

EP 1 266 888 A1

## Description

Technical Field

The present invention relates to a preventive or therapeutic agent for various diseases, which shows a pharmacological action to various cells in a living body, either by activating a peroxisome proliferator-activated receptor α or γ (hereinafter referred to as PPARα or γ) as an intranuclear transcription factor; or by controlling the production of a tumor necrosis factor α (hereinafter referred to as TNFα).

Background Art

Diabetes patients are increasing owing to a recent change in customs of living; and the number of patients in Japan is said to be about 7 millions and, when persons thought to be contacting diabetes are included, be 13 millions or more [according to the recent report by the diabetes survey group of the Welfare Ministry, about 10% of the Japanese people of 40 years or older are suffering from diabetes. Progress in Diabetic Medicine 1996 (Vol. 30), Shindan-To-Chiryo Sha, Tokyo, p25, 1996]. The above trend does not change over the whole world, and the countermeasure therefor is an urgent social task in view of the arrival of future advanced-age society.

The state of diabetes is said to be a state of persistent hyperglycemia caused by absolute or relative shortage of insulin action. This persistent hyperglycemia causes various chronic complications such as renal failure, retinopathy, neuropathy and the like, making the morbid states based on the diabetes complicated and serious (Diabetes Mellitus Metabolism, Vol. 36, Suppl. 1, p22, 1987). As a countermeasure therefor, it is important to develop an agent which improves glycometabolism and inhibits the state of persistent hyperglycemia. As the morbid state of diabetes, there are two types, that is, an insulin-dependent type (type 1) and an insulin-independent type (type 2). In Japan, diabetes is mostly a type 2 diabetes, that is, an insulin-independent diabetes. As the cause of the type 2 diabetes, insulin resistance and insulin secretion insufficiency are known; and investigation on the therapeutic agent for the type 2 diabetes is being made based on these two causes.

For the insulin secretion insufficiency, there have been widely used an insulin treatment and long-known sulfonyl urea (SU) agents such as Tolbutamide, Acetohexamide, Glibenclamide and the like (Oral Hypoglycemic Agents, N. Engl. J. Meg., Vol. 321, p1231, 1989). The SU agents have a strong action for blood sugar reduction but have a risk of hypoglycemia as a serious side effect (Diabetic Med., Vol. 5, p315, 1988); therefore, they need be used with care. Further, the long-term use of the SU agents has problems such as promotion of obesity (Curr. Opin. Nephrol. Hypertens., Vol. 1, p291, 1992), secondary vitiation and the like.

For the insulin resistance, there have heretofore been used biguanide agents such as Fenformin, Metformin and the like. These biguanide agents have, for example, problems of insufficiency in blood sugar-reducing action and tendency of inducing serious lactose-acidosis (Diabetic Med., Vol. 5, p315, 1988; Practice, Vol. 13, p331, 1996), and are considered to be agents which need be treated with care in clinical use.

In order to solve these problems, there have been clinically applied, in recent years, several agents having a thiazolidinedione skeleton, as a new agent for ameliorating insulin resistance (agents such as Troglitazone, Pioglitazone and the like; JP-A-55-22636, JP-A-60-51189, JP-A-6-157522, etc.); besides the above thiazolidinedione type agents, there are being developed isoxazole ring-containing compounds (WO 95/18125), phenylpropionic acid derivatives (WO 93/21166, WO 96/04260, JP-A-11-158144), malonic acid derivatives (JP-A-9-323982), tyrosine derivatives (JP-A-8-325263), etc. These agents, however, are not sufficiently satisfactory in action, and are anxieties in their uses such as toxicity to kidney, side effects to circulating system, etc. and the like (Lancet., Vol. 350, p1748, 1997).

In addition, in recent years, a tumor necrosis factor α (TNFα) produced by lipocytes, a free fatty acid or neutral fat in blood, etc. have come to be regarded as important as a substance causing insulin resistance (Prostaglandins Leukotriens Essent. Fatty Acid, Vol. 53, p385, 1995); and a thought is developing that it is necessary to develop a more efficient agent for ameliorating insulin resistance based on a wider mechanism.

Heretofore, however, there has been no technique for simply determining the TNFα produced by lipocytes; and there has been known substantially no compound which can efficiently inhibit the production of TNFα from lipocytes.

Meanwhile, up to now, PPARα, PPARβ (δ), PPARγ, etc. are known as sub types of PPAR (Latruffe N. and Vamecq J., Biochemie, Vol. 79, p81, 1997). In recent years, the agent for activating PPARα has come to be considered to promote mainly lipid metabolism and show a blood lipid-reducing action. For example, Fibrate type agents (e.g. Clofibrate and Bezafibrate) for curing hyperlipemia, which are already being applied clinically, have a PPARα-activating action. Although this action is weak, this action is considered to be one of the mechanisms for expression of pharmacological action. Also, it is considered that part of the blood sugar-reducing action of the above-mentioned agents improving insulin resistance (e.g. thiazolidine type agents) might be derived from the PPAR γ-activating action. Thus, it is becoming clear in recent years that PPAR has an important role for glycometabolism or lipid metabolism in a living body.

Further, both PPARα and PPARγ have come to be known to have an influence on a wide range of phlocytes in addition to their known influence on lipid metabolism and glycometabolism (Igaku No Ayumi, Vol. 190, No. 10, p928, 1999); and their application to a new antiinflammatory agent based on those novel mechanisms is expected.

Thus, PPARα or γ-activating agents are expected as a preventive or therapeutic agent for many diseases mentioned previously. However, the agents known heretofore show, for example, no sufficient activation regardless of whether they are used singly to PPAR sub types (PPARα and PPARγ); therefore, they have had inconveniences, for example, in that they are not fully effective or are effective only to limited patients. Further, they have many problems as a drug, in toxicity, phamacokinetics of drug, etc.; and it is desired to develop a preventive or therapeutic agent for the above-mentioned diseases, which is more effective and can be applied to wider patients.

Meanwhile, some quinoline derivatives are already being clinically applied or clinically developed as a medicine. Examples of such a medicine being clinically applied include Saquinavir methanesulfonate (Roche) as an antiviral agent, mefloquine hydrochloride (Roche) as an antimalarial agent, and sodium salt of Montelukast (Merck) as an antiallergic agent. However, no preventive or therapeutic agent has heretofore been known which can activate PPARα or γ or inhibit the production of TNFα produced by lipocytes and which is useful to diabetes or its complications, hyperlipemia, arteriosclerosis, rheumatoid arthritis, osteoarthritis, asthma, bronchitis, allergic diseases, inflammatory splanchnopathy, ulcerative colitis, Crohn's disease, sepsis, septic shock, Kepla tuberculosis, multiple sclerosis, ischemic angiopathies such as DIC and the like, cerebral malaria, hepatitis, cancer, autoimmune disease, cachexia which becomes a problem in cancer or viral diseases (e.g. AIDS), etc. Each of the quinoline derivatives shown in the Claims of the present invention is unknown yet and novel.

Disclosure of the Invention

The object of the present invention is to provide quinoline derivatives which activate PPARα or γ (an intranuclear transcription factor) or suppress the production of TNFα and thereby becomes a preventive or therapeutic agent against diabetes or its complications, hyperlipemia, arteriosclerosis, rheumatoid arthritis, osteoarthritis, asthma, bronchitis, allergic diseases, inflammatory splanchnopathy, ulcerative colitis, Crohn's disease, sepsis, septic shock, Kepla tuberculosis, multiple sclerosis, ischemic angiopathies such as DIC and the like, cerebral malaria, hepatitis, cancer, autoimmune disease, cachexia which becomes a problem in cancer or viral diseases (e.g. AIDS), etc.

In order to achieve the above object, the present inventors made an effort to find out a substance which shows, in various cell levels, a PPARα or γ-activating action, a TNF α production-inhibiting action in lipocytes or a glucose consumption-promoting activity and, in model animal levels of morbid states, a blood sugar-reducing action, a lipid-reducing action, etc. As a result, the present inventors found out the following matters concerning the group of the compounds represented by the formulas (1) or (2) of the present invention:

They have a strong action for PPARα or γ activation, a strong action for inhibition of TNFα production, or a strong activity for promotion of glucose consumption in various cells; and exhibit a strong action for blood sugar reduction or a strong action for lipid reduction to various animals. They have low toxicity. They are well absorbed when administered orally. They are therefore a very useful medicine.

These findings have led to the completion of the present invention.

The quinoline derivatives according to the present invention are the compounds represented by the following formula (1):

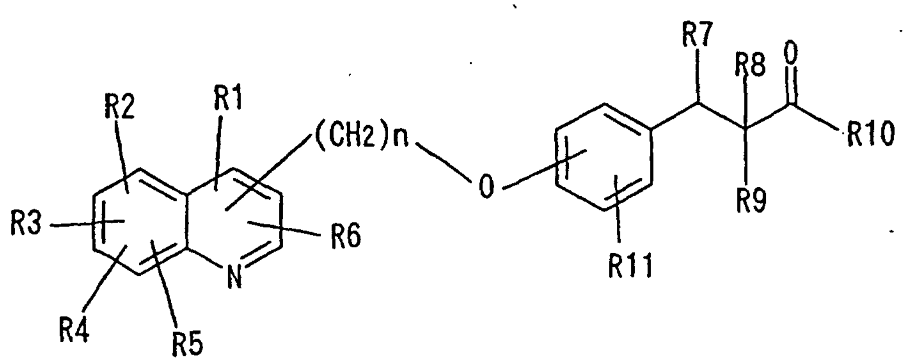

(1)

(wherein R1, R2, R3, R4, R5, R6 and R11 are each independently a hydrogen atom, a lower alkyl group of 1 to 4 carbon atoms, a halogen atom, a hydroxyl gorup, an alkyloxy group of 1 to 10 carbon atoms, a nitro group, an optionally substituted phenyl group, an amino group which may be substituted with a lower alkyl group of 1 to 4 carbon atoms,

a cyano group, a carboxyl group, a lower alkyloxycarbonyl group of 1 to 4 carbon atoms, an aminocarbonyl group which may be substituted with a lower alkyl group of 1 to 4 carbon atoms, or a trifluoromethyl group; R7 and R8 are a hydrogen atom or a lower alkyl group of 1 to 4 carbon atoms and may directly bond to each other to form a carbon-to-carbon double bond; R9 is a hydroxyl gorup, an alkyloxy group of 1 to 10 carbon atoms, an optionally substituted phenyloxy group, a thiol group, an alkylthiol group of 1 to 10 carbon atoms, an optionally substituted phenylthiol group, a lower alkylcarbonyl group of 1 to 4 carbon atoms, an optionally substituted benzoyl group, a lower alkyloxycarbonyl group of 1 to 4 carbon atoms, an aminocarbonyl group which may be substituted with a lower alkyl group of 1 to 4 carbon atoms, or a carboxyl group; R10 is a hydroxyl group, a lower alkyl group of 1 to 4 carbon atoms, a lower alkyloxy group of 1 to 4 carbon atoms, an optionally substituted phenyloxy group or an amino group which may be substituted with a lower alkyl group of 1 to 4 carbon atoms; and n is an integer of 1 to 4).

A preferred form of this quinoline derivative is a compound represented by the following formula (2):

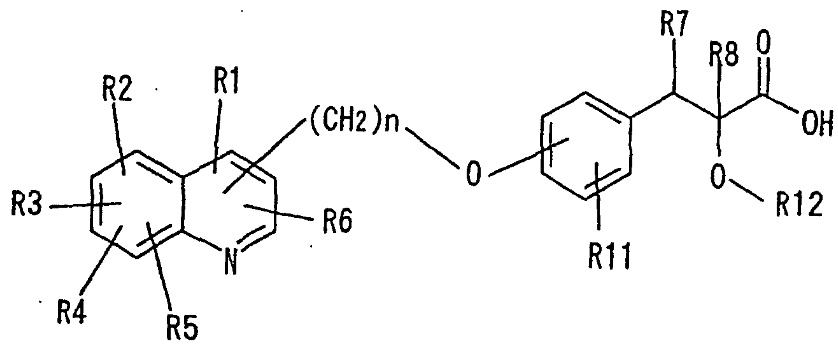

(2)

(wherein R1, R2, R3, R4, R5, R6, R7, R8 and R1 have the same definitions as in Claim 1; and R12 is a lower alkyl group of 1 to 4 carbon atoms or an optionally substituted phenyl group).

Other preferred form of the above quinoline derivative is a compound represented by the following formula (3):

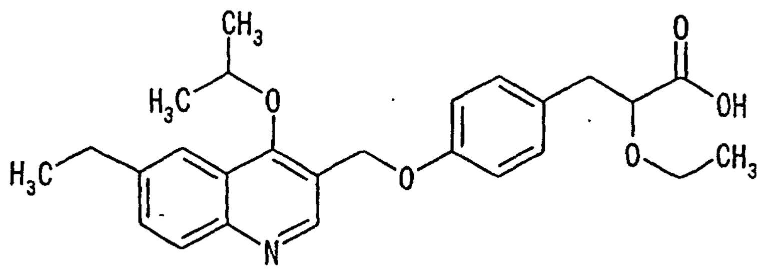

(3)

The pharmacologically acceptable salts of these quinoline derivatives are as well included in the present invention.

By using these quinoline derivatives and their pharmacologically acceptable salts as an active ingredient, there can be provided various medical compositions such as antagonist for peroxisome proliferator-activated receptor$\alpha$ or $\gamma$ which is an intranuclear transcription factor, inhibitor for production of tumor necrosis factor $\alpha$, preventive or therapeutic agent for diabetes, preventive or therapeutic agent for hyperlipemia, preventive or therapeutic agent for arterioscrelosis, and the like.

Best Mode for Carrying Out the Invention

The present invention is described in more detail below.

In the above formula (1) or (2), the lower alkyl group of 1 to 4 carbon atoms refers to methyl group, ethyl group, propyl group, isopropyl group, cyclopropyl group, butyl group, isobutyl group, tert-butyl group, cyclobutyl group or the like.

The halogen atom refers to fluorine atom, chlorine atom, bromine atom, iodine atom or the like.

The alkyloxy group of 1 to 10 carbon atoms refers to a straight chain, branched chain or cyclic alkyloxy group

such as methoxy group, ethoxy group, propoxy group, isopropoxy group, cyclopropoxy group, butoxy group, isobutoxy group, tert-butoxy group, cyclobutoxy group, pentyloxy group, hexyloxy group, heptyloxy group, octyloxy group, nonyloxy group, decyloxy group or the like.

The lower alkylcarbonyl group of 1 to 4 carbon atoms refers to acetyl group, propionyl group, butyryl group, isobutyryl group, valeryl group or the like.

The lower alkyloxycarbonyl group of 1 to 4 carbon atoms refers to methoxycarbonyl group, ethoxycarbonyl group, propoxycarbonyl group, isopropoxycarbonyl group, cyclopropoxycarbonyl group, butoxycarbonyl group, isobutoxycarbonyl group, tert-butoxycarbonyl group, cyclobutoxycarbonyl group or the like.

The pharmacologically acceptable salt refers to a salt of a base of a metal such as sodium, potassium, calcium or the like; a salt of an organic base such as pyridine, triethylamine, piperazine or the like; a salt of an inorganic acid such as hydrochloric acid, sulfuric acid, nitric acid or the like; or a salt of an organic acid such as acetic acid, fumaric acid, tartaric acid, malonic acid or the like.

The compound of the present invention includes those compounds having asymetric carbon atoms and being in the form of optical isomers. Therefore, all of these compounds are included in the present invention. Further, the present compound includes those compounds being in the form of geometrical isomers based on carbon-to-carbon unsaturated bonds. Therefore, all of these compounds are included in the present invention.

Description is made below on the synthesis processes of the compound of the present invention.

[Synthesis process 1]

A compound represented by the formula (1) can be synthesized by using a quinoline derivative represented by the above formula (3) and a phenol derivative represented by the following formula (4). Examples of the synthesis are shown in the following reaction schemes (1) and (2).

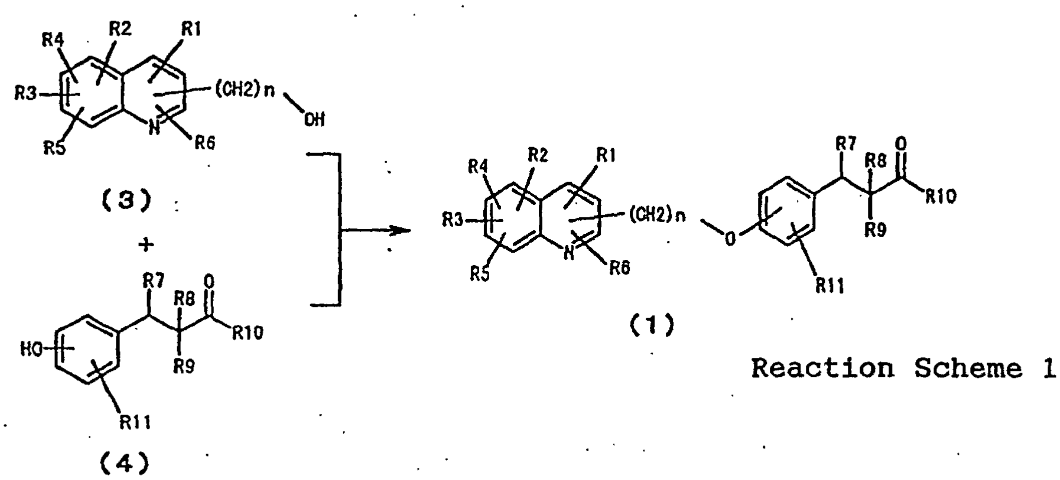

(3)

(4)

(1)

Reaction Scheme 1

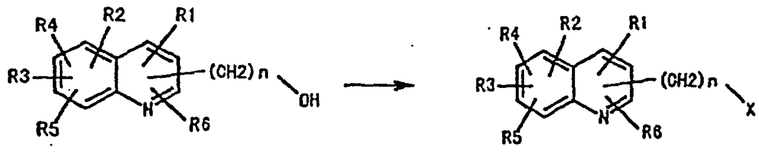

(3) (5)

(5)

+

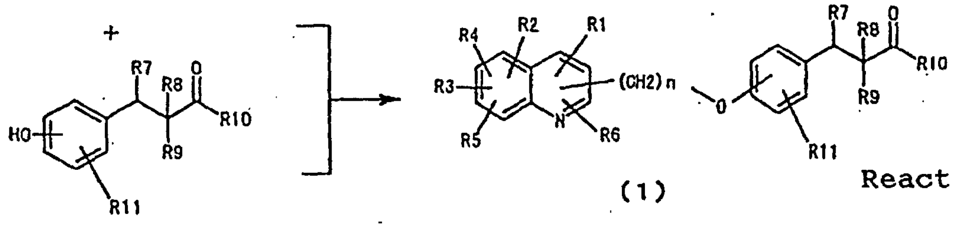

(4) (1)

Reaction Scheme 2

(In the above reaction schemes, R1, R2, R3, R4, R5, R6, R7, R8, R9, R10, R11 and n have the same definitions as given above; and X is a halogen atom or a substituted sulfonyloxy group.)

[I] The reaction scheme (1) is explained.

A quinoline alcohol derivative represented by the formula (3), obtained by a known or substantially known process is reacted with a phenol derivative represented by the formula (4), obtained by a known or substantially known process, in the presence of an alkyl or aryl phosphine or an azodicarboxylic acid derivative, whereby a compound represented by the formula (1) can be obtained. There is no particular restriction as to the usable alkyl or aryl phosphine, but it is exemplified by triphenylphosphine and tributylphosphine.

There is also no particular restriction as to the usable azodicarboxylic acid, but it is exemplified by diethylazodicarboxylic acid and diisopropylazodicarboxylic acid. There is no particular restriction as to the usable solvent, but it is exemplified by dichloromethane, chloroform, dichloroethane, tetrahydrofuran (hereinafter referred to as THF), dioxane, dimethylformamide (hereinafter referred to as DMF), dimethyl sulfoxide (hereinafter referred to as DMSO) and toluene. The reaction can be carried out in a temperature range of -100°C to the boiling point of the solvent used, preferably a temperature range of 0°C to the boiling point of the solvent.

[II] The reaction scheme (2) is explained.

A quinoline alcohol derivative represented by the formula (3) is converted into a halide represented by the formula (5) using a generally known agent for halogenation of alcohol or into a sulfonic acid ester compound represented by the formula (5) using a generally known agent for sulfonic acid esterification of alcohol; then, the compound of the formula (5) is reacted with a phenol derivative represented by the formula (4) under a basic condition; thereby, a compound represented by the formula (1) can be obtained.

There is no particular restriction as to the agent for halogenation of alcohol, and it is exemplified by hydrochloric acid, hydrobromic acid, thionyl chloride, sulfuryl chloride, phosphorus trichloride, phosphorus pentachloride, phosphorus oxychloride, thionyl bromide, phosphorus tribromide, methanesulfonyl chloride, and a mixed agent between triphenylphosphine and iodine, carbon tetrabromide, carbon tetrachloride or N-halogenated succinimide.

There is no particular restriction as to the usable agent for sulfonic acid esterification of alcohol. It is exemplified by methane sulfonic acid chloride, p-toluenesulfonic acid chloride and trifluoromethanesulfonic acid anhydride.

There is no particular restriction as to the base usable in the present reaction. It is exemplified by alkali or alkaline earth metals such as sodium, potassium, magnesium and the like; metal alkoxides such as sodium methoxide, sodium ethoxide, potassium butoxide and the like; inorganic bases such as sodium hydroxide, potassium hydroxide, sodium carbonate, potassium carbonate, cesium carbonate and the like; alkali metal amides such as lithium diisopropylamide and the like; and organic bases such as pyridine, triethylamine, 1,8-diazabicyclo-7-undecene (hereinafter referred to as DBU) and the like.

There is no particular restriction as to the usable solvent. It is exemplified by protic solvents such as water, methanol, ethanol, acetic acid and the like; and aprotic solvents such as dichloromethane, chloroform, dichloroethane, THF, dioxane, DMF, DMSO, toluene and the like.

The reaction can be carried out in a temperature range of -100°C to the boiling point of the solvent used, preferably in a temperature range of -20°C to the boiling point of the solvent.

[Synthesis process 2]

Alternatively, the compound represented by the formula (1) can also be synthesized by treating either a quinoline derivative represented by the formula (3), or a quinoline derivative represented by the formula (5) which can be obtained from the compound of the formula (3) by the method shown in the synthesis process 1, with a phenol derivative represented by the formula (6) in the same manner as in the ether linkage formation of the synthesis process 1, to obtain an aldehyde or ketone derivative represented by the formula (7) and then reacting the derivative of the formula (7) with a compound represented by the formula (8). An example of the synthesis process 2 is shown in the following reaction scheme (3).

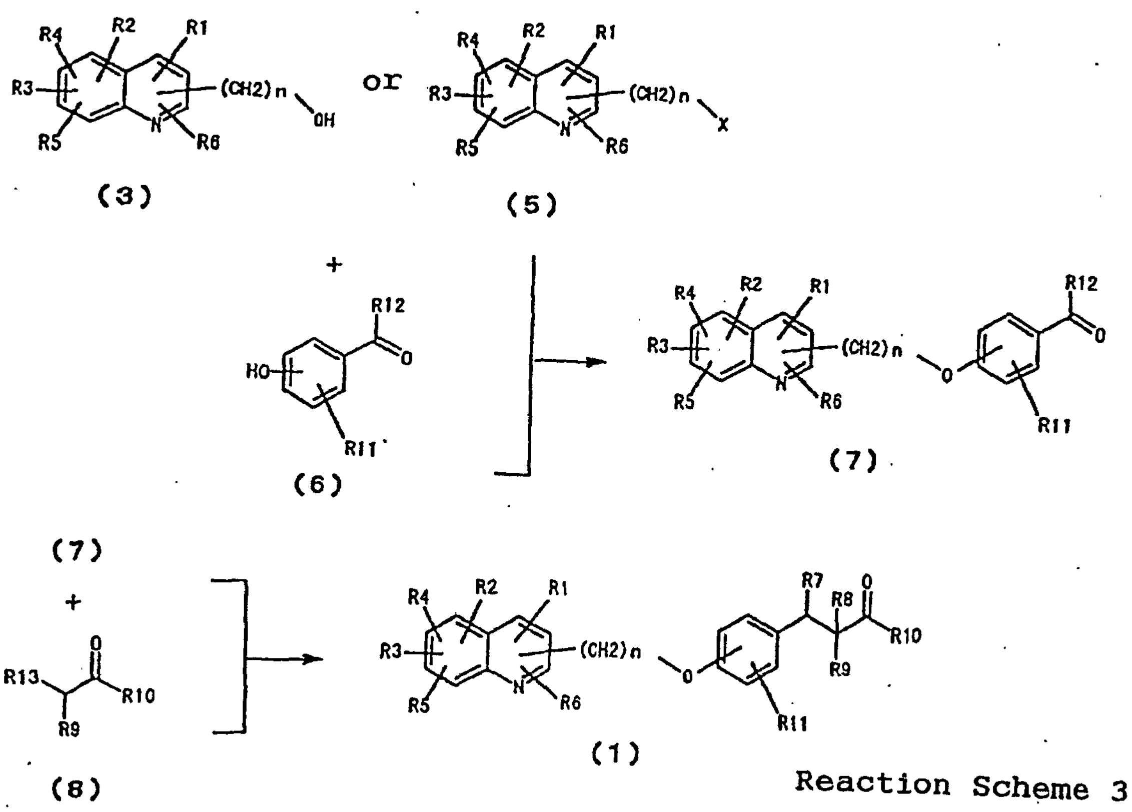
(3) or (5)
\+ (6)
(7)
(7) + (8)
(1)

Reaction Scheme 3

(In the above reaction scheme, R1, R2, R3, R4, R5, R6, R7, R8, R9, R10, R11, X and n have the same definitions as given above; R12 is a hydrogen atom or a lower alkyl group of 1 to 4 carbon atoms; and R13 a hydrogen atom or a lower alkyl group of 1 to 4 carbon atoms.)

The reaction scheme (3) is explained in more detail.

An aldehyde or ketone represented by the formula (7) is subjected to a dehydration and condensation reaction under a basic or acidic condition; thereby, an unsaturated derivative included in the formula (3) can be synthesized.

There is no particular restriction as to the base usable in the present synthesis. It is exemplified by alkali or alkaline earth metals such as sodium, potassium, magnesium and the like; metal alkoxides such as sodium methoxide, sodium ethoxide, potassium butoxide and the like; inorganic bases such as sodium hydroxide, potassium hydroxide, sodium carbonate, potassium carbonate, cesium carbonate and the like; alkali metal amides such as lithium diisopropylamide and the like; organic bases such as pyridine, piperidine, pyrrolidine, triethylamine, DBU and the like; and salts between such an organic base and acetic acid, sulfonic acid or the like.
There is no particular restriction, either, as to the usable acid. It is exemplified by inorganic acids such as hydrochloric acid, sulfuric acid, nitric acid and the like; organic protic acids such as acetic acid, methanesulfonic acid, p-toluenesulfonic acid, camphorsulfonic acid, trifluoroacetic acid and the like; and Lewis acids such as boron trifluoride and the like.
There is no particular restriction as to the usable solvent. It is exemplified by protic solvents such as water, methanol, ethanol, acetic acid and the like; and aprotic solvents such as dichloromethane, chloroform, dichloroethane, THF, dioxane, DMF, DMSO, toluene and the like.
The reaction can be carried out in a temperature range of -100°C to the boiling point of the solvent used.
Examples of the compounds included in the formula (1) or (2) are shown below. However, the compound of the present invention is not restricted thereto.

(1) 2-Hydroxy-3-{[4-(quinoline-3-yl)methyloxy]phenyl} propanoic acid
(2) 2-Methoxy-3-{[4-(quinoline-3-yl)methyloxylphenyl} propanoic acid
(3) 2-Ethoxy-3-{[4-(quinoline-3-yl)methyloxy]phenyl} propanoic acid
(4) 2-Propoxy-3-{[4-(quinoline-3-yl)methyloxy]phenyl} propanoic acid
(5) 2-Tert-butoxy-3-{[4-(quinoline-3-yl)methyloxy]phenyl} propanoic acid
(6) 2-Octyloxy-3-{[4-(quinoline-3-yl)methyloxy]phenyl} propanoic acid
(7) 2-Decyloxy-3-{[4-(quinoline-3-yl)methyloxy]phenyl} propanoic acid
(8) 2-Phenoxy-3-{[4-(quinoline-3-yl)methyloxy]phenyl} propanoic acid
(9) 2-[(4-Isopropxy)phenoxy]-3-{[4-(quinoline-3-yl) methyloxy]phenyl}propanoic acid
(10) 2-(4-Chlorophenoxy)-3-{[4-(quinoline-3-yl)methyloxy] phenyl}propanoic acid
(11) 2-Mercapto-3-{[4-(quinoline-3-yl)methyloxy]phenyl} propanoic acid
(12) 2-Ethylthio-3-{[4-(quinoline-3-yl)methyloxy] phenyl}propanoic acid
(13) 2-Decylthio-3-{[4-(quinoline-3-yl)methyloxy]phenyl} propanoic acid
(14) 2-Phenylthio-3-{[4-(quinoline-3-yl)methyloxy]phenyl} propanoic acid
(15) 2-[(4-Isopropoxy)phenylthio]-3-{[4-(quinoline-3-yl) methyloxy]phenyl}propanoic acid
(16) 2-(4-Chlorophenylthio)-3-{[4-(quinoline-3-yl) methyloxy]phenyl}propanoic acid
(17) 3-Oxo-2-{[4-(quinoline-3-yl)methyloxy]phenylmethyl} butanoic acid
(18) 3-Oxo-2-{[4-(quinoline-3-yl)methyloxy]phenylmethyl} pentanoic acid
(19) 3-Oxo-2-{[4-(quinoline-3-yl)methyloxy]phenylmethyl} heptanoic acid
(20) 3-Oxo-3-phenyl-2-{[4-(quinoline-3-yl)methyloxy] phenylmethyl}heptanoic acid
(21) 3-Oxo-3-(4-bromophenyl)-2-{[4-(quinoline-3-yl) methyloxy]phenylmethyl}heptanoic acid
(22) 3-Oxo-3-(2-ethylphenyl)-2-{[4-(quinoline-3-yl) methyloxy]phenylmethyl}heptanoic acid
(23) Monomethyl {[4-(quinoline-3-yl)methyloxy] phenyl}methylmalonate
(24) Monoethyl {[4-(quinoline-3-yl)methyloxy]phenyl} methylmalonate
(25) Monobutyl {[4-(quinoline-3-yl)methyloxy]phenyl} methylmalonate
(26) {[4-(Quinoline-3-yl)methyloxy]phenyl}methylmalonic acid mono-N-methylamide
(27) {[4-(Quinoline-3-yl)methyloxy]phenyl}methylmalonic acid mono-N,N-dibutylamide
(28) 2-Ethoxy-3-{[4-(quinoline-2-yl)methyloxy]phenyl} propanoic acid
(29) 2-Ethoxy-3-{[4-(quinoline-4-yl)methyloxy]phenyl} propanoic acid
(30) 2-Ethoxy-3-{[4-(quinoline-5-yl)methyloxy]phenyl} propanoic acid
(31) 2-Ethoxy-3-{[4-(quinoline-5-yl)methyloxy]phenyl} propanoic acid
(32) 2-Ethoxy-3-{[4-(quinoline-6-yl)methyloxy]phenyl} propanoic acid
(33) 2-Ethoxy-3-{[4-(quinoline-7-yl)methyloxy]phenyl} propanoic acid
(34) 2-Ethoxy-3-{[4-(quinoline-8-yl)methyloxy]phenyl} propanoic acid
(35) 2-Ethoxy-3-{[4-(quinoline-3-yl)ethyloxy]phenyl} propanoic acid
(36) 2-Ethoxy-3-{[4-(quinoline-3-yl)butyloxy]phenyl} propanoic acid
(37) 2-Ethoxy-2-methyl-3-{[4-(quinoline-3-yl)ethyloxy] phenyl}propanoic acid
(38) 2-Ethoxy-2-butyl-3-{[4-(quinoline-3-yl)ethyloxy] phenyl}propanoic acid
(39) 2-Ethoxy-3-{[4-(quinoline-3-yl)ethyloxy]phenyl} propenoic acid
(40) 3-{{[4-(Quinoline-3-yl)methyloxy]phenyl}methyl}-2,4-dioxopentane
(41) 5-{{[4-(Quinoline-3-yl)methyloxy]phenyl}methyl}-4,6-dioxodecane

(42) Methyl 2-ethoxy-3-{[4-(quinoline-3-yl)methyloxy] phenyl}propanoate
(43) Ethyl 2-ethoxy-3-{[4-(quinoline-3-yl)methyloxy] phenyl}propanoate
(44) Butyl 2-ethoxy-3-{[4-(quinoline-3-yl)methyloxy] phenyl}propanoate
(45) Phenyl 2-ethoxy-3-{[4-(quinoline-3-yl)methyloxy] phenyl}propanoate
(46) 4-Iodophenyl 2-ethoxy-3-{[4-(quinoline-3-yl) methyloxy]phenyl}propanoate
(47) 2-Ethoxy-3-{[4-(quinoline-3-yl)methyloxy]phenyl} propanoic acid amide
(48) 2-Ethoxy-3-{[4-(quinoline-3-yl)methyloxy]phenyl} propanoic acid N-methylamide
(49) 2-Ethoxy-3-{[4-(quinoline-3-yl)methyloxy]phenyl} propanoic acid N-butylamide
(50) 2-Ethoxy-3-{[4-(quinoline-3-yl)methyloxy]phenyl} propanoic acid N,N-dimethylamide
(51) 2-Ethoxy-3-{[3-(quinoline-3-yl)methyloxy]phenyl} propanoic acid
(52) 2-Ethoxy-3-{[2-(quinoline-3-yl)methyloxy]phenyl} propanoic acid
(53) 2-Ethoxy-3-{[4-methyl-[3-(quinoline-3-yl)methyloxy] phenyl}propanoic acid
(54) 2-Ethoxy-3-{[4-methyloxy-[3-(quinoline-3-yl) methyloxy]phenyl}propanoic acid
(55) 2-Ethoxy-3-{[4-chloro-[3-(quinoline-3-yl)methyloxy] phenyl}propanoic acid
(56) 2-Ethoxy-3-{[4-(4-methoxyquinoline-3-yl)methyloxy] phenyl}propanoic acid
(57) 2-Ethoxy-3-{[4-(4,6-dimethoxyquinoline-3-yl) methyloxy]phenyl}propanoic acid
(58) 2-Ethoxy-3-{[4-(4,6,7-trimethoxyquinoline-3-yl)methyloxy]phenyl}propanoic acid
(59) 2-Ethoxy-3-{[4-(4-ethoxy-6-methoxyquinoline-3-yl)methyloxy]phenyl}propanoic acid
(60) 2-Ethoxy-3-{[4-(4-propoxy-6-methoxyquinoline-3-yl)methyloxy]phenyl}propanoic acid
(61) 2-Ethoxy-3-{[4-(4-isopropoxy-6-methoxyquinoline-3-yl)methyloxy]phenyl}propanoic acid
(62) 2-Ethoxy-3-{[4-(4-heptyloxy-6-methoxyquinoline-3-yl)methyloxy]phenyl}propanoic acid
(63) 2-Ethoxy-3-{[4-(4-octyloxy-6-methoxyquinoline-3-yl)methyloxy]phenyl}propanoic acid
(64) 2-Ethoxy-3-{[4-(4-isopropoxy-7-methoxyquinoline-3-yl)methyloxy]phenyl}propanoic acid
(65) 2-Ethoxy-3-{[4-(4-isopropoxy-6,7-dimethoxyquinoline-3-yl)methyloxy]phenyl}propanoic acid
(66) 2-Ethoxy-3-{[4-(4-isopropoxy-6-methylquinoline-3-yl)methyloxy]phenyl}propanoic acid
(67) 2-Ethoxy-3-{[4-(4-isopropoxy-6-ethylquinoline-3-yl)methyloxy]phenyl}propanoic acid
(68) 2-Ethoxy-3-{[4-(4-decyloxy-6-methylquinoline-3-yl)methyloxy]phenyl}propanoic acid
(69) 2-Ethoxy-3-{[4-(4-isopropoxy-8-butylquinoline-3-yl)methyloxy]phenyl}propanoic acid
(70) 2-Ethoxy-3-{[4-(4-isopropoxy-5,6-dimethylquinoline-3-yl)methyloxy]phenyl}propanoic acid
(71) 2-Ethoxy-3-{[4-(4-isopropoxy-5,7-dimethylquinoline-3-yl)methyloxy]phenyl}propanoic acid
(72) 2-Ethoxy-3-{[4-(4-isopropoxy-2-chloroquinoline-3-yl)methyloxy]phenyl}propanoic acid
(73) 2-Ethoxy-3-{[4-(4-isopropoxy-5-chloroquinoline-3-yl)methyloxy]phenyl}propanoic acid
(74) 2-Ethoxy-3-{[4-(4-isopropoxy-6-chloroquinoline-3-yl)methyloxy]phenyl}propanoic acid
(75) 2-Ethoxy-3-{[4-(4-isopropoxy-7-chloroquinoline-3-yl)methyloxy]phenyl}propanoic acid
(76) 2-Ethoxy-3-{[4-(4-isopropoxy-8-chloroquinoline-3-yl)methyloxy]phenyl}propanoic acid
(77) 2-Ethoxy-3-{[4-(4-isopropoxy-2-bromoquinoline-3-yl)methyloxy]phenyl}propanoic acid
(78) 2-Ethoxy-3-{[4-(4-isopropoxy-6-bromoquinoline-3-yl)methyloxy]phenyl}propanoic acid
(79) 2-Ethoxy-3-{[4-(4-isopropoxy-8-bromoquinoline-3-yl)methyloxy]phenyl}propanoic acid
(80) 2-Ethoxy-3-{[4-(4-isopropoxy-2-fluoroquinoline-3-yl)methyloxy]phenyl}propanoic acid
(81) 2-Ethoxy-3-{[4-(4-isopropoxy-5-fluoroquinoline-3-yl)methyloxy]phenyl}propanoic acid
(82) 2-Ethoxy-3-{[4-(4-isopropoxy-8-fluoroquinoline-3-yl)methyloxy]phenyl}propanoic acid
(83) 2-Ethoxy-3-{[4-(4-isopropoxy-8-iodoquinoline-3-yl)methyloxy]phenyl}propanoic acid
(84) 2-Ethoxy-3-{[4-(4-isopropoxy-2-trifluoromethyl quinoline-3-yl)methyloxy]phenyl}propanoic acid
(85) 2-Ethoxy-3-{[4-(4-isopropoxy-7-trifluoromethyl quinoline-3-yl)methyloxy]phenyl}propanoic acid
(86) 2-Ethoxy-3-{[4-(4-isopropoxy-8-trifluoromethyl quinoline-3-yl)methyloxy]phenyl}propanoic acid
(87) 2-Ethoxy-3-{[4-(4-isopropoxy-2-hydroxyquinoline-3-yl)methyloxy]phenyl}propanoic acid
(88) 2-Ethoxy-3-{[4-(4-isopropoxy-2-acetamidoquinoline-3-yl)methyloxy]phenyl}propanoic acid
(89) 2-Ethoxy-3-{[4-(4-isopropoxy-5-nitroquinoline-3-yl)methyloxy]phenyl}propanoic acid
(90) 2-Ethoxy-3-{[4-(4-isopropoxy-5-cyanoquinoline-3-yl)methyloxy]phenyl}propanoic acid
(91) 2-Ethoxy-3-{[4-(4-isopropoxy-8-aminoquinoline-3-yl)methyloxy]phenyl}prppanoic acid
(92) 2-Ethoxy-3-{[4-(4-isopropoxy-8-N-ethylaminoquinoline -3-yl)methyloxy]phenyl}propanoic acid
(93) 2-Ethoxy-3-{[4-(4-isopropoxy-8-carboxyquinoline-3-yl)methyloxy]phenyl}propanoic acid
(94) 2-Ethoxy-3-{[4-(4-isopropoxy-8-ethoxycarbonyl quinoline-3-yl)methyloxy]phenyl}propanoic acid
(95) 2-Ethoxy-3-{[4-(4-isopropoxy-8-phenylquinoline-3-yl)methyloxy]phenyl}propanoic acid
(96) 2-Ethoxy-3-{[4-(2-phenylquinoline-3-yl)methyloxy] phenyl}propanoic acid
(97) 2-Ethoxy-3-{[4-(2-phenylquinoline-4-yl)methyloxy] phenyl}propanoic acid
(98) 2-Ethoxy-3-{[4-(2-methoxyquinoline-4-yl)methyloxy] phenyl}propanoic acid
(99) 2-Ethoxy-3-{[4-(2-methoxyquinoline-2-yl)methyloxy] phenyl}propanoic acid

(100) Methyl 2-ethoxy-3-{[4-(4-isopropoxy-6-methoxyquinoline-3-yl)methyloxy]phenyl}propanoate
(101) Ethyl 2-ethoxy-3-{[4-(4-isopropoxy-6-methoxyquinoline-3-yl)methyloxy]phenyl}propanoate
(102) 2-Ethoxy-3-{[4-(4-isopropoxy-6-methoxyquinoline-3-yl)methyloxy]phenyl}propanoic acid amide
(103) 2-Ethoxy-3-{[4-(4-isopropoxy-6-methoxyquinoline-3-yl)methyloxy]phenyl}propanoic acid N-ethylamide
(104) 2-Ethoxy-3-{[4-(4-isopropoxy-6-methoxyquinoline-3-yl)methyloxy]phenyl}propanoic acid N-butylamide
(105) 2-Ethoxy-3-{[4-(4-isopropoxy-6-methoxyquinoline-3-yl)methyloxy]phenyl}propanoic acid N,N-diethylamide
(106) 3-{[4-(4-Isopropoxy-6-methoxyquinoline-3-yl)methyloxy]phenyl}-2-phenoxypropanoic acid
(107) 3-{[4-(4-Isopropoxy-6-methoxyquinoline-3-yl)methyloxy]phenyl}-2-(4-chlorophenoxy)propanoic acid
(108) 3-{(4-(4-Isopropoxy-6-methoxyquinoline-3-yl)methyloxy]phenyl}-2-phenylthiopropanoic acid
(109) 3-{[4-(4-Isopropoxy-6-methoxyquinoline-3-yl)methyloxy]phenyl}-2-(4-chlorophenylthio)propanoic acid
(110) 3-{[4-(4-Isopropoxy-6-methoxyquinoline-3-yl)methyloxy]phenyl}-2-phenoxypropenoic acid
(111) 3-{[4-(4-Isopropoxy-6-methoxyquinoline-3-yl)methyloxy]phenyl}-2-(4-chlorophenoxy)propenoic acid
(112) 3-{[4-(4-Isopropoxy-6-methoxyquinoline-3-yl)methyloxy]phenyl}-2-phenylthiopropenoic acid
(113) 3-{[4-(4-Isopropoxy-6-methoxyquinoline-3-yl)methyloxy]phenyl}-2-(4-chlorophenylthio)propenoic acid
(114) 2-Ethoxy-3-{[3-(4-isopropoxy-6-methoxyquinoline-3-yl)methyloxy]phenyl}propanoic acid
(115) 2-Ethoxy-3-{[2-(4-isopropoxy-6-methoxyquinoline-3-yl)methyloxy]phenyl}propanoic acid
(116) 2-Ethoxy-3-{[3-(4-isopropoxy-6-methoxyquinoline-3-yl)methyloxy]-2-methylphenyl}propanoic acid
(117) 2-Ethoxy-3-{[3-(4-isopropoxy-6-methoxyquinoline-3-yl)methyloxy]-5-chlorophenyl}propanoic acid
(118) 2-Ethoxy-3-{[3-(4-isopropoxy-6-methoxyquinoline-3-yl)methyloxy]-4-methoxyhenyl}propanoic acid
(119) Monomethyl {[4-(4-isopropoxy-6-methoxyquinoline-3-yl)methyloxy]phenyl}methylmalonate
(120) Dimethyl {[4-(4-isopropoxy-6-methoxyquinoline-3-yl)methyloxy]phenyl}methylmalonate
(121) Monoethyl {[4-(4-isopropoxy-6-methoxyquinoline-3-yl)methyloxy]phenyl}methylmalonate
(122) Diethyl {[4-(4-isopropoxy-6-methoxyquinoline-3-yl)methyloxy]phenyl}methylmalonate
(123) Dipropyl {[4-(4-isopropoxy-6-methoxyquinoline-3-yl)methyloxy]phenyl}methylmalonate
(124) Dibutyl {[4-(4-isopropoxy-6-methoxyquinoline-3-yl)methyloxy]phenyl}methylmalonate
(125) Monomethyl {[2-(4-isopropoxy-6-methoxyquinoline-3-yl)methyloxy]phenyl}methylmalonate
(126) Dimethyl {[3-(4-isopropoxy-6-methoxyquinoline-3-yl)methyloxy]phenyl}methylmalonate
(127) Monoethyl {[3-(4-isopropoxy-6-methoxyquinoline-3-yl)methyloxy]phenyl}methylmalonate
(128) Diethyl {[3-(4-isopropoxy-6-methoxyquinoline-3-yl)methyloxy]phenyl}methylmalonate
(129) Diethyl {[3-(4-isopropoxy-6-methoxyquinoline-3-yl)methyloxy]-4-methoxyphenyl}methylmalonate
(130) Diethyl {[3-(4-isopropoxy-6-methoxyquinoline-3-yl)methyloxy]-6-methylphenyl}methylmalonate
(131) {[4-(4-Isopropoxy-6-methoxyquinoline-3-yl)methyloxy]phenyl}methylmalonic acid diamide
(132) {[4-(4-Isopropoxy-6-methoxyquinoline-3-yl) methyloxy]phenyl}methylmalonic acid N,N-dimethyldiamide
(133) 2-{[4-(4-Isopropoxy-6-methoxyquinoline-3-yl) methyloxy]phenylmethyl}-3-oxobutanoic acid
(134) Methyl 2-{[4-(4-isopropoxy-6-methoxyquinoline-3-yl)methyloxy]phenylmethyl}-3-oxobutanoate
(135) Ethyl 2-{[4-(4-isopropoxy-6-methoxyquinoline-3-yl)methyloxy]phenylmethyl}-3-oxobutanoate
(136) 2-{[4-(4-Isopropoxy-6-methoxyquinoline-3-yl)methyloxy]phenylmethyl}-3-oxopentanoic acid
(137) Methyl 2-{[4-(4-isopropoxy-6-methoxyquinoline-3-yl)methyloxy]phenylmethyl}-3-oxopentanoate
(138) Ethyl 2-{[4-(4-isopropoxy-6-methoxyquinoline-3-yl)methyloxy]phenylmethyl}-3-oxopentanoate
(139) Ethyl 2-{[4-(4-isopropoxy-6-methoxyquinoline-3-yl)methyloxy]phenylmethyl}-3-oxoheptanoic acid
(140) 2-{[4-(4-Isopropoxy-6-methoxyquinoline-3-yl)methyloxy]phenylmethyl}-3-oxobutanoic acid amide
(141) 2-{[4-(4-Isopropoxy-6-methoxyquinoline-3-yl)methyloxy]phenylmethyl}-3-oxobutanoic acid N-methylamide
(142) 2-{[4-(4-Isopropoxy-6-methoxyquinoline-3-yl)methyloxy]phenylmethyl}-3-oxobutanoic acid N,N-dibutylamide
(143) 2-{[4-(4-Isopropoxy-6-methoxyquinoline-3-yl)methyloxy]phenylmethyl}-3-oxobutanoic acid amide
(144) 2-{[4-(4-Isopropoxy-6-methoxyquinoline-3-yl)methyloxy]phenylmethylene}-3-oxobutanoic acid N-methylamide
(145) 2-{[4-(4-Isopropoxy-6-methoxyquinoline-3-yl)methyloxy]phenylmethylene}-3-oxobutanoic acid N,N-dibutylamide
(146) 3-{[4-(4-Isopropoxy-6-methoxyquinoline-3-yl)methyloxy]phenylmethyl}-2,4-dioxopentane
(147) 3-{[4-(4-Isopropoxy-6-methoxyquinoline-3-yl)methyloxy]phenylmethylene}-2,4-dioxopentane
(148) 4-{[4-(4-Isopropoxy-6-methoxyquinoline-3-yl)methyloxy]phenylmethyl}-3,5-dioxoheptane
(149) 4-{[4-(4-Isopropoxy-6-methoxyquinoline-3-yl)methyloxy]phenylmethylene}-3,5-dioxoheptane
(150) 5-{[4-(4-Isopropoxy-6-methoxyquinoline-3-yl)methyloxy]phenylmethylene}-4,6-dioxodecane

Some of the above compounds have asymetric carbon atoms and are in the form of optical isomers. Therefore, all of these isomers are included in the scope of the present invention.

Next, the compound of the present invention, when used as a medicine, can be administered orally or parenterally. The dose varies depending upon the disease condition, age, sex, etc. of a patient to which the compound is to be administered. However, the compound can be administered in a total amount of 1 to 1000 mg per adult, in one time or a plurality of times. In oral administration, the compound can be administered in the form of tablet(s), granule(s), powder, suspension, capsule(s), syrup or the like.

When the compound is made into, for example, tablets, it is possible to use, as an adsorbent, crystalline cellulose, light anhydrous silicic acid or the like; as an excipient, corn starch, lactose, calcium phosphate, magnesium stearate or the like; and, as necessary, a binder, a humectant, a lubricant, etc.

In parenteral administration, the present compound can be administered in the form of intravenous injection, subcutaneous injection, intramuscular injection, suppository, percutaneous agent or the like. When the compound is made into, for example, an injection, the compound can be used as an aqueous solution obtained by subjecting the compound to isotonization, sterilization, etc., or as an aqueous suspension obtained by using cottonseed oil, corn oil, olive oil or the like, or as an emulsion obtained by using a surfactant such as HCO-60 or the like.

The present invention is described in more detail below by way of Examples, Reference Examples and Test Examples. However, the present invention is in no way restricted by them.

[Example 1] Synthesis of ethyl 2-ethoxy-3-{[4-(4-isopropoxy-6-methoxyquinoline-3-yl)methyloxy]phenyl} propanoate: exemplary compound (101)

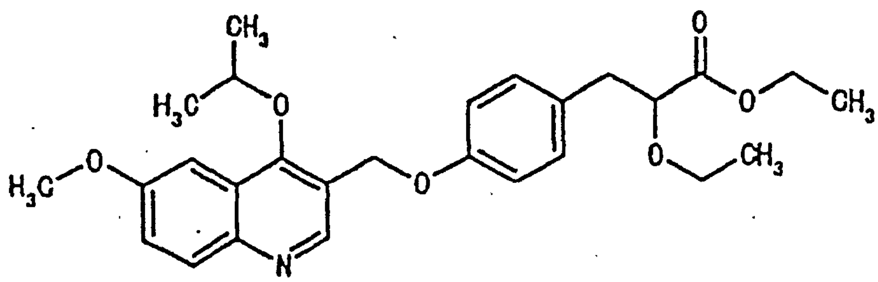

In methylene chloride (60 ml) were dissolved 4-isopropoxy-6-methoxyquinoline-3-methanol (1.98 g, 8 mmol) synthesized in Reference Example 4 and ethyl 2-ethoxy-3-(4-hydroxyphenyl)propanoate (1.91 g, 8 mmol). Thereto were added, at room temperature, triphenylphosphine (2.52 g, 9.6 mmol) and diethyl azodicarboxylic acid (4.18 g, 9.6 mmol), followed by stirring in that state for 2 hours. The reaction mixture was diluted with chloroform, followed by washing with water. The organic layer was dried over anhydrous magnesium sulfate and subjected to vacuum concentration. The residue was purified by silica gel column chromatography (equivalent product of Merck C-300: 150g, ethyl acetate : hexane =1 : 2) to obtain an intended title compound (2.4 g, 64%) as a colorless transparent syrup.
1H-M.M.R.(CDCl3, 270MHz)d=8.82(s, 1H), 7.98(d, 1H, J=8.0Hz), 7.40-7.30(m, 2H), 7.18 and 6.94(2d, each 2H, J=8.5Hz), 5.20(s, 2H), 4.58(quintet, 1H, J=6.5Hz), 4.21(q, 2H, J=7.3Hz), 3.96(t, 1H, J=7.3Hz), 3.95(s, 3H), 3.65-3.55 (m, 1H), 3.40-3.30(m, 1H), 2.95(d, 2H, J=7.3Hz), 1.40(d, 6H, J=6.5Hz), 1.35-1.15(m, 6H)

[Example 2] Synthesis of 2-ethoxy-3-{[4-(4-isopropoxy-6-methoxyquinoline-3-yl)methyloxy]phenyl}propanoic acid: exemplary compound (61)

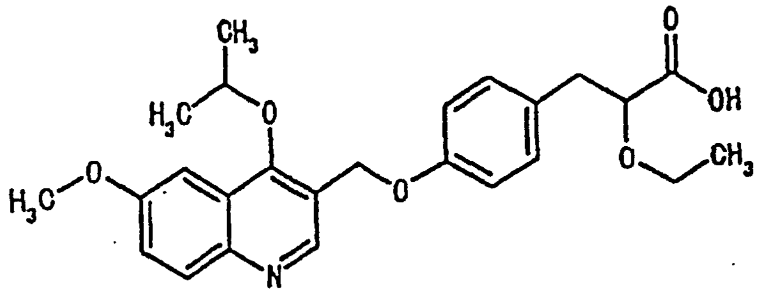

The ester compound (2.4 g, 5.13 mmol) obtained in Example 1 was dissolved in ethanol (40 ml). Thereto was added, at room temperature, a 2 N aqueous sodium hydroxide solution (3.85 ml, 7.70 mmol), followed by stirring at that temperature for 1 hour. The most part of ethanol was removed from the reaction mixture by vacuum distillation. The residue was dissolved in water (100 ml). The solution was made weakly acidic using 1 N hydrochloric acid, followed by extraction with ethyl acetate. The organic layer was dried over anhydrous magnesium sulfate and then subjected to vacuum concentration to obtain a colorless transparent syrup. The residue was subjected to crystallization using a mixed solvent consisting of ethyl acetate and hexane to obtain an intended title compound (2.10 g, 93%) as white crystals.

Melting point: 152 to 154°C
1H-N.M.R.(CDCl3, 270MHz)d=8.80(s, 1H), 8.07(d, 1H, J=9.0Hz), 7.40-7.30(m, 2H), 7.22 and 6.92(2d, each 2H, J=9.0Hz), 5.20(s, 2H), 4.60(quintet, 1H, J=6.5Hz), 4.08(dd, 1H, J=4.5, 7.3Hz), 3.95(s, 3H), 3.75-3.65(m, 1H), 3.50-3.40(m, 1H), 3.12(dd, 1H, J=4.5, 13.0Hz), 3.02(dd, 1H, J=7.3, 13.0Hz), 1.42(d, 6H, J=6.5Hz), 1.18(t, 3H, J=7.3Hz)

[Example 3] Synthesis of 2-ethoxy-3-{[4-(4-isopropoxy-6-methoxyquinoline-3-yl)methyloxy]phenyl}propanoic acid amide: exemplary compound (102)

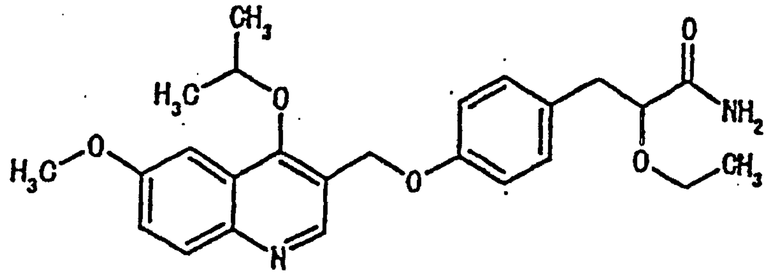

The carboxylic acid compound (150 mg, 0.341 mmol) obtained in Example 2 was dissolved in tetrahydrofuran (10 ml). Thereto was added 1,1-carbonylbisimidazole (110 mg, 0.682 mmol) at room temperature, followed by stirring in that state for 30 minutes. Successively, 28% ammonia water (2 ml) was added, followed by stirring for 30 minutes. The reaction mixture was diluted with water, and extraction of an intended compound was conducted using ethyl acetate. The organic layer was dried over anhydrous magnesium sulfate and then subjected to vacuum concentration to obtain a colorless transparent syrup. The residue was subjected to crystallization using a mixed solvent consisting of ethyl acetate and hexane, to obtain an intended title compound (125 mg, 84%) as a white amorphous solid.

1H-N.M.R.(CDCl3, 270MHz)d=8.82(s, 1H), 7.99(d, 1H, J=8.8Hz), 7.39-7.35(m, 2H), 7.19(d, 2H, J=8.8Hz), 6.93(d, 2H, J=8.8Hz), 6.48 and 5.58(2bs, each 1H), 5.21(s, 2H), 4.59(quintet, 1H, J=6.1Hz), 3.96(s, 3H), 3.90(dd, 1H, J=3.6, 7.6Hz), 3.53-3.42(m, 2H), 3.08(dd, 1H, J=3.6, 13.7Hz), 2.90(dd, 1H, J=7.6, 13.7Hz), 1.41(d, 6H, J=6.1Hz), 1.14(t, 3H, J=7.1Hz)

[Example 4] Synthesis of 2-ethoxy-3-{[4-(4-isopropoxy-6-methoxyquinoline-3-yl)methyloxy]phenyl}propanoic acid N-butylamide: exemplary compound (104)

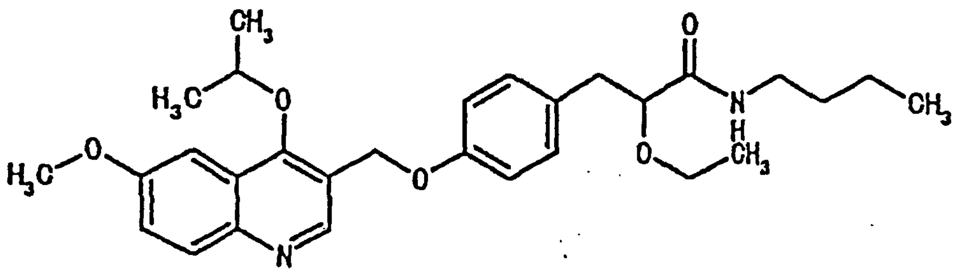

The carboxylic acid compound (150 mg, 0.341 mmol) obtained in Example 2 was dissolved in tetrahydrofuran (10 ml). Thereto was added 1,1-carbonylbisimidazole (110 mg, 0.682 mmol) at room temperature, followed by stirring in that state for 30 minutes. Successively, n-butylamine (150 mg, 2.05 mmol) was added, followed by stirring for 1 hour. The reaction mixture was diluted with water, and extraction of an intended compound was conducted using ethyl acetate. The organic layer was dried over anhydrous magnesium sulfate and then subjected to vacuum concentration. The residue was purified by silica gel column chromatography (equivalent product of Merck C-300: 20 g, ethyl acetate : hexane = 1 : 1) to obtain an intended title compound (135 mg, 80%) as white crystals.
Melting point: 66 to 68°C
1H-N.M.R.(CDCl3, 270MHz)d=8.82(s, 1H), 7.98(d, 1H, J=8.8Hz), 7.39-7.35(m, 2H), 7.17(d, 2H, J=8.3Hz), 6.92(d, 2H, J=8.3Hz), 6.50(t, 1H, J=5.9Hz), 5.21(s, 2H), 4.59(quintet, 1H, J=5.8Hz), 3.96(s, 3H), 3.90(dd, 1H, J=3.4, 7.3Hz), 3.50-3.41(m, 2H), 3.23-3.13(m, 2H), 3.09(dd, 1H, J=3.4, 14.1Hz), 2.85(dd, 1H, J=7.3, 14.1Hz), 1.50-1.35(m, 4H), 1.41 (d, 6H, J=5.8Hz), 1.14(t, 3H, J=6.8Hz), 0.91(t, 3H, J=6.8Hz)

[Example 5] Synthesis of 2-ethoxy-3-{[4-(4-isopropoxy-5,7-dimethylquinoline-3-yl)methyloxy]phenyl}propanoic acid: exemplary compound (71)

[Reaction 1] Synthesis of ethyl 2-ethoxy-3-{[4-(4-isopropoxy-5,7-dimethylquinoline-3-yl)methyloxylphenyl}propanoate

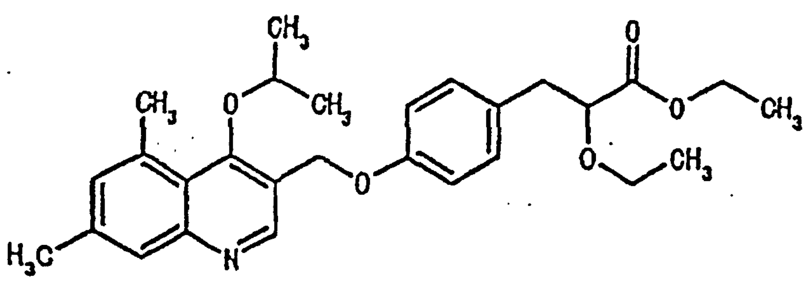

4-Isopropoxy-5,7-dimethylquinoline-3-methanol (756 mg, 3 mmol) synthesized in Reference Example 9 and ethyl 2-ethoxy-3-(4-hydroxy]phenyl)propanoate (715 mg, 3 mmol) synthesized in Reference Example 7 were subjected to the same treatment as in Example 1 to obtain an intended title compound (580 mg, 41%) as a colorless transparent syrup.
1H-N.M.R.(CDCl3, 270MHz)d=8.85(s, 1H), 7.70(s, 1H), 7.19 and 6.93(2d, each 2H, J=8.6Hz), 7.14(s, 1H), 5.16(s, 2H), 4.48(quintet, 1H, J=6.3Hz), 4.16(q, 2H, J=7.3Hz), 3.98(t, 1H, J=6.9Hz), 3.70-3.55(m, 1H), 3.40-3.30(m, 1H), 2.96(d, 1H, J=6.9Hz), 2.89 and 2.48(2s, each 3H), 1.30(d, 6H, J=6.3Hz), 1.21(t, 3H, J=7.3Hz), 1.17(t, 3H, J=7.3Hz)

[Reaction 2] Synthesis of 2-ethoxy-3-{[4-(4-isopropoxy-5,7-dimethylquinoline-3-yl)methyloxy]phenyl}propanoic acid: exemplary compound (71)

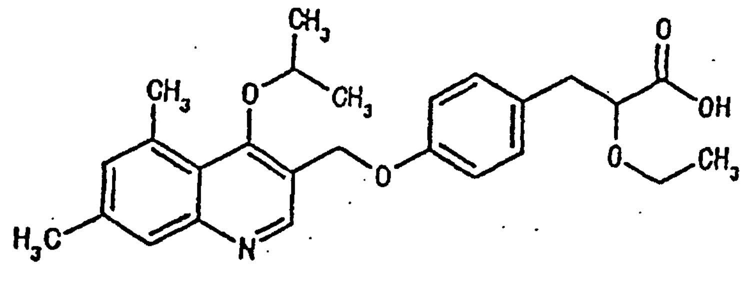

The ester compound (570 mg, 1.22 mmol) synthesized by the reaction 1 of Example 5 was subjected to the same treatment as in Example 2 to obtain an intended title compound (420 mg, 79%) as white crystals.
Melting point = 144°C (dec.)
1H-N.M.R.(CDCl3, 270MHz)d=8.82(s, 1H), 8.29(s, 1H), 7.38(s, 1H), 7.22 and 6.89(2d, each 2H, J=8.6Hz), 5.20(s, 2H), 4.50(quintet, 1H, J=6.0Hz), 4.08(dd, 1H, J=4.9, 7.3Hz), 3.69-3.58(m, 1H), 3.52-3.41(m, 1H), 3.08(dd, 1H, J=4.9, 13.9Hz), 3.99(dd, 1H, J=7.3, 13.9Hz), 2.92 and 2.56(2s, each 3H), 1.47(d, 6H, J=6.0Hz), 1.19(t, 3H, J=7.3Hz)

[Example 6] Synthesis of 2-ethoxy-3-{[4-(4-isopropoxy-6-ethylquinoline-3-yl)methyloxy]phenyl}propanoic acid: exemplary compound (67)

[Reaction 1] Synthesis of ethyl 2-ethoxy-3-{[4-(4-isopropoxy-6-ethylquinoline-3-yl)methyloxy]phenyl}propanoate

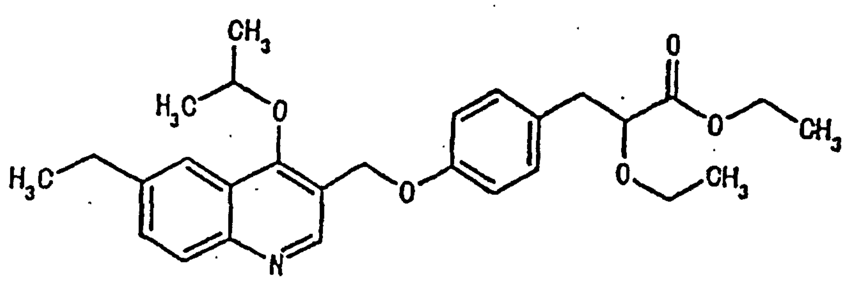

4-Isopropoxy-6-ethylquinoline-3-methanol (756 mg, 3 mmol) synthesized in Reference Example 11 and ethyl 2-ethoxy-3-(4-hydroxyphenyl)propanoate (715 mg, 3 mmol) were subjected to the same treatment as in Example 1, to obtain an intended title compound (970 mg, 69%) as a colorless transparent syrup.
1H-N.M.R.(CDCl3, 270MHz)d=8.90(s, 1H), 8.81(d, 1H, J=8.6Hz), 7.89(d, 1H, J=1.5Hz), 7.57(dd, 1H, J=1.5, 8.6Hz), 7.18 and 6.93(2d, each 2H, J=8.6Hz), 5.21(s, 2H), 4.60(quintet, 1H, J=6.3Hz), 4.16(q, 2H, J=7.3Hz), 3.98(t, 1H, J=6.9Hz), 3.61-3.58(m, 1H), 3.39-3.33(m, 1H), 2.96(d, 2H, J=6.9Hz), 2.86(q, 2H, J=7.6Hz), 1.40(d, 6H, J=6.3Hz), 1.35 (t, 3H, J=7.6Hz), 1.21(t, 3H, J=7.3Hz), 1.17(t, 3H, J=7.3Hz)

[Reaction 2] Synthesis of 2-ethoxy-3-{[4-(4-isopropoxy-6-ethylquinoline-3-yl)methyloxy]phenyl}propanoic acid: exemplary compound (67)

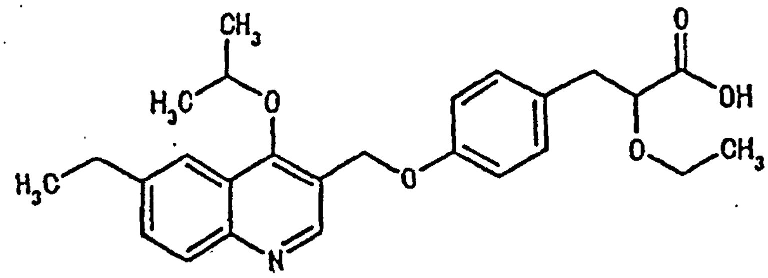

The ester compound (960 mg, 2.06 mmol) synthesized by the reaction 1 of Example 6 was subjected to the same treatment as in Example 2 to obtain an intended title compound (740 mg, 82%) as white crystals.
Melting point = 92 to 94°C
1H-N.M.R.(CDCl3, 270MHz)d=8.92(s, 1H), 8.26(d, 1H, J=8.6Hz), 7.94(d, 1H, J=1.3Hz), 7.67(dd, 1H, J=1.3, 8.6Hz), 7.23 and 6.92(2d, each 2H, J=8.7Hz), 5.21(s, 2H), 4.75(quintet, 1H, J=6.3Hz), 4.16-4.06(m, 3H), 3.73-3.62(m, 1H), 3.51-3.40(m, 1H), 3.10(dd, 1H, J=4.9, 14.1Hz), 3.01(dd, 1H, J=7.2, 14.1Hz), 2.89(q, 2H, J=7.8Hz), 1.44(d, 6H, J=6.3Hz), 1.35(t, 3H, J=7.8Hz), 1.19(t, 3H, J=7.8Hz)

[Example 7] Synthesis of 2-ethoxy-3-{[4-(4-octyloxy-6-methoxyquinoline-3-yl)methyloxy]phenyl}propanoic acid: exemplary compound (63)

[Reaction 1] Synthesis of ethyl 2-ethoxy-3-{[4-(4-octyloxy-6-methoxyquinoline-3-yl)methyloxy]phenyl}propanoate

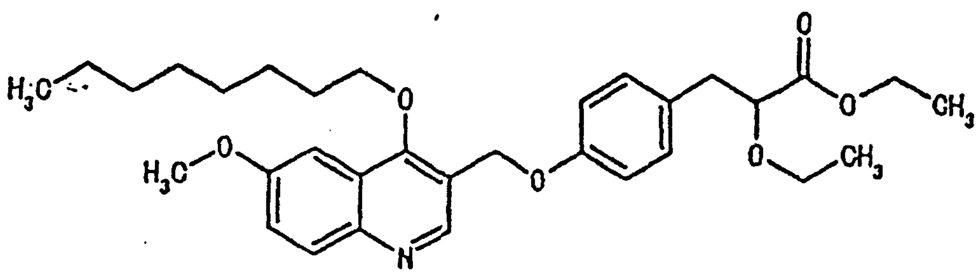

4-octyloxy-6-methoxyquinoline-3-methanol (952 mg, 3 mmol) synthesized in Reference Example 13 and ethyl 2-ethoxy-3-(4-hydroxyphenyl)propanoate (715 mg, 3 mmol) synthesized in Reference Example 7 were subjected to the same treatment as in Example 1 to obtain an intended title compound (1.07 g, 66%) as a colorless transparent syrup. 1H-N.M.R.(CDCl3, 270MHz)d=8.81(s, 1H), 8.00(d, 1H, J=8.6Hz), 7.45-7.28(m, 2H), 7.19 and 6.94(2d, each 2H, J=8.6Hz), 5.21(s, 2H), 4.25-4.15(m, 4H), 3.98(t, 1H, J=6.9Hz), 3.96(s, 3H), 3.65-3.55(m, 1H), 3.40-3.30(m, 1H), 2.97 (d, 2H, J=6.9Hz), 1.97-1.90(m, 2H), 1.60-1.45(m, 2H), 1.40-1.15(m, 17H), 0.88(t, 3H, J=6.6Hz)

[Reaction 2] Synthesis of 2-ethoxy-3-{[4-(4-isopropoxy-6-ethylquinoline-3-yl)methyloxy]phenyl}propanoic acid: exemplary compound (67)

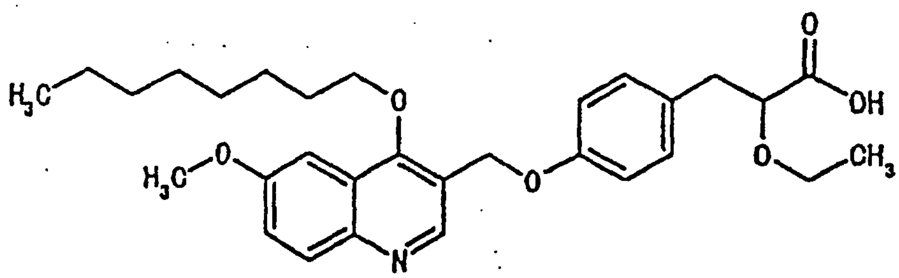

The ester compound (1.07 g, 2.00 mmol) synthesized by the reaction 1 of Example 7 was subjected to the same treatment as in Example 2 to obtain a free carboxylic acid. The carboxylic acid was converted into a hydrochloride using 4 N hydrochloric acid and dioxane to obtain an intended title compound (870 mg, 80%) as white crystals.
Melting point = 142 to 143°C
1H-N.M.R.(DMSO-d6, 270MHz)d=8.81(s, 1H), 8.00(d, 1H, J=8.6Hz), 7.45-7.28(m, 2H), 7.19 and 6.94(2d, each 2H, J=8.6Hz), 5.21(s, 2H), 4.20(t, 2H, J=6.9Hz), 3.98(t, 1H, J=6.9Hz), 3.96(s, 3H), 3.65-3.55(m, 1H), 3.40-3.30(m, 1H), 2.97(d, 2H, J=6.9Hz), 1.97-1.90(m, 2H), 1.60-1.45(m, 2H), 1.40-1.15(m, 11H), 0.88(t, 3H, J=6.6Hz)

[Example 8] Synthesis of 2-ethoxy-3-{[4-(4-isopropoxy-6,7-dimethoxyquinoline-3-yl)methyloxy]phenyl}propanoic acid: exemplary compound (65)

[Reaction 1] Synthesis of ethyl 2-ethoxy-3-{[4-(4-isopropoxy-6,7-dimethoxyquinoline-3-yl)methyloxy]phenyl} propanoate

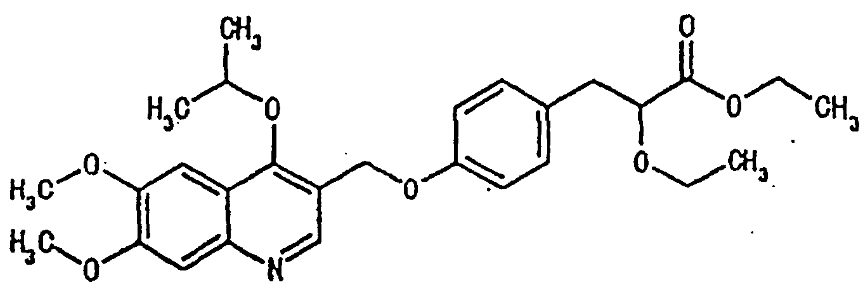

4-propoxy-6,7-dimethoxyquinoline-3-methanol (832 mg, 3 mmol) synthesized in Reference Example 14 and ethyl 2-ethoxy-3-(4-hydroxyphenyl)propanoate (715 mg, 3 mmol) synthesized in Reference Example 7 were subjected to the same treatment as in Example 1 to obtain an intended title compound (760 mg, 51%) as a colorless transparent syrup.
1H-N.M.R.(CDCl3, 270MHz)d=8.77(s, 1H), 7.41(s, 1H), 7.37(s, 1H), 7.19 and 6.94(2d, each 2H, J=8.9Hz), 5.18(s, 2H), 4.58(quintet, 1H, J=6.2Hz), 4.22(q, 2H, J=7.3Hz), 4.04(s, 6H), 3.98(t, 1H, J=6.3Hz), 3.70-3.55(m, 1H), 3.45-3.35 (m, 1H), 2.97(d, 2H, J=6.3Hz), 1.40(d, 6H, J=6.2Hz), 1.31-1.15(m, 6H)

[Reaction 2] Synthesis of 2-ethoxy-3-{[4-(4-isopropoxy-6,7-dimethoxyquinoline-3-yl)methyloxy]phenyl}propanoic acid: exemplary compound (65)

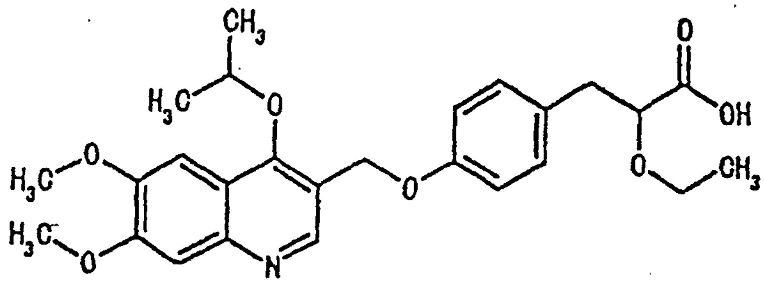

The ester compound (710 mg, 1.43 mmol) synthesized by the reaction 1 of Example 8 was subjected to the same treatment as in Example 2 to obtain a free carboxylic acid. The carboxylic acid was converted into a hydrochloride using 4 N hydrochloric acid and dioxane to obtain an intended title compound (520 mg, 72%) as white crystals.
Melting point = 155°C (dec.)
1H-N.M.R.(DMSO-d6, 270MHz)d=8.77(s, 1H), 7.41(s, 1H), 7.37(s, 1H), 7.19and6.94(2d, each 2H, J=8.9Hz), 5.18(s, 2H), 4.58(quintet, 1H, J=6.2Hz), 4.04(s, 6H), 3.98(t, 1H, J=6.3Hz), 3.70-3.55(m, 1H), 3.45-3.35(m, 1H), 2.97(d, 2H, J=6.3Hz), 1.40(d, 6H, J=6.2Hz), 1.21(t, 6H, J=7.3Hz)

[Example 9] Synthesis of 2-ethoxy-3-{[4-(2-phenylquinoline-4-yl)methyloxy]phenyl}propanoic acid: exemplary compound (97)

[Reaction 1] Synthesis of ethyl 2-ethoxy-3-{[4-(2-phenylquinoline-4-yl)methyloxy]phenyl}propanoate

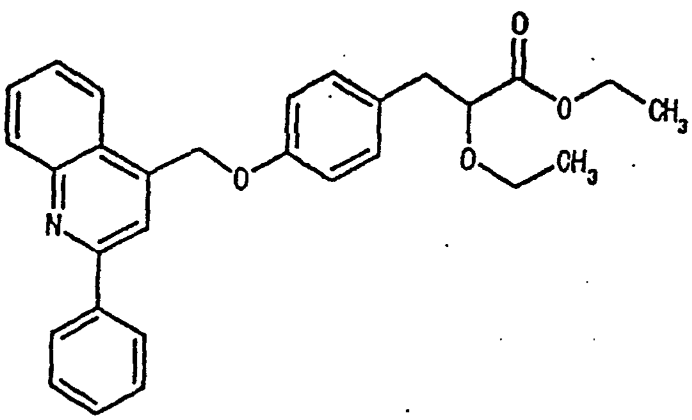

The alcohol compound (1.92 g) synthesized in Reference Example 15 and the phenol (1.94 g) synthesized in Reference Example 7 were subjected to the same treatment as in Example 1 to obtain an intended title compound (2.47 g, 66%) as a colorless transparent oil. 1H NMR (CDCl3, 270MHz) d=8.23(d, 1H, J=7.9Hz), 8.15(d, 2H, J=7.9Hz), 8.04(s, 1H), 7.99(d, 1H, J=8.2Hz), 7.76(t, 1H,J=8.2Hz), 7.56-7.46(m, 4H), 7.21(d, 2H, J=8.2Hz), 7.00(d, 2H, J=8.2Hz), 5.58(s, 2H), 4.16(q, 2H, J=6.9Hz), 3.99(t, 1H, J=6.9Hz), 3.67-3.55(m, 1H), 3.41-3.30(m, 1H), 2.98(d, 2H, J=6.9Hz), 1.29-1.13(m, 6H)

[Reaction 2] Synthesis of 2-ethoxy-3-{[4-(2-phenylquinoline-4-yl)methyloxy]phenyl}propanoic acid: exemplary compound (97)

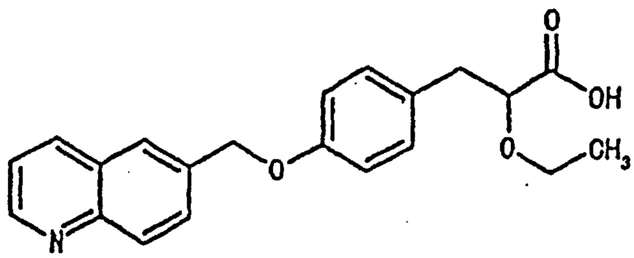

The ester compound (1.0 g) synthesized by the reaction 1 was subjected to the same treatment as in Example 2 to obtain an intended title compound (1.05 g, 100%) as white crystals.
Melting point = 142 to 143.5°C
1H NMR (CDCl3, 270MHz) d=8.24(d, 1H, J=8.5Hz), 8.15-8.12(m, 2H), 8.03(s, 1H), 7.98(d, 1H, J=8.5Hz), 7.76(t, 1H, J=7.1Hz), 7.60-7.44(m, 4H), 7.21(d, 2H, J=8.5Hz), 7.00(d, 2H, J=8.5Hz), 5.57(s, 2H), 4.06(dd, 1H, J=4.1, 7.6Hz), 3.64-3.57(m, 1H), 3.50-3.42(m, 1H), 3.10(dd, 1H, J=4.1, 14.3Hz), 2.98(dd, 1H, J=7.6, 14.3Hz), 1.17(t, 3H, J=7.1Hz)

[Example 10] Synthesis of 2-ethoxy-3-{[4-(quinoline-6-yl)methyloxy]phenyl}propanoic acid: exemplary compound (32)

[Reaction 1] Synthesis of ethyl 2-ethoxy-3-{[4-(quinoline-6-yl)methyloxy]phenyl}propanoate

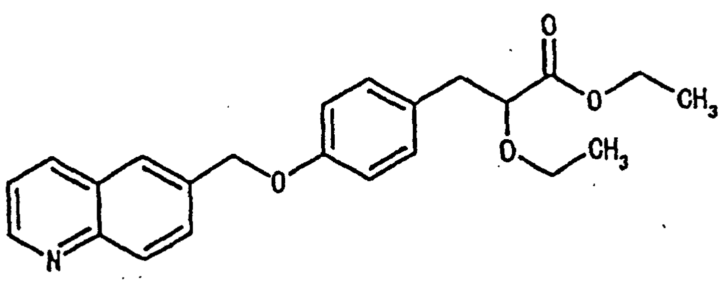

The alcohol compound (1.31 g) synthesized in Reference Example 16 and the phenol (1.96 g) synthesized in Reference Example 7 were subjected to the same treatment as in Example 1 to obtain an intended title compound (1.47 g, 47%) as a colorless transparent oil. 1H NMR (CDCl3, 270MHz) d=8.92(d, 1H, J=4.3Hz), 8.19-8.11(m, 2H), 7.88(s, 1H), 7.77(d, 1H, J=8.6Hz), 7.42(dd, 1H, J=4.3. 8.2Hz), 7.18(d, 2H, J=8.6Hz), 6.94(d, 2H, J=8.6Hz), 5.24(s, 2H), 4.16(q, 2H, J=7.3Hz), 3.98(t, 1H, J=6.9Hz), 3.66-3.55(m, 1H), 3.41-3.30(m, 1H), 2.96(d, 2H, J=6.6Hz), 1.29-1.15 (m, 6H)

[Reaction 2] Synthesis of 2-ethoxy-3-{[4-(quinoline-6-yl)methyloxy]phenyl}propanoic acid: exemplary compound (32)

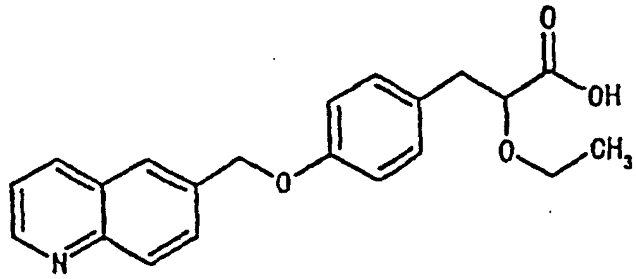

The ester compound (1.47 g) synthesized by the reaction 1 was subjected to the same treatment as in Example 2 to obtain an intended title compound (1.01 g, 75%) as white crystals.
Melting point = 96 to 98°C
1H NMR (CDCl3, 270MHz) d=8.94(d, 1H, J=4.4Hz), 8.23(d, 1H, J=8.3Hz), 8.09(d, 1H, J=8.5Hz), 7.90(s, 1H), 7.77(d, 1H, J=8.8Hz), 7.47(dd, 1H, J=4.4, 8.3Hz), 7.22(d, 2H, J=8.8Hz), 6.93(d, 2H, J=8.8Hz), 5.19(s, 2H), 4.11(dd, 1H, J=4.9, 7.1Hz), 3.71-3.64(m, 1H), 3.53-3.46(m, 1H), 3.13-3.01(m, 2H), 1.21(t, 3H, J=7.1Hz)

[Example 11] Synthesis of 2-ethoxy-3-{[4-(4-methoxyquinoline-2-yl)methyloxy]phenyl}propanoic acid: exemplary compound (99)

[Reaction 1] Synthesis of ethyl 2-ethoxy-3-{[4-(4-methoxyquinoline-2-yl)methyloxy]phenyl}propanoate

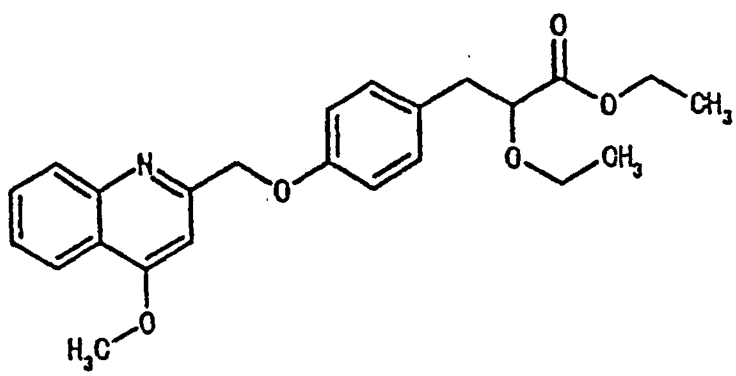

The alcohol compound (290 mg) synthesized in Reference Example 17 and the phenol (370 mg) synthesized in Reference Example 7 were subjected to the same treatment as in Example 1 to obtain an intended title compound (300 mg, 48%) as a colorless transparent oil.
1H NMR (CDCl3, 270MHz) d=8.18(d, 1H, J=8.2Hz), 7.99(d, 1H, J=8.2Hz), 7.71(t, 1H, J=6.6Hz), 7.50(t, 1H, J=6.6Hz), 7.16(d, 2H, J=6.6Hz), 7.03(s, 1H), 6.96(d, 2H, J=6.6Hz), 5.30(s, 2H), 4.15(q, 2H, J=6.9Hz), 4.04(s, 3H), 3.97(t, 1H, J=6.5Hz), 3.63-3.57(m, 1H), 3.38-3.31(m, 1H), 2.95(d, 1H, J=6.5Hz), 1.23-1.13(m, 6H)

[Reaction 2] Synthesis of 2-ethoxy-3-{[4-(4-methoxyquinoline-2-yl)methyloxy]phenyl}propanoic acid: exemplary compound (99)

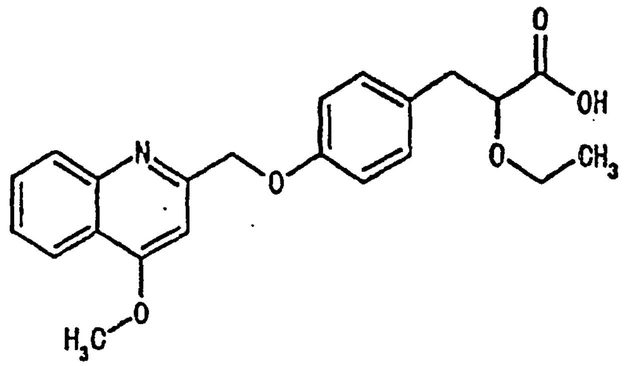

The ester compound (300 mg) synthesized by the reaction 1 was subjected to the same treatment as in Example 2 to obtain an intended title compound (126 mg, 45%) as white crystals.
Melting point = 138 to 141°C
1H NMR (CDCl3, 270MHz) d=8.18(d, 1H, J=8.4Hz), 8.06(d, 1H, J=8.4Hz), 7.72(t, 1H, J=8.3Hz), 7.50(t, 1H, J=8.3Hz), 7.20(d, 2H, J=8.6Hz), 7.03(s, 1H), 6.93(d, 2H, J=8.6Hz), 5.23(s, 2H), 4.10(d, 1H, J=6.6Hz), 4.04(s, 3H), 3.68-3.60(m, 1H), 3.55-3.49(m, 1H), 3.13-3.08(m, 1H), 3.04-2.98(m, 1H), 1.21(t, 3H, J=6.8Hz)

[Example 12] Synthesis of 2-ethoxy-3-([4-(4-isopropoxy-7-trifluoromethylquinoline-3-yl)methyloxy]phenyl}propanoic acid: exemplary compound (85)

[Reaction 1] Synthesis of ethyl 2-ethoxy-3-{[4-(4-isopropoxy-7-trifluoromethylquinoline-3-yl)methyloxy]phenyl} propanoate

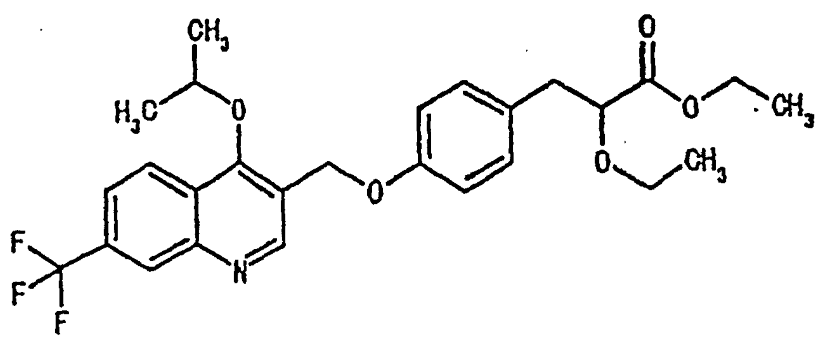

The alcohol compound (570 mg) synthesized in Reference Example 19 and the phenol (480 mg) synthesized in Reference Example 7 were subjected to the same treatment as in Example 1 to obtain an intended title compound (650 mg, 64%) as a colorless transparent oil.
1H NMR (CDCl3, 270MHz) d=9.07(s, 1H), 8.40(s, 1H), 8.26(d, 1H, J=8.9Hz), 7.72(d, 1H, J=8.9Hz), 7.20(d, 2H, J=8.9Hz), 6.93(d, 2H, J=8.9Hz), 5.24(s, 2H), 4.62(m, 1H), 4.17(q, 2H, J=6.9Hz), 3.98(t, 1H, J=6.9Hz), 3.64-3.56(m, 1H), 3.39-3.33(m, 1H), 2.97(d, 2H, J=6.6Hz), 1.41(d, 6H, J=6.3Hz), 1.25-1.14(m, 6H)

[Reaction 2] Synthesis of 2-ethoxy-3-{[4-(4-isopropoxy-7-trifluoromethylquinoline-3-yl)methyloxy]phenyl}propanoic acid: exemplary compound (85)

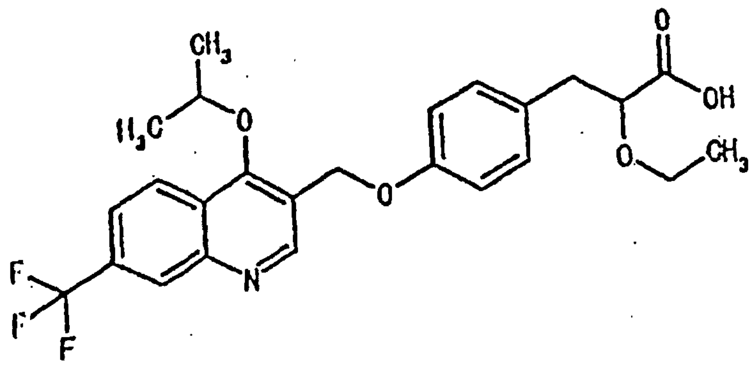

The ester compound (626 mg) synthesized by the reaction 1 was subjected to the same treatment as in Example 2 to obtain an intended title compound (450 mg, 76%) as a white amorphous solid.
1H NMR (CDCl3, 270MHz) d=9.06(s, 1H), 8.41(s, 1H), 8.27(d, 1H, J=8.8Hz), 7.73(d, 1H, J=8.8Hz), 7.21(d, 2H, J=8.7Hz), 6.95(d, 2H, J=8.7Hz), 5.24(s, 2H), 4.65-4.61(m, 1H), 4.09(dd, 1H, J=4.4, 7.3Hz), 3.64-3.60(m, 1H), 3.53-3.49 (m, 1H), 3.12(dd, 1H, J=4.4, 14.1Hz), 3.00(dd, 1H, JJ=7.1, 14.1Hz), 1.42(d, 6H, J=6.1Hz), 1.20(t, 3H, J=7.1Hz)

[Example 13] Synthesis of 3-{[4-(8-chloro-4-isopropoxy quinoline-3-yl)methyloxy]phenyl}-2-ethoxypropanoic acid: exemplary compound (76)

[Reaction 1] Synthesis of ethyl 3-{[4-(8-chloro-4-isopropoxyquinoline-3-yl)methyloxy]phenyl}-2-ethoxypropanoate

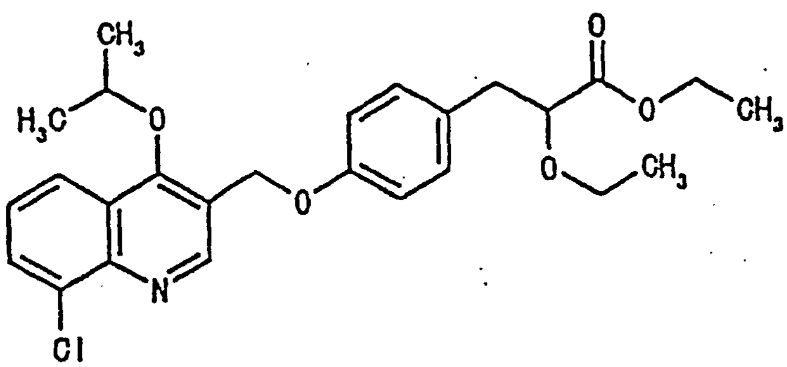

The alcohol compound (650 mg, 2.6 mmol) synthesized in Reference Example 20 and the phenol (610 mg, 2.6 mmol) synthesized in Reference Example 7 were subjected to the same treatment as in Example 1 to obtain an intended title compound (890 mg, 73%) as a colorless transparent oil.
1H NMR (CDCl3, 270MHz) d=9.09(1H s), 8.08(1H, dd, J=8.2, 1.4Hz), 7.84(1H, dd, J=7.6, 1.4Hz), 7.47(1H, dd., J=8.2, 7.6Hz), 7.19(2H, dd, J=6.5, 2.4Hz), 6.92(2H, dd, J=6.5, 1.9Hz), 5.24 (2H, s), 4.67(1H, sept, J=6.2Hz), 4.16(2H, dd, J=14, 7.3Hz), 3.97 (1H, t, J=6.8Hz), 3.59(1H, dd, J=14, 7.0Hz), 3.37(1H, dd., J=14, 7.0Hz), 2.69(2H, d, J=6.8Hz), 1.40 (6H, d, J=6.2Hz), 1.21(3H, t, J=7.3Hz), 1.17(3H, t, J=7.0Hz)

[Reaction 2] Synthesis of 3-{[4-(8-chloro-4-isopropoxy quinoline-3-yl)methyloxy]phenyl}-2-ethoxypropanoic acid: exemplary compound (76)

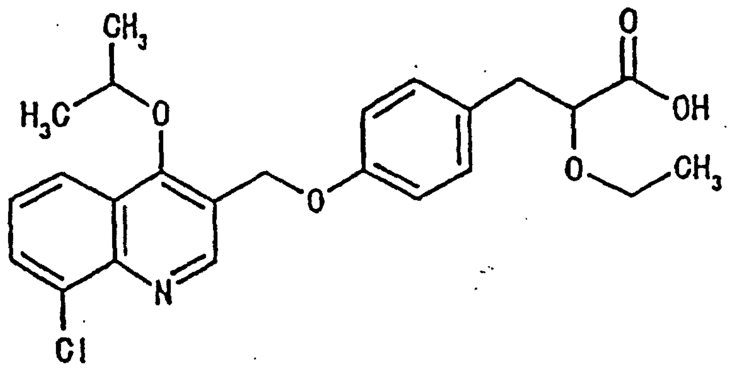

The ester compound (880 mg, 1.9 mmol) synthesized by the reaction 1 was subjected to the same treatment as in Example 2 to obtain an intended title compound (740 mg, 89%) as white crystals.
Melting point = 87 to 88°C
1H NMR (CDCl3, 270MHz) d=9.10(lH, s), 8.08(1H, dd, J=8.1, 1.4Hz), 7.83(1H, dd, J=7.6, 1.4Hz), 7.47(1H, dd., J=8.1, 7.6Hz), 7.21(2H, d, J=8.6Hz), 6.93(2H, d, J=8.6Hz), 5.23(2H, s), 4.62(1H, sept, J=6.2Hz), 4.07(1H, dd, J=7.8, 4.6Hz), 3.64(1H, dq, J=9.0, 6.8Hz), 3.44(1H, dq, J=9.0, 4.8Hz), 3.09(1H, dd, J=14, 4.6Hz), 2.98(1H, dd, J=14, 7.8Hz), 1.39 (6H, d, J=6.2Hz), 1.18(3H, t, J=6.8Hz)
[Example 14] Synthesis of 2-ethoxy-3-{[4-(4-isopropoxy-8-trifluoromethylquinoline-3-yl)methyloxy]phenyl}propanoic acid: exemplary compound (86)

[Reaction 1] Synthesis of ethyl 2-ethoxy-3-{[4-(4-isopropoxy-8-trifluoromethylquinoline-3-yl)methyloxy]phenyl} propanoate

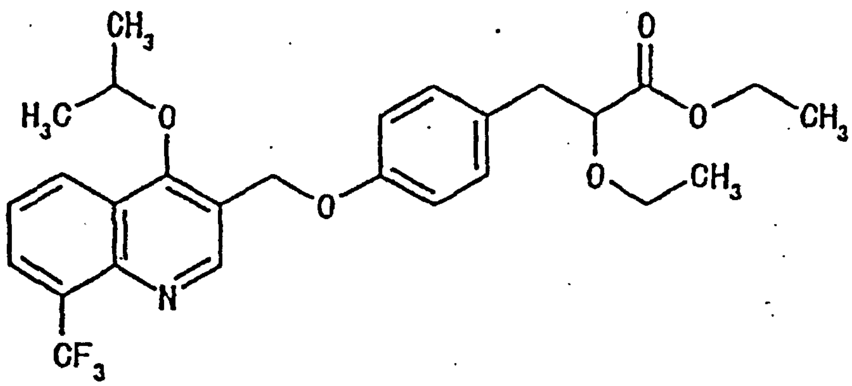

The alcohol compound (710 mg, 2.5 mmol) synthesized in Reference Example 23 and the phenol (610 mg, 2.5 mmol) synthesized in Reference Example 7 were subjected to the same treatment as in Example 1 to obtain an intended title compound (920 mg, 77%) as a colorless transparent oil.
1H NMR (CDCl3, 270MHz) d=9.13(1H, s), 8.37(1H, dd, J=8.5, 0.9Hz), 8.08(1H, d, J=7.0Hz), 7.60(1H, t, J=7.7Hz), 7.19(2H, dd, J=6.5, 2.2Hz), 6.92(2H, dd, J=6.8, 2.0Hz), 5.24(2H, s), 4.61(1H, sept, J=6.0Hz), 4.16(2H, dd, J=14, 7.2Hz), 3.98(1H, t, J=6.8Hz), 3.62(1H, dd, J=14, 7.0Hz), 3.38(1H, dd, J=14, 7.0Hz), 2.97(2H, d, J=6.8Hz), 1.41(6H, d, J=6.0Hz), 1.21(3H, t, J=7.2Hz), 1.17(3H, t, J=7.0Hz)

[Reaction 2] Synthesis of 2-ethoxy-3-{[4-(4-isopropoxy-8-trifluoromethylquinoline-3-yl)methyloxy]phenyl}propanoic acid: exemplary compound (86)

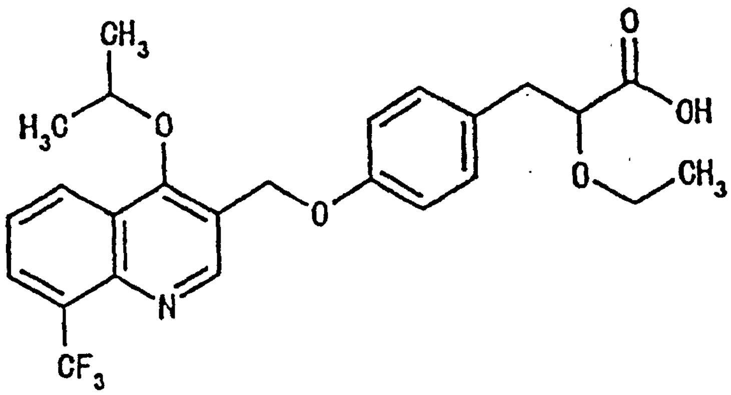

The ester compound (900 mg, 1.8 mmol) synthesized by the reaction 1 was subjected to the same treatment as in Example 2 to obtain an intended title compound (740 mg, 83%) as white crystals.
Melting point = 113 to 114°C
1H NMR (CDCl3, 270MHz) d=9.12(1H, s), 8.36(1H, dd, J=8.6, 0.8Hz), 8.09(1H, dd, J=6.5, 0.8Hz), 7.61(1H, m), 7.20 (2H, dd, J=6.8, 2.1Hz), 6.93(2H, dd, J=6.8, 2.1Hz), 5.24(2H, s), 4.61(1H, sept, J=6.0Hz), 4.07(1H, dd, J=7.3, 4.4Hz), 3.62(1H, dq, J=14, 7.0Hz), 3.47(1H, dq, J=14, 7.0Hz), 3.10(1H, dd., J=14, 4.4Hz), 2.97(1H, dd, J=14, 7.3Hz), 1.41(6H, d, J=6.0Hz), 1.19(3H, t, J=7.0Hz)

[Example 15] Synthesis of 2-ethoxy-3-{[4-(8-fluoro-4-isopropoxyquinoline-3-yl)methyloxy]phenyl}propanoic acid: exemplary compound (82)

[Reaction 1] Synthesis of ethyl 2-ethoxy-3-{[4-(8-fluoro-4-isopropoxyquinoline-3-yl)methyloxy]phenyl}propanoate

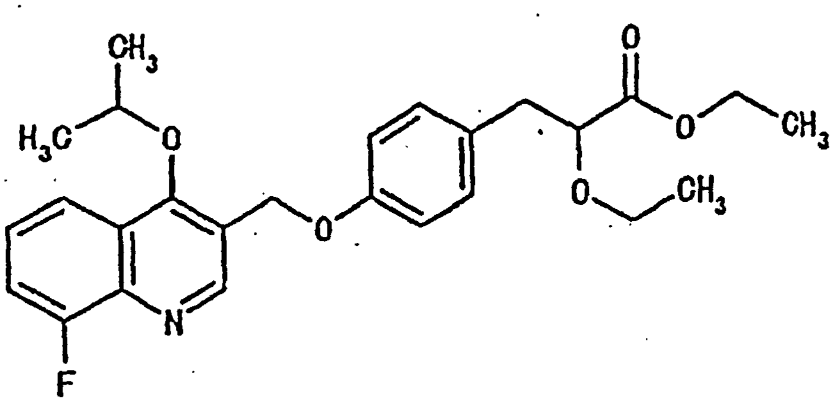

The alcohol compound (610 mg, 2.6 mmol) synthesized in Reference Example 25 and the phenol (620 mg, 2.6 mmol) synthesized in Reference Example 7 were subjected to the same treatment as in Example 1 to obtain an intended title compound (920 mg, 77%) as a colorless transparent oil.
1H NMR (CDCl3, 270MHz) d=9.01(1H, s), 7.90(1H, dd, J=8.1, 1.4Hz), 7.52-7.36(2H, m), 7.19(2H, dd, J=7.0, 2.2Hz), 6.93(2H, dd, J=7.0, 2.2Hz), 5.23(2H, s), 4.63(1H, sept, J=5.9), 4.16(2H, dd, J=14, 7.0Hz), 3.98(1H, t, J=6.5Hz), 3.59(1H, dq, J=14, 7.0Hz), 3.37(1H, dq, J=14, 7.0Hz), 2.97(2H, d, J=6.5Hz), 1.40(6H, d, J=5.9Hz), 1.22(3H, t, J=7.0Hz), 1.17(3H, t, J=7.0Hz)

[Reaction 2] Synthesis of 2-ethoxy-3-{[4-(8-fluoro-4-isopropoxyquinoline-3-yl)methyloxy]phenyl}propanoic acid: exemplary compound (82)

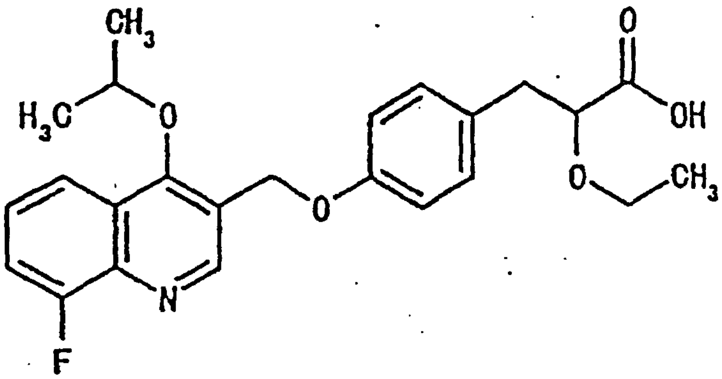

The ester compound (910 mg, 2.0 mmol) synthesized by the reaction 1 was subjected to the same treatment as in Example 2 to obtain an intended title compound (730 mg, 85%) as white crystals.
Melting point = 131 to 132°C
1H NMR (CDCl3, 270MHz) d=9.05(1H s), 7.92(1H, dd, J=8.1, 1.5Hz), 7.65-7.38(2H, m), 7.23(2H, dd, J=6.5, 2.2Hz), 6.94(2H, dd, J=6.5, 2.2Hz), 5.22(2H, s), 4.65(1H, sept, J=6.0Hz), 4.08(1H, dd, J=7.8, 4.6Hz), 3.67(1H, dq, J=9.2, 7.0Hz), 3.44(1H, dq, J=9.2, 7.0Hz), 3.11(1H, dd, J=14, 4.6Hz), 3.00(1H, dd, J=14, 7.8Hz), 1.40(6H, d, J=6.0Hz), 1.19 (3H, t, J=7.0Hz)

[Example 16] Synthesis of 2-ethoxy-3-{[4-(quinoline-8-yl)methyloxy]phenyl}propanoic acid: exemplary compound (32)

[Reaction 1] Synthesis of ethyl 2-ethoxy-3-{[4-(quinoline-8-yl)methyloxy]phenyl}propanoate

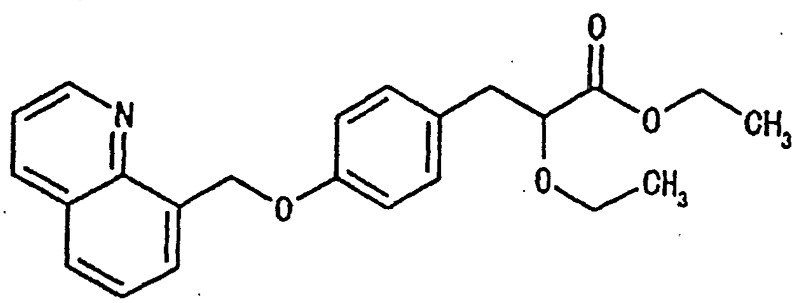

The alcohol compound (330 mg, 2.1 mmol) synthesized in Reference Example 27 and the phenol (500 mg, 2.1 mmol) synthesized in Reference Example 7 were subjected to the same treatment as in Example 1 to obtain an intended title compound (610 mg, 76%) as a colorless transparent oil.
1H NMR (CDCl3, 270MHz) d=8.94(1H, dd, J=4.2, 1.8Hz), 8.19(1H, dd, J=8.1, 1.8Hz), 7.93(1H, dd, J=7.3, 1.4Hz), 7.77 (1H, d, J=8.4Hz), 7.56(1H, t, J=8.1Hz), 7.45(1H, dd, J=8.1, 4.3Hz), 7.17(2H, m), 7.02(2H, m), 5.83(2H, s), 4.16(2H, dd, J=14, 7.1Hz), 3.98(1H, t, J=7.0Hz), 3.60(1H, dq, J=9.0, 7.0Hz), 3.36(1H, dq, J=9.0, 7.0Hz), 2.96(2H, d, J=7.0Hz), 1.21(3H, t, J=7.lHz) , 1.17(3H, t, J=7.0Hz)

[Reaction 2] Synthesis of 2-ethoxy-3-{[4-(quinoline-8-yl)methyloxy]phenyl}propanoic acid: exemplary compound (32)

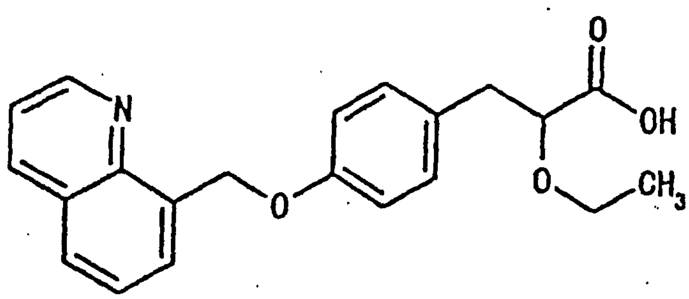

The ester compound (600 mg, 1.6 mmol) synthesized by the reaction 1 was subjected to the same treatment as in Example 2 to obtain an intended title compound (520 mg, 94%) as white crystals.
Melting point = 178°C (dec.)
1H NMR (DMSO-d6, 270MHz) d=12.7(1H, brd.s), 8.98(1H, dd, J=4.2, 1.8Hz), 8.43(1H, dd, J=8.1, 1.8Hz), 7.98(1H, d, J=8.4Hz), 7.88(1H, d, J=7.0Hz), 7.64(1H, d, J=8.4Hz), 7.60(1H, m), 7.16(2H, d, J=8.4Hz), 6.97(2H, d, J=8.4Hz), 5.72 (2H, s), 3.95(1H, dd, J=8.1, 5.4Hz), 3.51(1H, dq, J=14, 7.0Hz), 3.31(1H, dq, J=14, 7.0Hz), 2.90(1H, dd, J=14, 5.4Hz), 2.81(1H, dd, J=14, 8.1Hz), 1.05(3H, t, J=7.0Hz)

[Example 17] Synthesis of 3-{[4-(4-isopropoxy-6-methoxyquinoline-3-yl)methyloxy]phenyl}-2-phenoxypropanoic acid: exemplary compound (106)

[Reaction 1] Synthesis of methyl 3-{[4-(4-isopropoxy-6-methoxyquinoline-3-yl)methyloxy]phenyl}-2-phenoxypropanoate

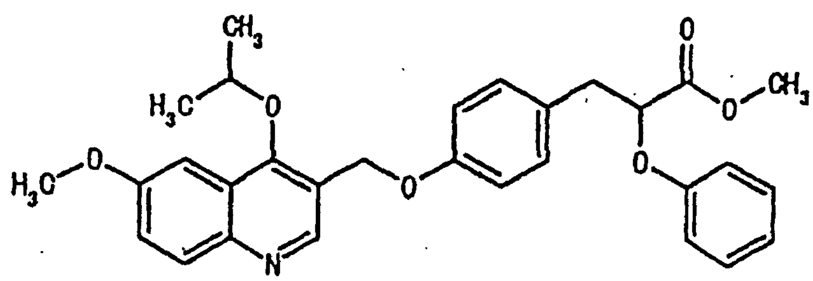

4-Propoxy-6-methoxyquinoline-3-methanol (495 mg, 2 mmol) synthesized in Reference Example 4 and methyl 3-(4-hydroxyphenyl)-2-phenoxypropanoate (545 mg, 2 mmol) synthesized in Reference Example XX were subjected to the same treatment as in Example 1 to obtain an intended title compound (640 mg, 64%) as a colorless transparent syrup.
1H-N.M.R.(CDCl3, 270MHz)d=8.71(s, 1H), 8.04(d, 1H, J=9.5Hz), 7.40-7.19(m, 6H), 6.96-6.88(m, 5H), 5.15(s, 2H), 4.86(t, 1H, J=6.0Hz), 4.62(quintet, 1H, J=6.1Hz), 3.94 and 3.60(2s, each 3H), 3.31(d, 2H, J=6.0Hz), 1.39(d, 6H, J=6.1Hz)

[Reaction 2] Synthesis of 3-{[4-(4-isopropoxy-6-methoxyquinoline-3-yl)methyloxy]phenyl}-2-phenoxypropanoic acid: exemplary compound (106)

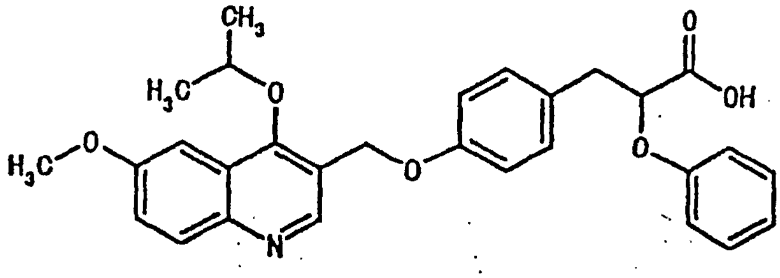

The ester compound (630 mg, 1.26 mmol) synthesized by the reaction 1 of Example 17 was subjected to the same treatment as in Example 2 to obtain an intended title compound (480 mg, 78%) as white crystals.
Melting point = 137 to 139°C
1H-N.M.R.(CDCl3, 270MHz)d=8.71(s, 1H), 8.04(d, 1H, J=9.5Hz), 7.40-7.19(m, 6H), 6.96-6.88(m, 5H), 5.15(s, 2H), 4.86(t, 1H, J=6.0Hz), 4.62(quintet, 1H, J=6.1Hz), 3.94(s, 3H), 3.31(d, 2H, J=6.0Hz), 1.39(d, 6H, J=6.1Hz)

[Example 18] Synthesis of 3-{[4-(4-isopropoxy-6-methoxyquinoline-3-yl)methyloxy]phenyl}-2-phenylthiopropenoic acid: exemplary compound (112)

[Reaction 1] Synthesis of ethyl 3-{[4-(4-isopropoxy-6-methoxyquinoline-3-yl)methyloxy]phenyl}-2-phenylthiopropenoate

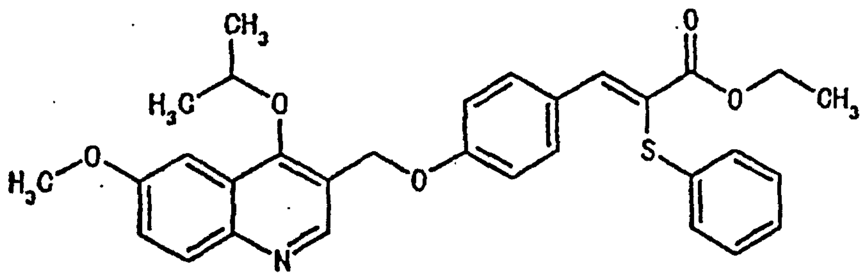

4-Propoxy-6-methoxyquinoline-3-methanol (613 mg, 2.48 mmol) synthesized in Reference Example 4 and ethyl 3-(4-hydroxyphenyl)-2-phenylthiopropenoate (750 mg, 2.48 mmol) synthesized in Reference Example 33 were subjected to the same treatment as in Example 1 to obtain an intended title compound (1.25 g, 97%) as a colorless transparent syrup.
1H-N.M.R.(CDCl3, 270MHz)d=8.81(s, 1H), 8.10(s, 1H), 7.99(d, 1H, J=9.9Hz), 7.91(d, 2H, J=9.0Hz), 7.40-7.10(m, 6H), 7.03(d, 2H, J=9.0Hz), 5.27(s, 2H), 4.57(quintet, 1H, J=6.2Hz), 4.12(q, 2H, J=7.3Hz), 3.95(s, 3H), 1.41(d, 6H, J=6.1Hz), 1.06(t, 3H, J=7.3Hz)

[Reaction 2] Synthesis of 3-{[4-(4-isopropoxy-6-methoxyquinoline-3-yl)methyloxy]phenyl}-2-phenylthiopropenoic acid: exemplary compound (112)

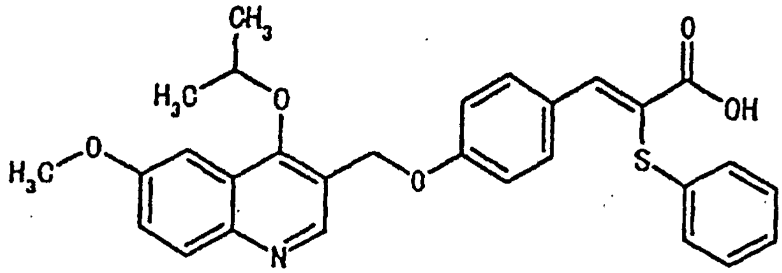

The ester compound (1.25 g, 2.36 mmol) synthesized by the reaction 1 of Example 18 was subjected to the same treatment as in Example 2 to obtain an intended title compound (890 mg, 75%) as light yellow crystals.
Melting point = 189 to 191°C
1H-N.M.R.(CDCl3, 270MHz)d=8.81(s, 1H), 8.10(s, 1H), 7.99(d, 1H, J=9.9Hz), 7.91(d, 2H, J=9.0Hz), 7.40-7.10(m, 6H), 7.03(d, 2H, J=9.0Hz), 5.27(s, 2H), 4.57(quintet, 1H, J=6.2Hz), 3.95(s, 3H), 1.41(d, 6H, J=6.1Hz)

[Example 19] Synthesis of 3-{[4-(4-isopropoxy-6-methoxyquinoline-3-yl)methyloxy]phenyl}-2-phenylthiopropanoic acid: exemplary compound (108)

[Reaction 1] Synthesis of methyl 3-{[4-(4-isopropoxy-6-methoxyquinoline-3-yl)methyloxy]phenyl}-2-phenylthiopropanoate

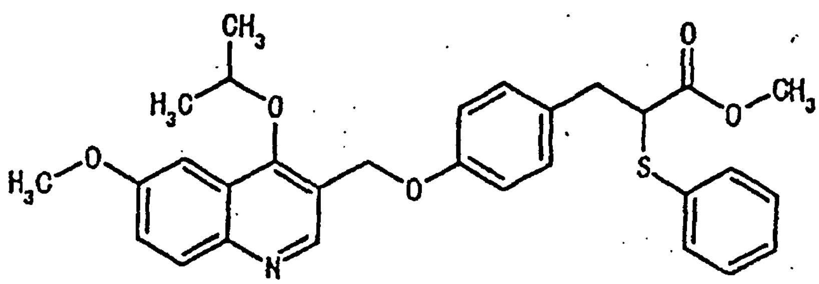

4-Propoxy-6-methoxyquinoline-3-methanol (618 mg, 2.5 mmol) synthesized in Reference Example 4 and methyl 3-(4-hydroxyphenyl)-2-phenylthiopropanoate (660 mg, 2.29 mmol) synthesized in Reference Example 35 were subjected to the same treatment as in Example 1 to obtain an intended title compound (790 g, 67%) as a colorless transparent syrup.
1H-N.M.R.(CDCl3, 270MHz)d=8.81(s, 1H), 7.99(d, 1H, J=9.6Hz), 7.45-7.30(m, 7H), 7.13 and 7.03(2d, each 2H, J=8.9Hz), 5.20(s, 2H), 4.58(quintet, 1H, J=6.3Hz), 3.95 and 3.58(2s, each 3H), 3.87(dd, 1H, J=6.6, 9.2Hz), 3.12(dd, 1H, J=9.2, 13.0Hz), 3.03(dd, 1H, J=6.6, 13.0Hz), 1.40(d, 6H, J=6.3Hz)

[Reaction 2] Synthesis of 3-{[4-(4-isopropoxy-6-methoxyquinoline-3-yl)methyloxy]phenyl}-2-phenylthiopropanoic acid: exemplary compound (108)

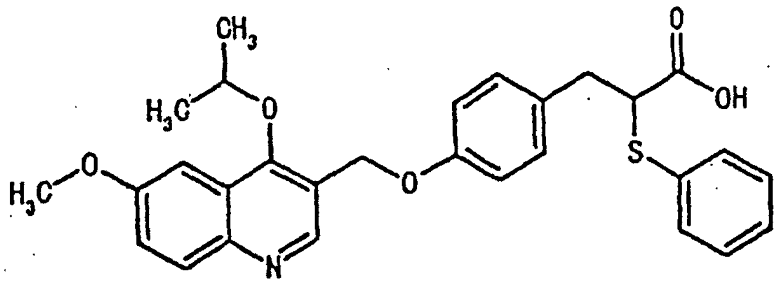

The ester compound (790 mg, 1.53 mmol) synthesized by the reaction 1 of Example 19 was subjected to the same treatment as in Example 2 to obtain an intended title compound (660 mg, 86%) as a light yellow amorphous material.
1H-N.M.R.(CDCl3, 270MHz)d=8.81(s, 1H), 7.99(d, 1H, J=9.6Hz), 7.45-7.30(m, 7H), 7.13 and 7.03(2d, each 2H, J=8.9Hz), 5.20(s, 2H), 4.58(quintet, 1H, J=6.3Hz), 3.95(2s, each 3H), 3.87(dd, 1H, J=6.6, 9.2Hz), 3.12(dd, 1H, J=9.2, 13.0Hz), 3.03(dd, 1H, J=6.6, 13.0Hz), 1.40(d, 6H, J=6.3Hz)

[Example 20] Synthesis of 2-ethoxy-3-{[3-(4-isopropoxy-6-methoxyquinoline-3-yl)methyloxy]phenyl}propanoic acid: exemplary compound (114)

[Reaction 1] Synthesis of ethyl 2-ethoxy-3-{[3-(4-isopropoxy-6-methoxyquinoline-3-yl)methyloxy]phenyl}propanoate

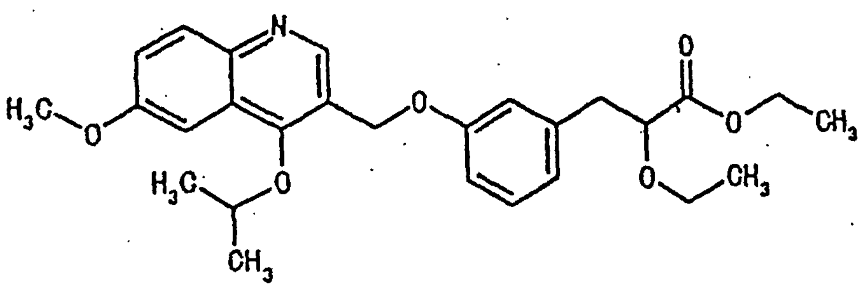

4-Isopropoxy-6-methoxyquinoline-3-methanol (903 mg, 3.65 mmol) synthesized in Reference Example 4 and ethyl 2-ethoxy-3-(3-hydroxyphenyl)propanoate (870 mg, 3.65 mmol) synthesized in Reference Example 36 were subjected to the same treatment as in Example 1 to obtain an intended title compound (1.28 g, 75%) as a colorless transparent syrup.
1H-N.M.R.(CDCl3, 270MHz)d=8.83(s, 1H), 8.00(d, 1H, J=8.6Hz), 7.45-7.20(m, 3H), 6.94(s, 1H), 6.88(d, 2H, J=8.5Hz), 5.21(s, 2H), 4.60(quintet, 1H, J=5.9Hz), 4.20(q, 2H, J=7.3Hz), 4.02(t, 1H, J=6.9Hz), 3.96(s, 3H), 3.70-3.52(m, 1H), 3.40-3.30(m, 1H), 3.00(d, 2H, J=6.9Hz), 1.41(d, 6H, J=5.9Hz), 1.31-1.13(m, 6H)

[Reaction 2] Synthesis of 2-ethoxy-3-{[3-(4-isopropoxy-6-methoxyquinoline-3-yl)methyloxy]phenyl}propanoic acid: exemplary compound (114)

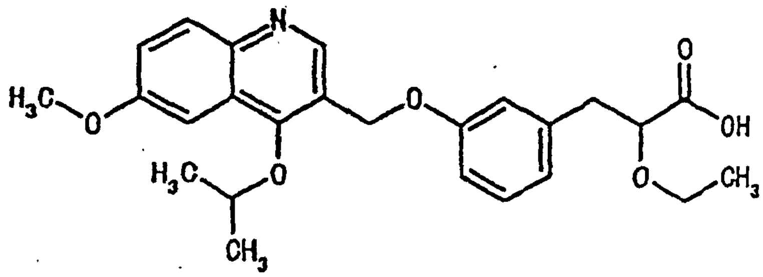

The ester compound (1.28 g, 2.74 mmol) synthesized by the reaction 1 of Example 20 was subjected to the same treatment as in Example 2 to obtain an intended title compound (900 mg, 69%) as white crystals.
Melting point = 99 to 102°C
1H-N.M.R.(CDCl3, 270MHz)d=8.83(s, 1H), 8.00(d, 1H, J=8.6Hz), 7.45-7.20(m, 3H), 6.94(s, 1H), 6.88(d, 2H, J=8.5Hz), 5.21(s, 2H), 4.60(quintet, 1H, J=5.9Hz), 4.02(t, 1H, J=6.9Hz), 3.96(s, 3H), 3.70-3.52(m, 1H), 3.40-3.30(m, 1H), 3.00 (d, 2H, J=6.9Hz), 1.41(d, 6H, J=5.9Hz), 1.21(t, 3H, J=7.3Hz)

[Example 21] Synthesis of 2-ethoxy-3-{[3-(4-isopropoxy-6-methoxyquinoline-3-yl)methyloxy]-4-methoxyphenyl} propanoic acid: exemplary compound (118)

[Reaction 1] Synthesis of ethyl 2-ethoxy-3-{[3-(4-isopropoxy-6-methoxyquinoline-3-yl)methyloxy]-4-methoxyphenyl} propanoate

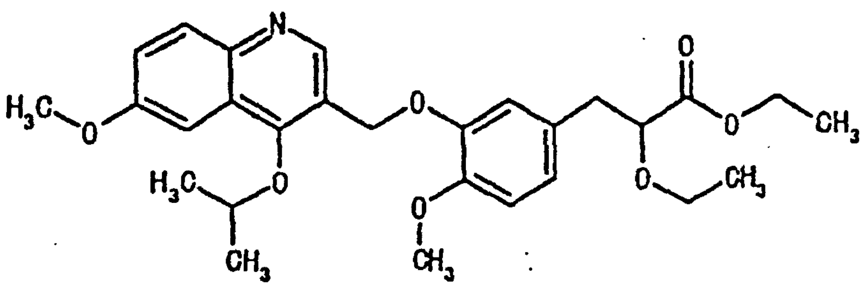

4-Isopropoxy-6-methoxyquinoline-3-methanol (558 mg, 2.26 mmol) synthesized in Reference Example 4 and ethyl 2-ethoxy-3-(3-hydroxy-4-methoxyphenyl)propanoate (610 mg, 2.26 mmol) synthesized in Reference Example 37 were subjected to the same treatment as in Example 1 to obtain an intended title compound (580 g, 52%) as a colorless transparent syrup.
1H-N.M.R.(CDCl3, 270MHz)d=8.87(s, 1H), 7.97(d, 1H, J=8.6Hz), 7.40-7.33(m, 3H), 6.96(s, 1H), 6.82(s, 1H), 5.29(s, 2H), 4.65(quintet, 1H, J=5.9Hz), 4.14(q, 2H, J=7.3Hz), 3.95 and 3.84(2s, each 3H), 3.92(t, 1H, J=6.7Hz), 3.60-3.45 (m, 1H), 3.30-3.20(m, 1H), 2.90(d, 2H, J=6.7Hz), 1.42(d, 6H, J=5.9Hz), 1.22(t, 3H, J=7.3Hz), 1.11(t, 3H, J=7.3Hz)

[Reaction 2] Synthesis of 2-ethoxy-3-{[3-(4-isopropoxy-6-methoxyquinoline-3-yl)methyloxy]-4-methoxyphenyl} propanoic acid: exemplary compound (114)

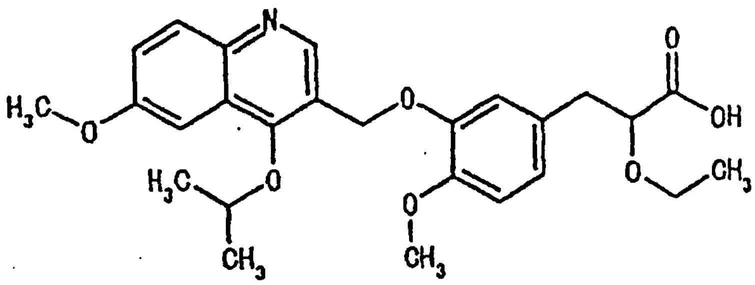

The ester compound (570 g, 1.14 mmol) synthesized by the reaction 1 of Example 21 was subjected to the same treatment as in Example 2 to obtain an intended title compound (510 mg, 88%) as white crystals.
Melting point = 144 to 146°C
1H-N.M.R.(DMSO-d6, 270MHz)d=8.23(d, 1H, J=9.3Hz), 7.75(dd, 1H, J=2.6, 9.3Hz), 7.55(d, 1H, J=2.6Hz), 7.12(d, 1H, J=2.6Hz), 6.93(d, 1H, J=9.3Hz), 6.84(d, 1H, J=9.3Hz), 5.31(s, 2H), 5.04(quintet, 1H, J=5.9Hz), 4.00 and 3.74(2s, each 3H), 3.98(dd, 1H, J=2.2, 5.0Hz), 3.51-3.45(m, 1H), 3.30-3.25(m, 1H), 2.92(dd, 1H, J=5.0, 11.2Hz), 2.80(dd, 1H, J=2.2, 11.2Hz), 1.43(d, 6H, J=5.9Hz), 1.01(t, 3H, J=6.9Hz)

[Example 22] Synthesis of dimethyl {[4-(4-isopropoxy-6-methoxyquinoline-3-yl)methyloxy]phenyl}methylmalonate hydrochloride: exemplary compound (120)

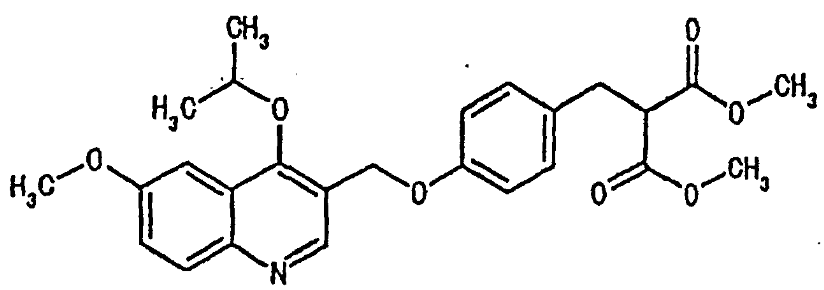

4-Isopropoxy-6-methoxyquinoline-3-methanol (742 mg, 3 mmol) synthesized in Reference Example 4 and dimethyl (4-hydroxyphenyl)methylmalonate (1.05 g, 4.5 mmol) synthesized in Reference Example 40 were subjected to the same treatment as in Example 1, followed by treatment with 4 N hydrochloric acid and dioxane, to obtain an intended title compound (890 mg, 59%) as white crystals.
Melting point = 140°C (dec.)
1H-N.M.R.(DMSO-d6, 270MHz)d=9.18(s, 1H), 8.23(d, 1H, J=9.3Hz), 7.75(dd, 1H, J=2.7, 9.3Hz), 7.54(d, 1H, J=2.7Hz), 7.18 and 7.02(2d, each 2H, J=8.4Hz), 5.32(s, 2H), 4.97(quintet, 1H, J=6.3Hz), 4.00(s, 3H), 3.83(t, 1H, J=7.9Hz), 3.61 (s, 6H), 3.05(d, 2H, J=7.9Hz), 1.43(d, 6H, J=6.3Hz)

[Example 23] Synthesis of methyl 2-{[4-(4-isopropoxy-6-methoxyquinoline-3-yl)methyloxy]phenylmethyl}-3-oxopentanoate hydrochloride: exemplary compound (137)

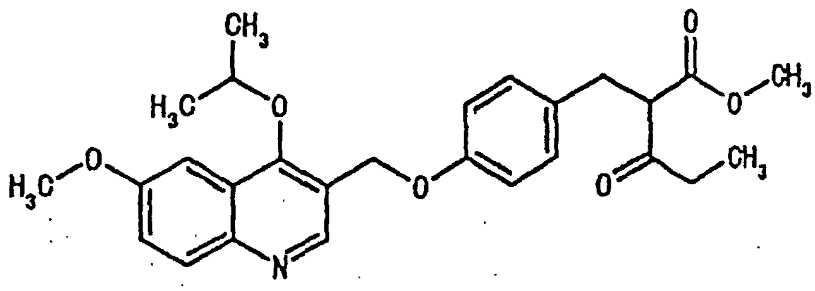

4-Isopropoxy-6-methoxyquinoline-3-methanol (989 mg, 4 mmol) synthesized in Reference Example 4 and methyl 2-[(4-hydroxyphenyl)methyl]-3-oxopentanoate (1.0 g, 4 mmol) synthesized in Reference Example 40 were subjected to the same treatment as in Example 1, followed by treatment with 4 N hydrochloric acid and dioxane, to obtain an intended title compound (1.25 g, 62%) as white crystals.
Melting point = 107°C (dec.)
1H-N.M.R.(DMSO-d6, 270MHz)d=9.18(s, 1H), 8.28(d, 1H, J=9.3Hz), 7.77(dd, 1H, J=2.7, 9.3Hz), 7.55(d, 1H, J=2.7Hz), 7.18 and 7.02(2d, each 2H, J=8.4Hz), 5.32(s, 2H), 4.98(quintet, 1H, J=6.0Hz), 4.02(t, 1H, J=8.0Hz). 4.00(s, 3H), 3.59 (s, 3H), 2.99(d, 2H, J=8.0Hz), 2.61-2.37(m, 2H), 1.42(d, 6H, J=6.0Hz), 0.86(t, 3H, J=7.3Hz)

[Example 24] Synthesis of 2-{[4-(4-isopropoxy-6-methoxyquinoline-3-yl)methyloxy]phenylmethyl}-3-oxobutanoic acid N-methylamide: exemplary compound (141)

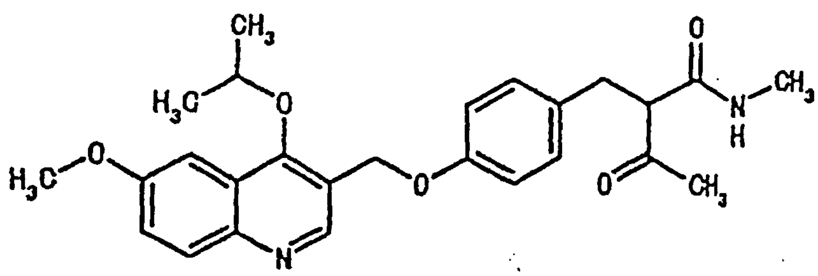

4-Isopropoxy-6-methoxyquinoline-3-methanol (1.12 g, 4.52 mmol) synthesized in Reference Example 4 and 2-[(4-hydroxyphenyl)methyl]-3-oxobutanoic acid N-methylamide (1.0 g, 4.52 mmol) synthesized in Reference Example 42 were subjected to the same treatment as in Example 1 to obtain an intended title compound (870 mg, 43%) as a colorless transparent syrup.
1H-N.M.R.(CDCl3, 270MHz)d=8.80(s, 1H), 7.98(d, 1H, J=9.6Hz), 7.70-7.35(m, 2H), 7.09 and 6.92(2d, each 2H, J=8.5Hz), 6.20(q, 1H, J=4.6Hz), 5.20(s, 2H), 4.57(quintet, 1H, J=5.3Hz), 3.96(s, 3H), 3.60(dd, 1H, J=5.9, 8.2Hz.), 3.16 (dd, 1H, J=5.9, 13.5Hz), 3.05(dd, 1H, J=8.2, 13.5Hz), 2.76(d, 3H, J=4.6Hz), 2.10(s, 3H), 1.40(d, 6H, J=5.3Hz)

[Example 25] Synthesis of 2-{[4-(4-isopropoxy-6-methoxyquinoline-3-yl)methyloxy]phenylmethylene}-3-oxobutanoic acid N-methylamide hydrochloride: exemplary compound (144)

[Reaction 1] Synthesis of (4-isopropoxy-6-methoxyquinoline-3-yl)methyloxybenzaldehyde

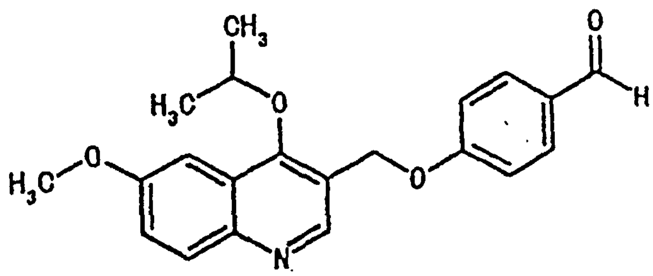

4-Isopropoxy-6-methoxyquinoline-3-methanol (4.95 g, 20 mmol) synthesized in Reference Example 4 and 4-hydroxybenzaldehyde (2.93 g, 24 mmol) were subjected to the same treatment as in Example 1 to obtain an intended title compound (3.84 g, 55%) as a colorless transparent syrup.
1H-N.M.R.(CDCl3, 270MHz)d=9.00(s, 1H), 8.82(s, 1H), 8.01(d, 1H, J=9.9Hz), 7.86(d, 2H, J=8.9Hz), 7.41-7.36(m, 2H), 7.12(d, J=8.9Hz), 5.33(s, 2H), 4.58(quintet, 1H, J=6.0Hz), 3.96(s, 3H), 1.42(d, 6H, J=6.0Hz)

[Reaction 2] Synthesis of 2-{[4-(4-isopropoxy-6-methoxyquinoline-3-yl)methyloxy]phenylmethylene}-3-oxobutanoic acid N-methylamide hydrochloride: exemplary compound (144)

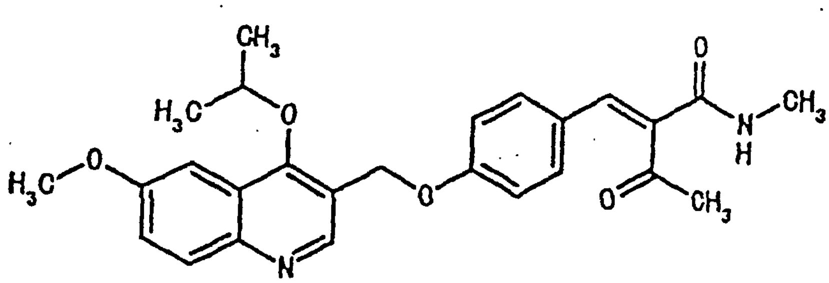

The aldehyde compound (1.0 g, 2.85 mmol) synthesized by the reaction 1 of Example 25 was suspended in toluene (25 ml). Thereto were added a 65% aqueous N-methylacetoacetamide solution (531 mg, 3.0 mmol) and piperidine acetate (200 mg). The mixture was refluxed for 6 hours with heating. The reaction mixture was allowed to cool and poured into water (100 ml), and extraction of an intended compound was conducted using ethyl acetate. The organic layer was subjected to vacuum concentration. The residue was purified by silica gel column chromatography (equivalent product of Merck C-300: 60 g, acetone : toluene = 1 : 2) to obtain a colorless transparent syrup (450 mg). The syrup was treated with 4 N hydrochloric acid and dioxane to obtain an intended title compound (270 mg, 20%) as a colorless amorphous material.
1H-N.M.R.(DMSO-d6, 270MHz)d=9.21(s, 1H), 8.28-8.24(m, 2H), 7.77(dd, 1H, J=2.5, 8.5Hz), 7.61(d, 2H, J=8.9Hz), 7.57-7.49(m, 2H), 7.20(d, 2H, J=8.9Hz), 5.41(s, 2H), 4.97(quintet, 1H, J=5.9Hz), 4.01(s, 3H), 2.69(d, 3H, J=4.6Hz), 2.33(s, 3H), 1.43(d, 6H, J=5.9Hz)

[Example 26] Synthesis of 3-{[4-(4-isopropoxy-6-methoxyquinoline-3-yl)methyloxy]phenylmethylene}-2,4-dioxopentane: exemplary compound (147).

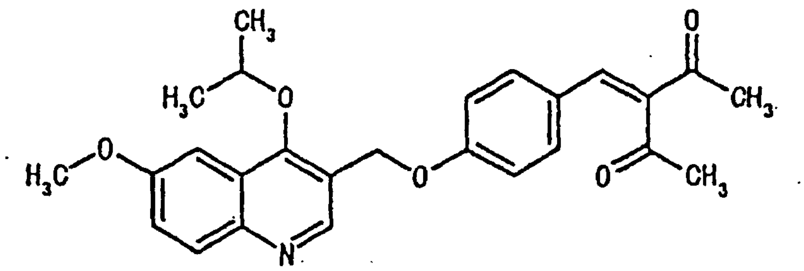

The aldehyde compound (1.0 g, 2.85 mmol) synthesized by the reaction 1 of Example 25 and acetylacetone (300 mg, 3.0 mmol) were subjected to the same treatment as in the reaction 2 of Example 25 to obtain an intended title compound (680 mg, 55%) as light yellow crystals.
Melting point = 114 to 115°C
1H-N.M.R.(CDCl3, 270MHz)d=8.81(s, 1H), 7.98(dd, 1H, J=3.0, 6.9Hz), 7.42-7.36(m, 5H), 7.01(d, 2H, J=8.7Hz), 5.28 (s, 2H), 4.56(quintet, 1H, J=6.1Hz), 3.97(s, 3H), 2.41 and 2.33(2s, each 3H), 1.42(d, 6H, J=6.1Hz)

[Example 27] Synthesis of diethyl {[3-(4-isopropoxy-6-methoxyquinoline-3-yl)methyloxy]phenyl}methylmalonate hydrochloride: exemplary compound (128)

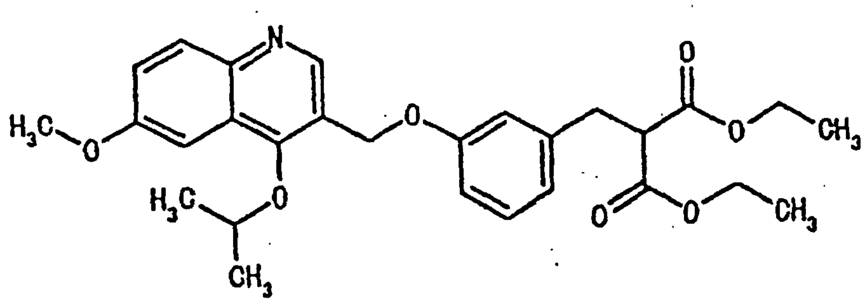

4-Isopropoxy-6-methoxyquinoline-3-methanol (742 mg, 3 mmol) synthesized in Reference Example 4 and diethyl 3-hydroxyphenyl)methylmalonate (1.2 g, 4.5 mmol) synthesized in Reference Example 44 were subjected to the same treatment as in Example 1, followed by treatment with 4 N hydrochloric acid and dioxane, to obtain an intended title compound (1.34 g, 90%) as white crystals.
Melting point = 114°C (dec.)
1H-N.M.R.(DMSO-d6, 270MHz)d=9.20(s, 1H), 8.26(d, 1H, J=9.2Hz), 7.76(dd, 1H, J=2.9, 9.5Hz), 7.55(d, 1H, J=2.9Hz), 7.25(t, 1H, J=8.0Hz), 6.99(s, 1H), 6.96(d, 1H, J=8.6Hz), 6.86(d, 1H, J=8.6Hz), 5.33(s, 2H), 4.99(quintet, 1H, J=6.0Hz), 4.10(q, 4H, J=7.3Hz), 4.01(s, 3H), 3.84(t, 1H, J=8.1Hz), 3.08(d, 2H, J=8.1Hz), 1.44(d, 6H, J=6.3Hz), 1.10(t, 6H, J=7.3Hz)

[Example 28] Synthesis of diethyl {[3-(4-isopropoxy-6-methoxyquinoline-3-yl)methyloxy]-4-methoxyphenyl} methylmalonate hydrochloride: exemplary compound (129)

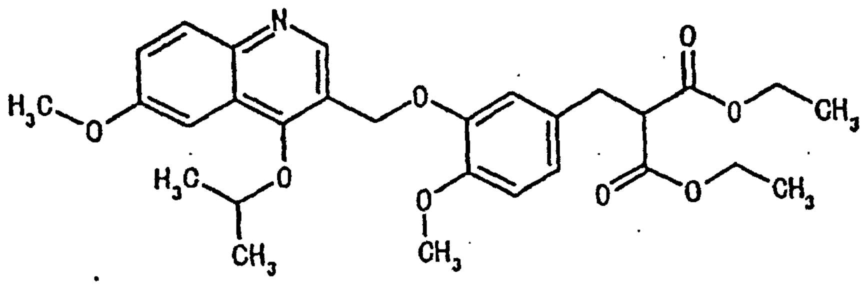

4-Isopropoxy-6-methoxyquinoline-3-methanol (620 mg, 2.5 mmol) synthesized in Reference Example 4 and diethyl 3-hydroxy-4-methoxyphenyl)methylmalonate (1.11 g, 3.76 mmol) synthesized in Reference Example 46 were subjected to the same treatment as in Example 1, followed by treatment with 4 N hydrochloric acid and dioxane, to obtain an intended title compound (920 mg, 65%) as white crystals.
Melting point = 132 to 134°C
1H-N.M.R.(DMSO-d6, 270MHz)d=9.17(s, 1H), 8.29(d, 1H, J=9.2Hz), 7.78(dd,1H, J=2.9, 9.2Hz), 7.56(d, 1H, J=2.9Hz), 7.13(s, 1H), 6.92(d, 1H, J=8.3Hz), 6.82(d, 1H, J=8.3Hz), 5.31(s, 2H), 5.05(quintet, 1H, J=6.3Hz), 4.05(q, 4H, J=7.2Hz), 4.01(s, 3H), 3.83(t, 1H, J=8.0Hz), 3.73(s, 3H), 3.03(d, 2H, J=8.0Hz), 1.44(d, 6H, J=6.3Hz), 1.10(t, 6H, J=7.2Hz)

[Reference Example 1] Synthesis of diethyl 4-methoxyphenylaminomethylenemalonate

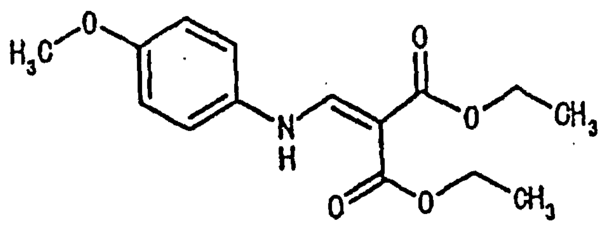

4-Methoxyaniline (49.3 g, 0.4 mol) was mixed with diethyl ethoxymethylenemalonate (86.5 g, 0.4 mol). The mixture was stirred at 120°C for 2 hours and then cooled. The ethanol formed was removed by vacuum distillation to obtain an intended title compound as a brown crude syrup (117 g, 100%).
1H-N.M.R.(DMSO-d6, 270MHz)d=10.70(d, 1H, J=9.0Hz), 8.31(d, 1H, J=9.0Hz), 7.30and6.95(2d, each 2H, J=8.5Hz), 4.18 and 4.12(2q, each 2H, J=7.3Hz), 3.75(s, 3H), 1.25 and 1.23(2t, each 3H, J=7.3Hz)

[Reference Example 2] Synthesis of ethyl 4-hydroxy-6-methoxyquinoline-3-carboxylate

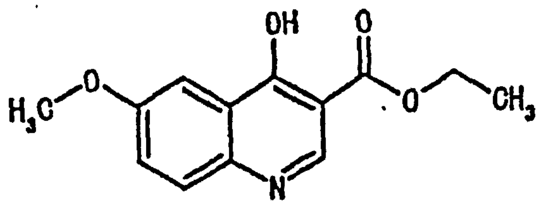

The malonic acid derivative (117 g, 0.4 mol) synthesized in Reference Example 1 was dissolved in diphenyl ether (500 ml). The solution was stirred at 260°C for 1 hour. The resulting material was cooled slowly to room temperature with stirring, to obtain intended crude crystals. The crude crystals were collected by filtration and then subjected to sludging with ethyl acetate (300 ml) to obtain an intended title compound (44.2 g, 45%) as brown crystals.
Melting point = >260°C
1H-N.M.R.(DMSO-d6, 270MHz)d=8.49(s, 1H), 7.59(d, 1H, J=8.8Hz), 7.58(d, 1H, J=2.9Hz), 7.33(dd, 1H, J=2.9, 8.8Hz), 4.24(q, 2H, J=7.1Hz), 3.85(s, 3H), 1.29(t, 3H, J=7.1Hz)

[Reference Example 3] Synthesis of ethyl 4-isopropoxy-6-methoxyquinoline-3-carboxylate

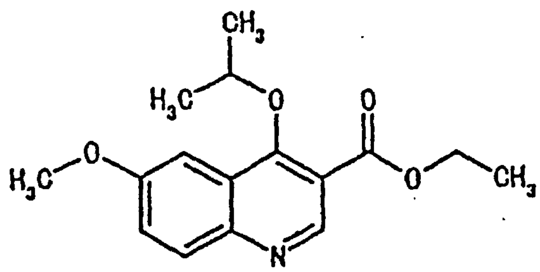

The quinolinol derivative (25 g, 0.1 mol) synthesized in Reference Example 2 was dissolved in methylene chloride (400 ml). Thereto were added, at room temperature, 2-propanol (12.1 g, 0.2 mol), triphenylphosphine (34.3 g, 0.13 mol) and diethyl azodicarboxylate (22.8 g, 0.13 mol). The mixture was stirred at room temperature for 4 hours and then diluted with chloroform (500 ml). The resulting material was washed with water. The organic layer was dried over anhydrous magnesium sulfate and then subjected to vacuum concentration. The residue was purified by silica gel column chromatography (equivalent product of Merck C-300: 500 g, ethyl acetate : hexane = 1 : 2) to obtain an intended title compound (27.5 g, 94%) as a light yellow transparent syrup.
1H-N.M.R.(CDCl3, 270MHz)d=8.90(s, 1H), 7.95(d, 1H, J=8.8Hz), 7.55-7.45(m, 2H), 4.60(quintet, 1H, J=6.3Hz), 4.40 (q, 2H, J=7.3Hz), 3.90(s, 3H), 1.35(t, 3H, J=7.3HZ), 1.30(d, 6H, J=7.3Hz)

[Reference Example 4] Synthesis of 4-isopropoxy-6-methoxyquinoline-3-methanol

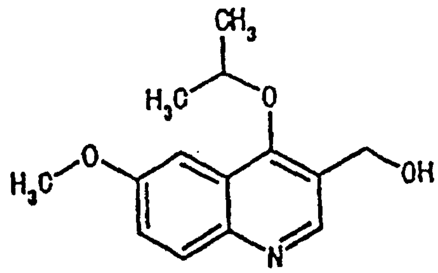

The quinoline derivative (5.0 g, 17.3 mmol) synthesized in Reference Example 3 was dissolved in methylene chloride (150 ml). Thereto was dropwise added a toluene solution containing 1 M diisobutyl aluminum hydride at -60 to -50°C. The mixture was heated to 0°C and stirred for 2 hours. To the reaction mixture was added a saturated aqueous sodium sulfate solution. The resulting precipitate was collected by filtration, followed by washing with ethyl acetate. The filtrate was subjected to vacuum concentration. The residue was purified by silica gel column chromatography (equivalent product of Merck C-300: 120 g, methanol : chloroform = 1 : 20). Successively, crystallization was conducted using a small amount of a mixed solvent consisting of ethyl acetate and hexane, to obtain an intended title compound (4.05 g, 95%) as light yellow crystals.
Melting point = 66 to 68°C
1H-N.M.R.(CDCl3, 270MHz)d=8.75(s, 1H), 7.95(d, 1H, J=8.5Hz), 7.35-7.25(m, 2H), 4.88(s, 2H), 4.62(quintet, 1H, J=6.5Hz), 4.40(q, 2H, J=7.3Hz), 3.93(s, 3H), 1.42(d, 6H, J=7.3Hz)

[Reference Example 5] Synthesis of ethyl 3-(4-benzyloxyphenyl)-2-ethoxy-3-hydroxypropanaote

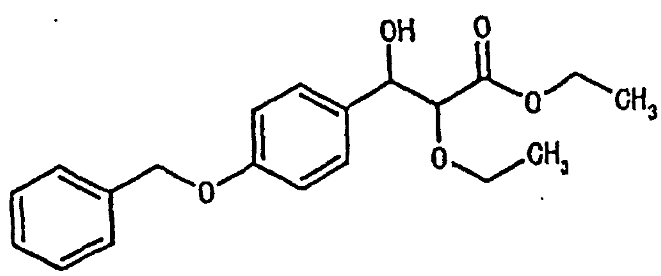

Diisopropylamine (6.07 g, 60 mmol) was dissolved in tetrahydrofuran (120 ml). Thereto was dropwise added a hexane solution of 1.5 M n-butyl lithium (40 ml, 60 mmol) slowly at -60°C. The mixture was heated to 0°C, stirred for 20 minutes, and cooled again to -60°C. Thereto was dropwise added slowly a solution of ethyl ethoxyacetate (7.93 g, 60 mmol) dissolved in tetrahydrofuran (40 ml). The mixture was stirred at the same temperature for 1 hour. Then was dropwise added a solution of 4-benzyloxybenzaldehyde (10.6 g, 50 mmol) dissolved in tetrahydrofuran (80 ml). The mixture was stirred at the same temperature for 1 hour. To the reaction mixture was added a saturated aqueous ammonium chloride solution (250 ml) and the mixture was heated to room temperature. Extraction of an intended compound was conducted using ethyl acetate. The organic layer was washed with water, dried over anhydrous magnesium sulfate, and subjected to vacuum concentration. The residue was purified by silica gel column chromatography (NHDM-1020 produced by Fuji Silysia Chemical Ltd.: 250 g, ethyl acetate : hexane = 1 : 2) to obtain an intended title compound (14.6 g, 85%) as a colorless transparent syrup-like diastereomer mixture.
1H-N.M.R.(CDCl3, 270MHz)d=7.90-7.26(m, 7H), 6.94(d, 2H, J=9.8Hz), 5.06(s, 2H), 4.90-4.85(m, 0.75H), 4.80-4.75 (m, 0.25H), 4.14-3.90(m, 3H), 3.75-3.40(m, 2H), 2.98(d, 0.25H, J=3.9Hz), 2.90(d, 0.75H, J=3.9Hz), 1.26-1.05(m, 6H)

[Reference Example 6] Synthesis of ethyl 3-(4-benzyloxyphenyl)-2-ethoxypropanoate

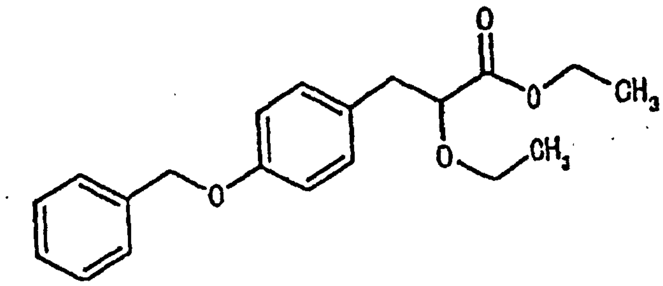

The alcohol compound (13.0 g, 37.7 mmol) obtained in Reference Example 5 was dissolved in methylene chloride (120 ml). Thereto were added trifluoroacetic acid (30 ml) and triethylsilane (13.2 g, 113 mmol) at room temperature. The mixture was stirred at room temperature for 4 hours and then poured into a saturated aqueous sodium bicarbonate solution. The organic layer was separated, dried over anhydrous magnesium sulfate and subjected to vacuum concentration. The residue was purified by silica gel column chromatography (equivalent product of Merck C-300: 200 g, ethyl acetate : hexane = 1 : 4) to obtain an intended title compound (11.5 g, 92%) as a colorless transparent syrup.
1H-M.M.R.(CDCl3, 270MHz)d=7.45-7.25(m, 5H), 7.16 and 6.89(2d, each 2H, J=8.5Hz), 5.04(s, 2H), 4.16(q, 2H, J=7.3Hz), 3.97(t, 1H, J=6.3Hz), 3.63-3.57(m, 1H), 3.38-3.32(m, 1H), 2.95(d, 2H, J=6.6Hz), 1.67(t, 3H, J=7.7Hz), 1.22 (t, 3H, J=7.7Hz)

[Reference Example 7] Synthesis of ethyl 2-ethoxy-3-(4-hydroxyphenyl)propanoate

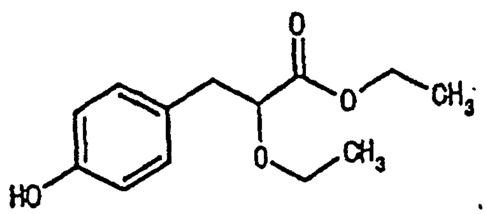

The ester compound (11.0 g, 33.3 mmol) obtained in Reference Example 6 was dissolved in ethanol (159 mmol). Thereto was added 10% palladium-carbon (containing 50% of water) (2.2 g, 1 wt. % as Pd). The mixture was stirred vigorously in a hydrogen atmosphere at normal pressure at room temperature for 3 hours. The catalyst was removed from the reaction mixture by filtration using Celite. The filtrate was subjected to vacuum concentration to obtain an intended title compound (7.06 g, 89%) as a crude syrup.
1H-N.M.R.(CDCl3, 270MHz)d=7.08 and 6.73(2d, each 2H, J=8.6Hz), 4.17(q, 2H, J=7.0Hz), 3.99(t, 1H, J=6.6Hz), 3.66-3.55(m, 1H), 3.43-3.32(m, 1H), 2.95(d, 2H, J=6.6Hz), 1.23(t, 3H, J=7.7Hz), 1.17(t, 3H, J=7.7Hz)

[Reference Example 8] Synthesis of ethyl 4-isopropoxy-5,7-dimethylquinoline-3-carboxylate

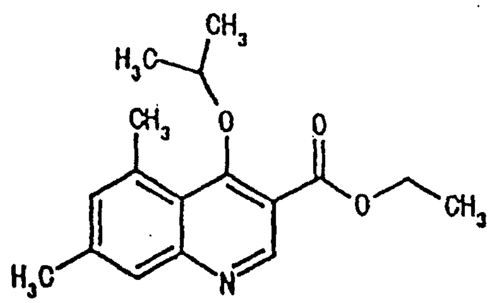

3,5-Dimethylaniline (12.1 g, 0.1 mol) and diethyl ethoxymethylenemalonate (21.6 g, 0.1 mol) were used as starting materials and subjected to the same treatment as in Reference Examples 1 to 3 to obtain an intended title compound (10.1 g, 35%) as a brown syrup.
1H-N.M.R.(CDCl3, 270MHz)d=9.04(s, 1H), 7.68(s, 1H), 7.15(s, 1H), 4.61(quintet, 1H, J=6.3Hz), 4.44(q, 2H, J=7.0Hz), 2.89 and 2.49(2s, each 3H), 1.44(t, 3H, J=7.0HZ), 1.33(d, 6H, J=6.3Hz)

[Reference Example 9] Synthesis of 4-isopropoxy-5,7-dimethylquinoline-3-methanol

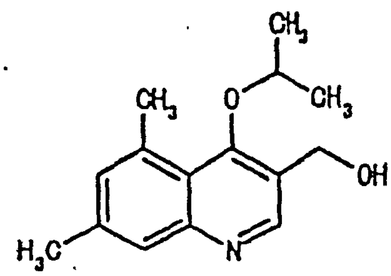

The ester compound (3.5 g, 12.2 mmol) obtained in Reference Example 8 was subjected to the same treatment as in Reference Example 4 to obtain an intended title compound (2.24 g, 75%) as a light yellow syrup.
1H-N.M.R.(CDCl3, 270MHz)d=8.72(s, 1H), 7.64(s, 1H), 7.10(s, 1H), 4.85(s, 2H), 4.54(quintet, 1H, J=6.3Hz), 2.82 and 2.45(2s, each 3H), 1.29(d, 6H, J=6.3Hz)

[Reference Example 10] Synthesis of ethyl 6-ethyl-4-isopropoxyquinoline-3-carboxylate

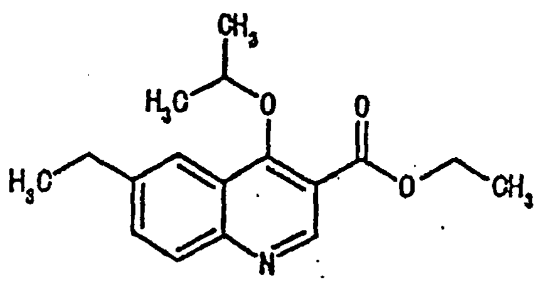

4-Ethylaniline (12.1 g, 0.1 mol) and diethyl ethoxymethylenemalonate (21.6 g, 0.1 mol) were used as starting materials and subjected to the same treatment as in Reference Examples 1 to 3 to obtain an intended title compound (13.5 g, 55%) as a brown syrup.
1H-N.M.R.(CDCl3, 270MHz)d=9.14(s, 1H), 8.06(d, 1H, J=1.9Hz), 8.00(d, 1H, J=8.6Hz), 7.64(dd, 1H, J=1.9, 8.6Hz), 4.69(quintet, 1H, J=6.3Hz), 4.46(q, 2H, J=7.3Hz), 2.86(q, 2H, J=7.5Hz), 1.45(t, 3H, J=7.3Hz), 1.39(d, 6H, J=6.3Hz), 1.34(t, 3H, J=7.5Hz)

[Reference Example 11] Synthesis of 6-ethyl-4-isopropoxyquinoline-3-methanol

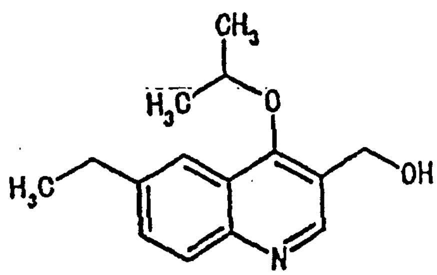

The ester compound (4.91 g, 20 mmol) obtained in Reference Example 10 was subjected to the same treatment as in Reference Example 4 to obtain an intended title compound (3.53 g, 72%) as light yellow crystals.
Melting point = 43 to 46°C
1H-N.M.R.(CDCl3, 270MHz)d=8.80(s, 1H), 7.98(d, 1H, J=1.5Hz), 7.84(d, 1H, J=8.6Hz), 7.54(dd, 1H, J=1.5, 8.6Hz), 4.90(s, 2H), 4.65(quintet, 1H, J=6.0Hz), 2.85(q, 2H, J=7.6Hz), 1.40(d, 6H, J=6.0Hz), 1.34(t, 3H, J=7.6Hz)

[Reference Example 12] Synthesis of ethyl 6-methoxy-4-octyloxyquinoline-3-carboxylate

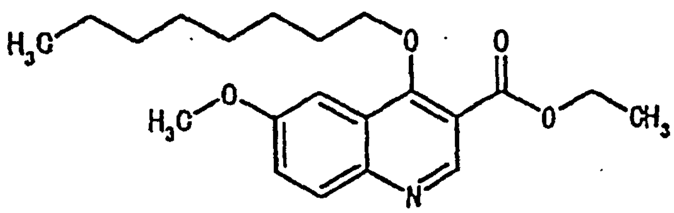

The quinolinol derivative (2.47 g, 10 mmol) synthesized in Reference Example 2 was dissolved in methylene chloride (70 ml). Thereto were added, at room temperature, 1-octanol (3.26 g, 25 mmol), triphenylphosphine (3.93 g, 15 mmol) and diethyl azodicarboxylate (2.61 g, 15 mmol). The mixture was stirred at room temperature for 4 hours and then diluted with chloroform (200 ml). The resulting material was washed with water. The organic layer was dried over anhydrous magnesium sulfate and then subjected to vacuum concentration. The residue was purified by silica gel column chromatography (equivalent product of Merck C-300: 100 g, ethyl acetate : hexane = 1 : 5) to obtain an intended title compound (2.82 g, 79%) as a light yellow transparent syrup.
1H-N.M.R.(CDCl3, 270MHz)d=9.03(s, 1H), 7.99(d, 1H, J=9.2Hz), 7.53(d, 1H, J=2.5Hz), 7.43(dd, 1H, J=2.5, 9.2Hz), 4.46(q, 2H, J=7.3Hz), 4.23(t, 2H, J=7.2Hz), 3.95(s, 3H), 1.98-1.90(m, 2H), 1.60-1.50(m, 2H), 1.30-1.20(m, 11H), 0.88 (t, 3H, J=6.6Hz)

[Reference Example 13] Synthesis of 6-methoxy-4-octyloxyquinoline-3-methanol

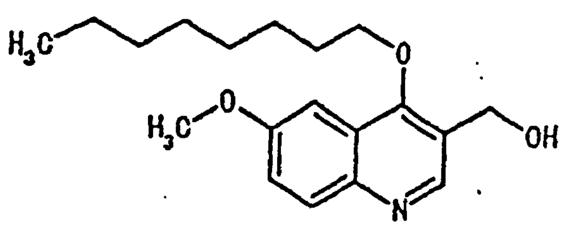

The ester compound (2.6 g, 7.23 mmol) obtained in Reference Example 12 was subjected to the same treatment as in Reference Example 4 to obtain an intended title compound (1.25 g, 54%) as a light yellow syrup.
1H-N.M.R.(CDCl3, 270MHz)d=8.81(s, 1H), 8.00(d, 1H, J=9.2Hz), 7.40-7.35(m, 2H), 4.88(s, 2H), 4.20(t, 2H, J=7.0Hz), 3.95(s, 3H), 2.00-1.90(m, 2H), 1.60-1.50(m, 2H), 1.30-1.15(m, 8H), 0.88(t, 3H, J=6.6Hz)

[Reference Example 14] Synthesis of 6,7-dimethoxy-4-isopropoxyquinoline-3-methanol

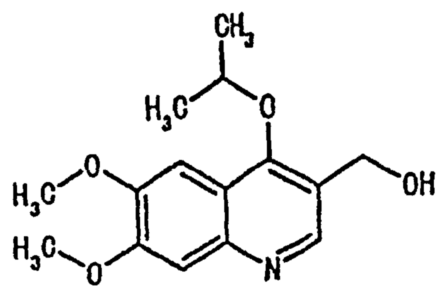

3,5-Dimethoxylaniline (15.3 g, 0.1 mol) and diethyl ethoxymethylenemalonate (21.6 g, 0.1 mol) were used as starting materials and subjected to the same treatment as in Reference Examples 1 to 3 to obtain an intended title compound (7.98 g, 25%) as a brown crude syrup. The ester obtained (2.00 g, 6.26 mmol) was subjected to the same treatment as in Reference Example 4 to obtain an intended title compound (1.33 g, 77%) as a light yellow syrup. 1H-N.M.R.(CDCl3, 270MHz)d=8.77(s, 1H), 7.41(s, 1H), 7.37(s, 1H), 4.88(s, 2H), 4.62(quintet, 1H, J=6.5Hz), 4.04 and 3.98(2s, each 3H), 1.40(d, 6H, J=6.5Hz)

[Reference Example 15] Synthesis of 2-phenylquinoline-4-methanol

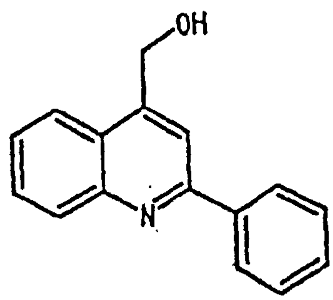

Methyl 2-phenylquinoline-4-carboxylate (3.0 g) was subjected to the same treatment as in Reference Example 3 to obtain an intended title compound (1.93 g, 72%). 1H-N.M.R.(CDCl3, 270MHz)d=8.22-8.11(m, 3H), 7.96-7.90(m, 2H), 7.73(t, 1H, J=8.6Hz), 7.57-7.42(m, 4H), 5.23(s, 2H)

[Reference Example 16] Synthesis of quinoline-6-methanol

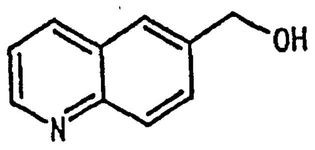

Ethyl quinoline-6-carboxylate (1.78 g) was subjected to the same treatment as in Reference Example 3 to obtain an intended title compound (1.31 g, 93%).

1H-N.M.R.(CDCl3, 270MHz)d=8.88(d, 1H, J=4.0Hz), 8.20-8.06(m, 2H), 7.81(s, 1H), 7.70(d, 1H, J=8.6Hz), 7.40(d, 1H, J=8.6Hz), 4.91(s, 2H)

[Reference Example 17] Synthesis of methyl 4-methoxyquinoline-2-carboxylate

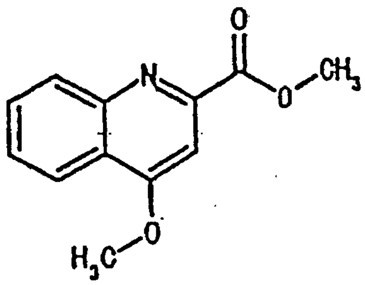

4-Methoxyquinoline-2-carboxylic acid (2.42 g) was dissolved in 13% hydrochloric acid-methanol (45 ml). The solution was refluxed for 8 hours, with heating. The reaction mixture was subjected to vacuum distillation. To the residue were added a saturated aqueous sodium bicarbonate solution and ethyl acetate. The ethyl acetate layer was separated, dried over anhydrous magnesium sulfate, and subjected to vacuum concentration to obtain an intended title compound (1.08 g, 42%) as a light brown syrup.
1H-N.M.R.(CDCl3, 270MHz)d=8.24(d, 2H, J=8.6Hz), 7.77(t, 1H, J=6.9Hz), 7.64-7.58(m, 2H), 4.14(s, 3H), 4.09(s, 3H)

[Reference Example 18] Synthesis of 4-methoxyquinoline-2-methanol

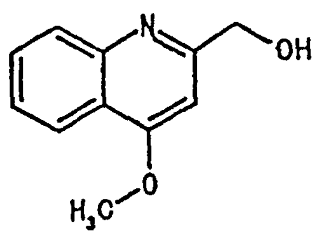

The ester compound (500 mg) obtained in the synthesis of Reference Example 17 was subjected to the same treatment as in Reference Example 3 to obtain an intended title compound (340 mg, 78%) as a colorless transparent syrup.
1H-N.M.R.(CDCl3, 270MHz)d=8.17(d, 1H, J=8.3Hz), 7.99(d, 1H, J=8.3Hz), 7.70(t, 1H, J=6.9Hz), 7.49(t, 1H, J=6.9Hz), 6.62(s, 1H), 4.86(s, 2H), 4.05(s, 3H)

[Reference Example 19] Synthesis of 4-isopropyl-7-trifluoromethylquinoline-3-methanol

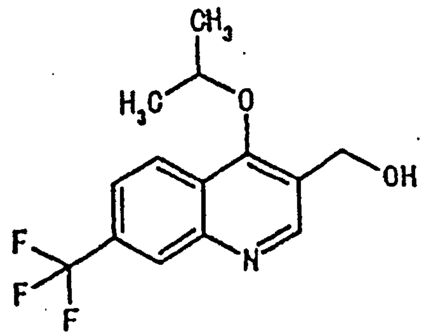

Ethyl 4-isopropyl-7-trifluoromethylquinoline-3-carboxylate (4.73 g) was subjected to the same treatment as in Reference Example 3 to obtain an intended title compound (570 mg, 14%) as a colorless transparent syrup.
1H-N.M.R.(CDCl3, 270MHz)d=9.02(s, 1H), 8.38(s, 1H), 8.22(d, 1H, J=8.9Hz), 7.70(dd, 1H, J=1.7, 8.6Hz), 4.96(s, 2H), 4.66(m, 1H), 1.43(d, 6H, J=5.9Hz)

[Reference Example 20] Synthesis of ethyl 8-chloro-4-isopropoxyquinoline-3-carboxylate

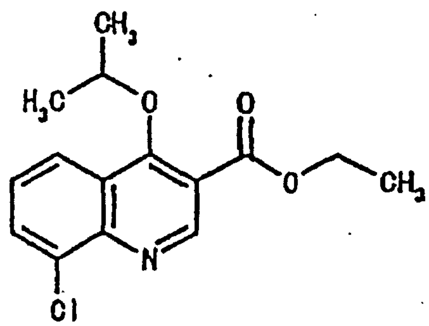

Ethyl 8-chloro-4-hydroxyquinoline-3-carboxylate (5.22 g, 22.2 mmol) was subjected to the same treatment as in Reference Example 3 to obtain an intended title compound (3.32 g, 92%) as a colorless transparent oil.
1H NMR (CDCl3, 270MHz)) d=9.27(1H, d, J=3.2Hz), 8.20(1H, d, J=2.2Hz), 7.87(1H, d, J=2.2Hz), 4.75(1H, sept, J=5.9Hz), 4.48(2H, dd, J=14, 7.2Hz), 1.45(3H, t, J=7.2Hz), 1.39(6H, d, J=5.9Hz)

[Reference Example 21] 8-Cloro-4-isopropoxyquinoline-3-methanol

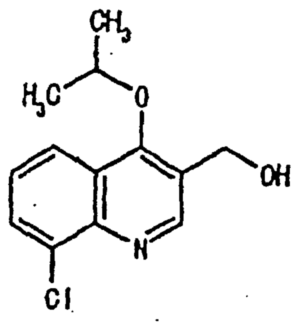

The ester compound (3.28 g, 11.2 mmol) synthesized in Reference Example 20 was subjected to the same treatment as in Reference Example 4 to obtain an intended title compound (2.25 g, 79%) as white crystals.
Melting point = 88 to 89°C
1H NMR (CDCl3, 270MHz)d=9.02(1H, s), 8.01(1H, dd, J=8.6, 1.4Hz), 7.80(1H, dd, J=7.3, 1.4Hz), 7.43(1H, dd., J=8.6, 7.3Hz), 4.92(2H, brd.s), 4.63(1H, sept, J=6.2Hz), 2.79(1H, brd.s), 1.39(6H, d, J=6.2Hz)

[Reference Example 22] Ethyl 4-isopropoxy-8-trifluoromethylquinoline-3-carboxylate

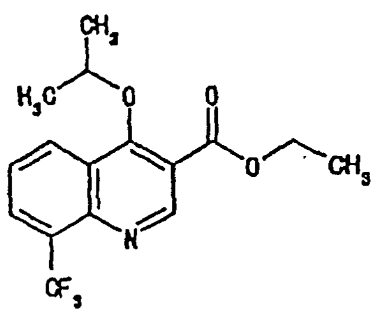

Ethyl 4-hydroxy-8-trifluoromethylquinoline-3-carboxylate (2.11 g, 7.4 mmol) was subjected to the same treatment as in Reference Example 3 to obtain an intended title compound (2.24 g, 92%) as a colorless transparent oil.
1H NMR (CDCl3, 270MHz) d=9.37(1H, d, J=3.5Hz), 8.54(1H, dd, J=8.6, 0.8Hz), 8.14(1H, dd, J=7.3, 0.8Hz), 7.62(1H, dd., J=8.6, 7.3Hz), 4.77(1H, sept, J=6.2Hz), 4.48(2H, dd, J=14, 7.3Hz), 1.45(3H, t, J=7.3Hz), 1.40(6H, d, J=6.2Hz)

[Reference Example 23] 4-Isopropoxy-8-trifluoromethylquinoline-3-methanol

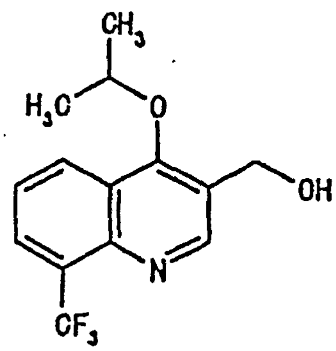

The ester compound (1.24 g, 3.8 mmol) synthesized in Reference Example 22 was subjected to the same treatment as in Reference Example 4 to obtain an intended title compound (0.91 g, 84%) as white crystals.
Melting point = 73 to 74°C
1H NMR (CDCl3, 270MHz) d=9.07(1H, s), 8.32(1H, dd, J=8.6, 0.5Hz), 8.05(1H, dd, J=7.3, 0.5Hz), 7.57(1H, t, J=8.. 0Hz), 4.93(2H, d, J=5.8Hz), 4.64(1H, sept, J=6.0Hz), 2.39(1H, t, J=5.8Hz), 1.41(6H, d, J=6.0Hz)

[Reference Example 24] Ethyl 8-fluoro-4-isopropoxyquinoline-3-carboxylate

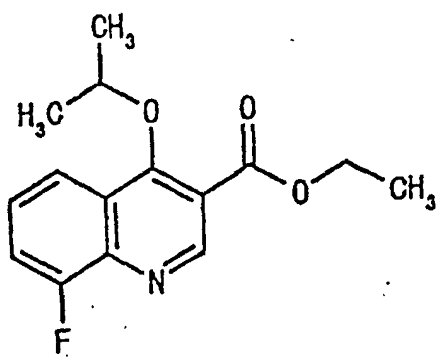

Ethyl 8-fluoro-4-hydroxyquinoline-3-carboxylate (5.22 g, 22.2 mmol) was subjected to the same treatment as in Reference Example 3 to obtain an intended title compound (5.76 g, 94%) as a light yellow oil.
1H NMR (CDCl3, 270MHz) d=9.23(1H, s), 8.14-7.98(1H, m), 7.55-7.45(2H, m), 4.74(1H, sept, J=6.2Hz), 4.48(2H, dd, J=15, 7.2Hz), 1.46(3H, t, J=7.2Hz), 1.39(6H, d, J=6.2Hz)

[Reference Example 25] 8-Fluoro-4-isopropoxyquinoline-3-methanol

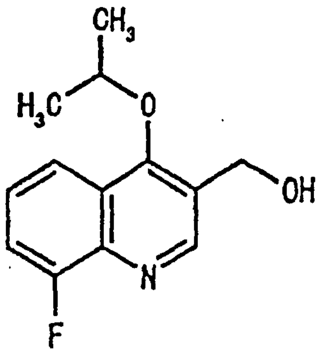

The ester compound (2.00 g, 7.2 mmol) synthesized in Reference Example 24 was subjected to the same treatment as in Reference Example 4 to obtain an intended title compound (1.19 g, 71%) as white crystals.
1H NMR (CDCl3, 270MHz) d=8.95(1H, s), 7.65(1H, dt, J=7.8, 1.6Hz), 7.45(1H, m), 7.38(1H, ddd, J=9.0, 2.7, 1.6Hz), 4.93(2H, s), 4.65(1H, sept, J=6.2Hz), 3.51(1H, brd.s), 1.39(6H, d, J=6.2Hz)

[Reference Example 26] Ethyl quinoline-8-carboxylate

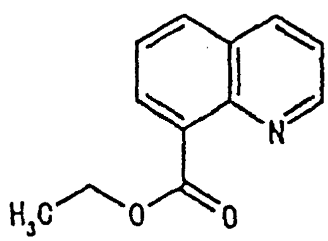

Quinoline-8-carboxylic acid (1.43 g, 8.3 mmol) was dissolved in ethanol (40 ml). Thereto was added sulfuric acid (0.3 ml), followed by refluxing for 12 hours with heating. The most part of ethanol was removed from the reaction mixture by vacuum distillation. To the residue was added a saturated aqueous sodium bicarbonate solution. Extraction of an intended compound was conducted using ethyl acetate. The organic layer was dried over anhydrous magnesium sulfate and then subjected to vacuum concentration to obtain an intended title compound (0.56 g, 34%) as a yellow oil.
1H NMR (CDCl3, 270MHz) d=9.05(1H, dd, J=4.1, 1.6Hz), 8.18(1H, dd, J=8.4, 1.6Hz), 8.02(1H, dd, J=7.2. 1.6Hz), 7.93 (1H, dd, J=8.4, 1.6 Hz), 7.56(1H, dd, J=8.4, 7.2Hz), 7.45(1H, dd, J=8.4, 4.1Hz), 4.54(2H, dd, J=15, 7.3Hz), 1.45(3H, t, J=7.3Hz)

[Reference Example 27] Quinoline-8-methanol

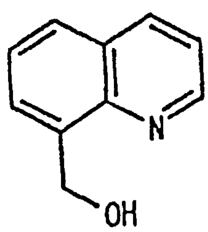

The ester compound (0.53 g, 2.6 mmol) synthesized in Reference Example 26 was subjected to the same treatment as in Reference Example 4 to obtain an intended title compound (0.34 g, 81%) as white crystals.
Melting point = 74 to 75°C
1H NMR (CDCl3, 270MHz) d=8.88(1H, dd, J=4.3, 1.9Hz), 8.20(1H, dd, J=8.5, 1.6Hz), 7.76(1H, dd, J=8.1, 1.6Hz), 7.59 (1H, dd, J=6.8, 0.8Hz), 7.51(1H, d, J=8.1Hz), 7.45(1H, dd, J=8.1, 4.3Hz), 5.21(2H, brd.s), 5.10(1H, brd.s)

[Reference Example 28] Synthesis of methyl 3-(4-benzyloxyphenyl)-3-hydroxy-2-phenoxypropanoate

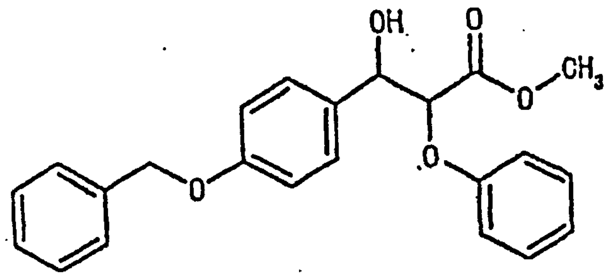

4-Benzyloxybenzaldehyde (3.35 g, 15.8 mmol) and methyl phenoxyacetate (2.62 g, 15.8 mmol) were subjected to the same treatment as in Reference Example 5 to obtain an intended title compound (2.57 g, 43%) as a colorless transparent syrup-like diastereomer mixture.
1H-N.M.R.(CDCl3, 270MHz)d=7.44-7.31(m, 9H), 7.00-6.80(m, 5H), 5.17-5.10(m, 1H), 5.06(s, 2H), 4.76((d, 0.7H, 3=6.0Hz), 4.70(d, 0.3Hz, J=5.6Hz), 3.69(s, 2.1Hz), 3.61(s, 0.9Hz), 2.98(d, 0.3Hz, J=5.0Hz), 2.81(d, 0.7Hz, J=4.3Hz)

[Reference Example 29] Synthesis of methyl 3-(4-benzyloxyphenyl)-2-phenoxypropanoate

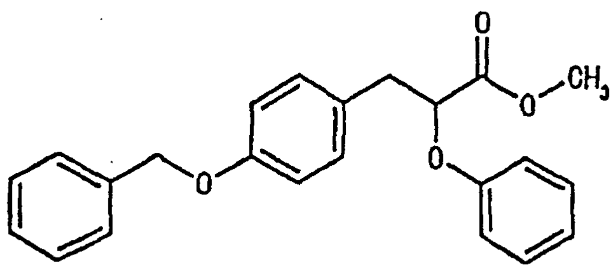

The alcohol compound (2.50 g, 6.61 mmol) synthesized in Reference Example 28 was subjected to the same treatment as in Reference Example 6 to obtain an intended title compound (2.2 g, 92%) as a colorless transparent syrup.
1H-N.M.R.(CDCl3, 270MHz)d=7.43-7.20(m, 9H), 6.98-6.81(m, 5H), 5.03(s, 2H), 4.76(dd, 1H, J=5.6, 7.7Hz), 3.70(s, 3H), 3.20-3.17(m, 2H)

[Reference Example 30] Synthesis of methyl 3-(4-hydroxyphenyl)-2-phenoxypropanoate

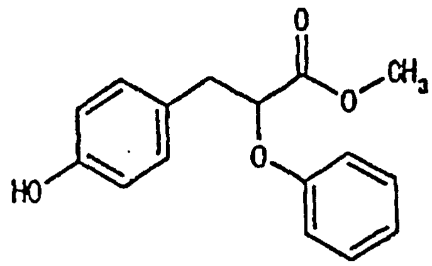

The ester compound (2.2 g, 6.07 mmol) synthesized in Reference Example 29 was subjected to the same treatment as in Reference Example 7 to obtain an intended title compound (1.57 g, 95%) as white crystals.
Melting point = 103 to 105°C
1H-N.M.R.(CDCl3, 270MHz)d=7.27-7.14(m, 4H), 6.95(t, 1H, J=8.6Hz), 6.83(dd, 2H, J=1.0, 8.6Hz), 6.75(d, 2H, J=8.6Hz), 4.76(dd, 1H, J=5.6, 7.2Hz), 3.71(s, 3H), 3.19-3.15(m, 2H)

[Reference Example 31] Synthesis of 4-methoxymehtyloxybenzaldehyde

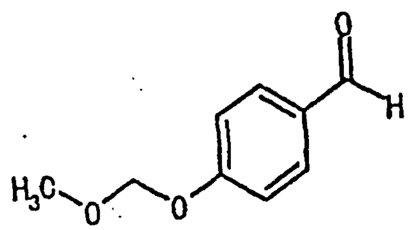

4-hydroxybenzaldehyde (25.3 g, 0.21 mol) was dissolved in methylene chloride (250 ml). Thereto were added chloromethyl methyl ether (25.0 g, 0.41 mol) and diisopropylethylamine (54 g, 0.41 mol). The mixture was stirred at room temperature for 12 hours. The reaction mixture was poured into water (300 ml). Chloroform (200 ml) was added. The organic layer was separated, dried over anhydrous magnesium sulfate, and subjected to vacuum concentration. The residue was purified by silica gel column chromatography (equivalent product of Merck C-300: 250 g, ethyl acetate : hexane = 1 : 4) to obtain an intended title compound (35 g, 100%) as a light brown syrup.
1H-N.M.R.(CDCl3, 270MHz)d=9.90(s, 1H), 7.83 and 7.14(2d, each 2H, J=8.6Hz), 5.26(s, 2H), 3.49(s, 3H)

[Reference Example 32] Synthesis of ethyl 3-(4-methoxymethyloxyphenyl)-2-phenylthiopropenoate

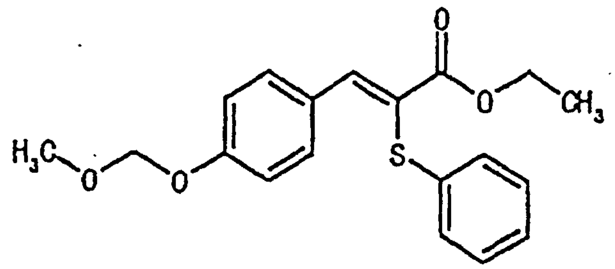

The aldehyde compound (4.99 g, 30 mmol) synthesized in Reference Example 31 and ethyl phenylthioacetate (4.03 g, 30 mmol) were dissolved in ethanol (60 ml). Thereto was added a 1 N ethanol solution (30 ml) of sodium ethoxide at room temperature. The mixture was heated to 60°C and stirred for 6 hours. The reaction mixture was allowed to cool and diluted with water (200 ml). Extraction of an intended compound was conducted using ethyl acetate. The organic layer was dried over anhydrous magnesium sulfate and subjected to vacuum concentration. The residue was purified by silica gel column chromatography (NHDM-1020 produced by Fuji Silysia Chemical Ltd.: 100 g, ethyl acetate : hexane =1 : 6) to obtain an intended title compound (2.78 g, 27%) as a light yellow syrup.
1H-N.M.R.(CDCl3, 270MHz)d=8.09(s, 1H), 7.87 and 7.05(2d, each 2H, J=8.5Hz), 7.30-7.20(m, 5H), 5.21(s, 2H), 4.11 (q, 2H, J=7.3Hz), 3.47(s, 3H), 1.06(t, 3H, 7.3Hz)

[Reference Example 33] Synthesis of ethyl 3-(4-hydroxyphenyl)-2-phenylthiopropenoate

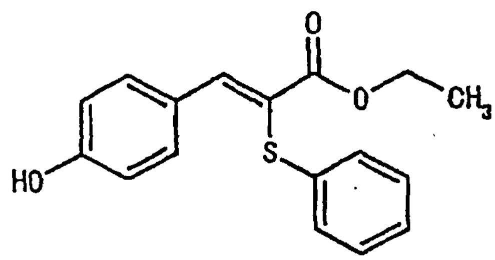

The ester compound (1.0 g, 2.90 mmol) synthesized in Reference Example 32 was dissolved in methylene chloride (15 ml). Thereto was added a 4 N hydrochloric acid-dioxane solution (5 ml) at room temperature. The mixture was stirred for 2.5 hours. The reaction mixture was poured into a saturated aqueous sodium bicarbonate solution. Thereto was added chloroform. The organic layer was separated and the reaction mixture was subjected to vacuum concentration to obtain an intended title compound (760 mg, 87%) as a light yellow syrup. 1H-N.M.R.(CDCl3, 270MHz) d=8.11(s, 1H), 7.86 and 6.86(2d, each 2H, J=8.5Hz), 7.20-7.10(m, 5H), 4.12(q, 2H, J=6.9Hz), 1.08(t, 3H, 6.9Hz)

[Reference Example 34] Synthesis of methyl 3-(4-methoxymethyloxyphenyl)-2-phenylthiopropanoate

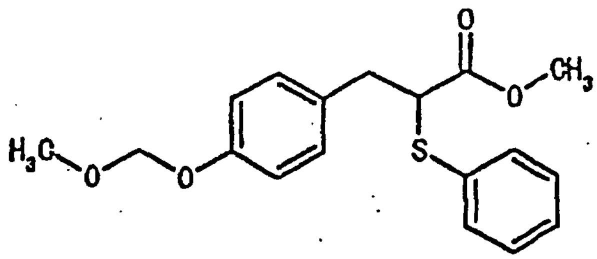

The ester compound (1.72 g, 5 mmol) synthesized in Reference Example 32 was dissolved in methanol (50 ml). Thereto were added magnesium chips (2.43 g, 0.1 mol) at room temperature. The mixture was stirred for 5 hours. The unnecessary matter was removed by filtration. The filtrate was subjected to vacuum concentration. The residue was purified by silica gel column chromatography (equivalent product of Merck C-300: 80 g, ethyl acetate : hexane =1 : 10) to obtain an intended title compound (740 g, 45%) as a light yellow syrup.
1H-N.M.R.(CDCl3, 270MHz)d=7.45-7.41(m, 2H), 7.32-7.26(m, 3H), 7.10 and 6.94(2d, each 2H, J=8.9Hz), 5.14(s, 2H), 3.86(dd, 1H, J=6.6, 9.2Hz), 3.59 and 3.47(2s, each 3H), 3.14(dd, 1H, J=6.6, 12.8Hz), 3.00(dd, 1H, J=9.2, 12.8Hz)

[Reference Example 35] Synthesis of methyl 3-(4-hydroxyphenyl)-2-phenylthiopropanoate

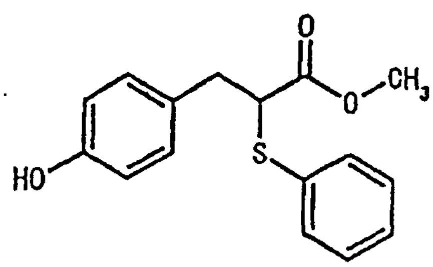

The methyl ester compound (760 mg, 2.29 mmol) synthesized in Reference Example 34 was treated in the same manner as in Reference Example XX to obtain an intended title compound (660 mg, 100%) as a light yellow syrup.
1H-N.M.R.(CDCl3, 270MHz)d=7.45-7.40(m, 2H), 7.33-7.24(m, 3H), 7.04 and 6.73(2d, each 2H, J=8.6Hz), 4.99(s, 1H), 3.84(dd, 1H, J=6.6, 9.2Hz), 3.58(s, 3H), 3.12(dd, 1H, J=9.2, 12.8Hz), 2.99(dd, 1H, J=6.6, 12.8Hz)

[Reference Example 36] Synthesis of ethyl 2-ethoxy-3-(3-hydroxyphenyl)propanoate

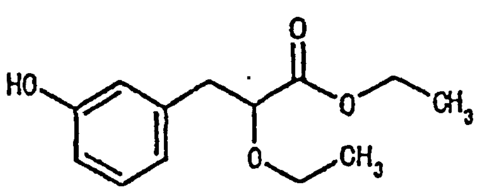

3-Benzyloxybenzaldehyde (8.49 g, 40 mmol) was used as a starting material and the same operation as in

Reference Examples 5 to 7 was used, whereby an intended title compound (2.0 g, 21%) was obtained as a colorless transparent syrup.
1H-N.M.R.(CDCl3, 270MHz)d=7.15(t, 1H, J=8.0Hz), 6.82-6.69(m, 3H), 5.36(s, 1H), 4.18(q, 2H, J=7.3Hz), 4.02(t, 1H, J=6.9Hz), 3.64-3.56(m, 1H), 3.43-3.34(m, 1H), 2.96(d, 2H, J=6.9Hz), 1.23(t, 3H, J=7.3Hz), 1.16(t, 3H, J=7.3Hz)

[Reference Example 37] Synthesis of ethyl 2-ethoxy-3-(3-hydroxy-4-methoxyphenyl)propanoate

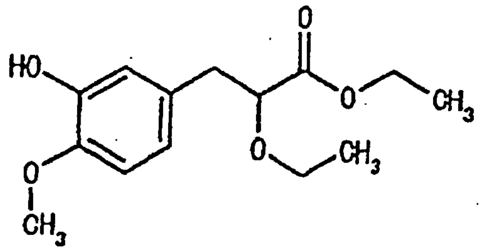

3-Benzyloxy-4-methoxybenzaldehyde (6.06 g, 25 mmol) was used as a starting material and the same operation as in Reference Examples 5 to 7 was used, whereby an intended title compound (4.56 g, 68%) was obtained as a colorless transparent syrup.
1H-N.M.R.(CDCl3, 270MHz)d=7.25(d, 1H, J=8.2Hz), 6.84-6.80(m, 2H), 4.14(q, 2H, J=7.1Hz), 3.92(t, 1H, J=6.9Hz), 3.86(s, 3H), 3.33-3.27(m, 1H), 3.43-3.34(m, 1H), 2.88(d, 2H, J=6.9Hz), 1.23(t, 3H, J=7.3Hz), 1.12(t, 3H, J=7.3Hz)

[Reference Example 38] Synthesis of dimethyl (4-hydroxyphenyl)methylenemalonate

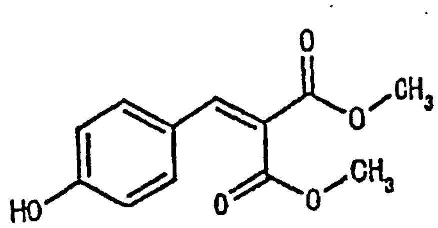

4-Hydroxybenzaldehyde (12.2 g, 0.1 mol) and dimethyl malonate (13.2 g, 0.1 mol) were suspended in benzene (100 ml). Thereto was added piperidine (1 ml). The mixture was refluxed for 4 hours with heating and then allowed to cool. The resulting crystals were collected by filtration and washed with toluene to obtain an intended title compound (17.7 g, 75%) as milky-white crystals.
Melting point = 155 to 157°C
lH-N.M.R.(CDCl3/DMSO-d6, 270MHz)d=9.25(bs, 1H), 7.67(s, 1H), 7.29 and 6.84(2d, each 2H, J=8.6Hz), 3.86 and 3.82(2s, each 3H)

[Reference Example 39] Synthesis of dimethyl (4-hydroxyphenyl)methylmalonate

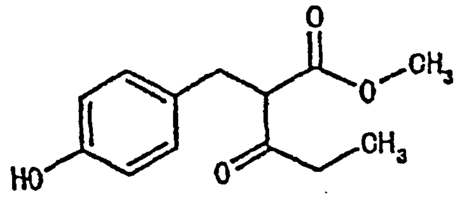

The olefin compound (11.8 g, 50 mmol) synthesized in Reference Example 38 was dissolved in methanol (200 ml). Thereto was added a 10% palladium carbon powder (water content: 50%) (2.4 g, 1 wt. % as Pd). The mixture was stirred vigorously in a hydrogen atmosphere at normal pressure at room temperature for 1.5 hours. The unnecessary matter was removed by filtration using Celite. The filtrate was subjected to vacuum distillation to obtain an intended title compound (12.0 g, 100%) as a colorless transparent syrup.
1H-N.M.R.(CDCl3, 270MHz)d=7.02 and 6.70(2d, each 2H, J=8.5Hz), 4.90(s, 1H), 3.68(s, 6H), 3.65(t, 1H, J=7.0Hz), 3.10(d, 2H, J=7.0Hz)

[Reference Example 40] Synthesis of methyl 2-(4-hydroxyphenyl)methyl-3-oxopentanoate

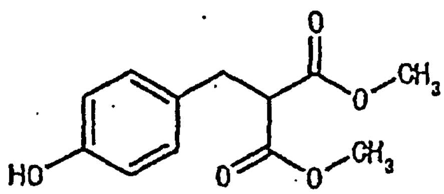

4-Hydroxybenzaldehyde (4.0 g, 33 mmol) and methyl 3-oxopentanoate (4.33 g, 33 mmol) were used as starting materials and the same operation as in Reference Examples 38 and 39 was used, whereby an intended title compound (5.0 g, 61%) was obtained as a colorless transparent syrup.
1H-N.M.R.(CDCl3, 270MHz)d=7.01 and 6.81(2d, each 2H, J=8.4Hz), 5.63(s, 1H), 3.77(t, 1H, J=7.7Hz), 3.68(s, 3H), 3.08(d, 1H, J=7.7Hz), 2.65-2.10(m, 2H), 0.98(t, 3H, J=7.3Hz)

[Reference Example 41] Synthesis of 2-(4-benzyloxyphenyl)methylene-3-oxobutanoic acid N-methylamide

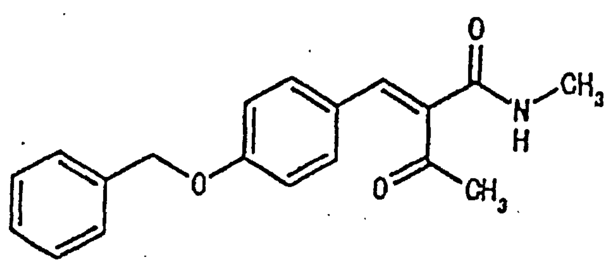

4-Benzyloxybenzaldehyde (7.22 g, 34 mmol) and a 65% aqueous N-methylacetoacetamide solution (6.02 g, 30 mmol) were treated in the same manner as in Reference Example to obtain an intended title compound (3.27 g, 32%) as a light brown syrup.
1H-N.M.R.(CDCl3, 270MHz)d=7.52-7.32(m, 8H), 6.97(d, 2H, J=9.2Hz), 5.83(q, 1H, J=4.9Hz), 5.10(s, 2H), 2.92(d, 3H, J=4.9Hz), 2.41(s, 3H)

[Reference Example 42] Synthesis of 2-(4-hydroxyphenyl)methyl-3-oxobutanoic acid N-methylamide

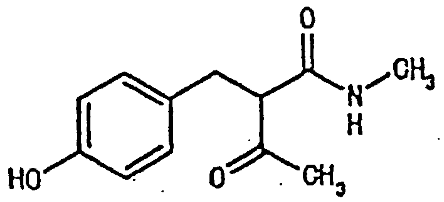

The olefin compound (3.26 g, 10.5 mmol) synthesized in Reference Example 41 was treated in the same manner as in Reference Example 7 to obtain an intended title compound (1.97 g, 85%) as a colorless transparent syrup.
1H-N.M.R.(CDCl3, 270MHz)d=8.53(s, 1H), 6.98 and 6.74(2d, each 2H, J=8.8Hz), 6.75-6.70(m, 1H), 3.57(t, 1H, J=7.5Hz), 3.13-2.97(m, 2H), 2.73(d, 3H, J=5.0Hz), 2.11(s, 3H)

[Reference Example 43] Synthesis of diethyl (3-hydroxyphenyl)methylenemalonate

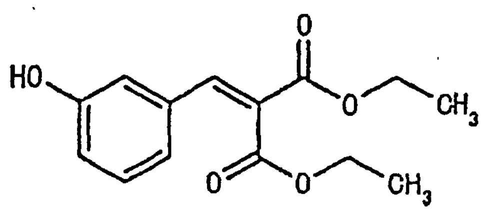

3-Hydroxybenzaldehyde (12.2 g, 0.1 mol) and diethyl malonate (16.0 g, 0.1 mol) were treated in the same manner as in Reference Example 38 to obtain an intended title compound (17.2 g, 65%) as light brown crystals.
Melting point = 72 to 74°C
1H-N.M.R.(CDCl3, 270MHz)d=7.68(s, 1H), 7.22(t, 1H, J=8.0Hz), 6.96(d, 1H, J=8.0Hz), 6.92(s, 1H), 6.90(d, 1H, J=8.0Hz), 6.24(s, 1H), 4.35 and 4.31(2q, each 2H, J=6.9Hz), 1.36-1.24(m, 6H)

[Reference Example 44] Synthesis of diethyl (3-hydroxyphenyl)methylmalonate

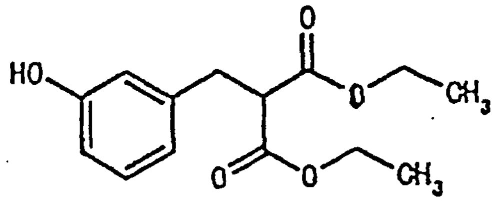

The olefin compound (13.2 g, 50 mmol) synthesized in Reference Example 43 was treated in the same manner as in Reference Example 39 to obtain an intended title compound (13.5 g, 100%) as a colorless transparent syrup.
1H-N.M.R.(CDCl3, 270MHz)d=7.13(t, 1H, J=8.0Hz), 6.76-6.67(m, 3H), 4.16(q, 4H, J=7.3Hz), 3.65(t, 1H, J=8.0Hz), 3.17(d, 2H, J=8.0Hz), 1.21(t, 6H, J=7.3Hz)

[Reference Example 45] Synthesis of diethyl (3-hydroxy-4-methoxyphenyl)methylenemalonate

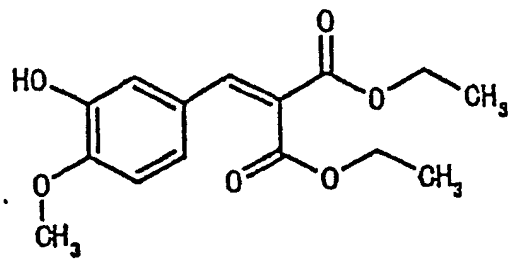

3-Hydroxy-4-methoxybenzaldehyde (7.61 g, 50 mmol) and diethyl malonate (9.61 g, 60 mol) were treated in the same manner as in Reference Example 38 to obtain an intended title compound (11.8 g, 80%) as milky-white crystals.
Melting point = 81 to 83°C
1H-N.M.R.(CDCl3, 270MHz)d=7.61(s, 1H), 7.05-6.99(m, 2H), 6.83(d, 1H, J=8.2Hz), 5.65(s, 1H), 4.37 and 4.29(2q, each 2H, J=7.3Hz), 3.92(s, 3H), 1.36-1.30(m, 6H)

[Reference Example 46] Synthesis of diethyl (3-hydroxy-4-methoxyphenyl)methylmalonate

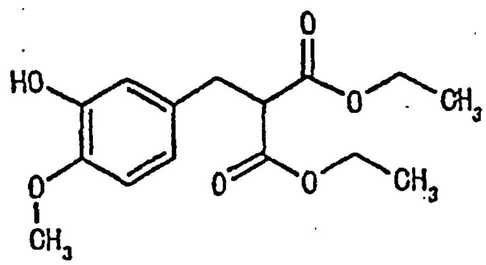

The olefin compound (5.89 g, 20 mmol) synthesized in Reference Example 45 was treated in the same manner as in Reference Example 39 to obtain an intended title compound (5.63 g, 950%) as a colorless transparent syrup. 1H-N.M.R.(CDCl3, 270MHz)d=6.78-6.66(m, 3H), 5.59(s, 1H), 4.14(q, 4H, J=7.3Hz), 3.59(t, 1H, J=8.0Hz), 3.12(d, 2H, J=8.0Hz), 1.22(t, 6H, J=7.3Hz)

[Test Example 1] In-vitro evaluation of PPARα and PPAR γ agonist activities

It was proved by the following experiments that the compounds represented by formulae (1) and (2) according to the present invention have the activity to control PPAR receptor.

Measurement of PPARα agonist activity and PPARγ agonist activity

1. Materials for assay of luciferase using human PPARα and PPARγ receptors

The entire procedure was carried out according to the fundamental methods of genetic engineering and the ordinary method in the yeast one-hybrid or two-hybrid system was applied.

PGL2-UAS5-TK-luc was prepared as a reporter plasmid carrying a response sequence of Gal4 protein as a basic transcription factor of yeast, an enhancer sequence with five repeat part of UAS, a luciferase gene (luc) under the control of timidine kinase (TK) promoter. Specifically, a DNA fragment encoding a TK promoter (-105/+51) on the downstream side of an enhancer sequence comprising two time repeat of UAS was amplified by PCR using pRL-TK carrying a TK promoter (Promega, Trade name: Catalogue No. E2241) as the template and the primers: 5' primer (SEQ ID NO: 1)

5'-GCTAGATCT(CGACGGAGTACTGTCCTCCGAGCT)x2

CGAGGCCCCGCCCAGC GTCTTGTC-3'

3' primer (SEQ ID NO:2)

5'-TTAAGCTTCTGCGGCACGCTGTTGACGCTGTTAAGCGGGTC

GCTGCAGGG-3'.

After the fragment was cleaved with XhoI-Hind III, pGL2-UAS2-TK-luc was obtained by inserting the resultant fragment into the XhoI-Hind III site located on the upstream side of a luciferase structural gene on pGL2-Basic vector (Promega, Trade name: Catalogue No. E1641).

After cleavage of a synthetic DNA as an enhancer sequence comprising three time repeat of Ga14 response sequence (SEQ ID NO: 3): 5'-ATTGGTAC(CGACGGAGTACTGTCCTCCGAGCT)x3 AGATCTCGAC) using KpnI and BglII was carried out, pGL2-UAS5-TK-luc was prepared by inserting the resulting fragment into the KpnI - BglIIb site

of pGL2-UAS2-TK-luc.

A vector for expression of a chimera receptor protein, in which a ligand binding region of an internuclear receptor human PPARα or PPARγ receptor was fused with the carboxyl terminal of a DNA binding region of yeast Ga14 protein, was prepared as follows:

The structural gene of pSG5 (STRATAGENE, Trade Name: Catalogue No. 216201) as the basic expression vector was changed for the chimera receptor except that the promoter-enhancer region was maintained as it was.

The DNA encoding the ligand binding region of the human PPARα or PPARγ receptor was fused on the downstream side of the DNA encoding the DNA binding region and the amino acid sequence from 1 to 147 positions of Ga14 protein so that their reading frames were fitted and the resulting fused DNA was inserted into pSG5 (trade name) on the down stream side of the promoter-enhancer region. In this step, the DNA sequence was arranged so that a signal for transfer into the nucleus (Ala Pro Lys Lys Lys Arg Lys Val Gly (SEQ ID: No:4) derived from SV-40T antigen was located on the amino terminal of the ligand binding region of PPARα or PPARγ receptor in order to localize the chimera receptor in the nucleus.

Based on comparison of the human PPAR receptors described in R. Mukherjee et al., [see J. Steroid Biochem. Molec. Biol., Vol. 51, p157 (1994)] and M. E. Green et al., [see Gene Expression, Vol. 4, p281 (1995))], as the structural gene part used for the ligand binding region PPARα or PPARγ, the human PPARα ligand binding region: Ser167-Tyr468; and the PPARγ ligand binding region: Ser176-Tyr478 were used, which had the same sequences corresponding to Ser204-Tyr506 for human PPARγ1 receptor and human PPARγ2, respectively.

In order to monitor influence against the basic transcription, an expression vector was prepared which carried a DNA encoding only the DNA binding region and the amino acid sequence from 1 to 147 position of Gal4 protein defective in its PPAR ligand binding region and a signal for transfer into the nucleus of SV-40T-antigen.

2. Assay of luciferase using human PPARα or PPARγ receptor

CV-1 cells used as a host cell were cultivated according to an ordinary method. Specifically, a fetal bovine serum (Intergent, Catalogue No. 1020-90) was added to a Dulbecco's modified Eagle's medium (DMEM) so that the final concentration became 10%. Further, penicillin G (50 U/ml) and streptomycin sulfate (50 μg/ml) were added. Cultivation was conducted in the resulting medium in a 5% carbon dioxide gas at 37°C.

On the previous day of transfection, the cells were planted into a 24-well plate in an amount of $1.5x10^5$ cells/well, and transfection was conducted using Lipofect AMINE (trade name, GIBCOBRL, Catalogue No. 26300-61). Specifically, to a serum-free medium, OPti-MEM (trade name, GIBCOBRL, Catalogue No. 31985-070) (40 μl/well) were added a reporter plasmid (100 ng/well), a Gal4-PPAR expression vector (12.5 ng/well), pRL-TK (trade name) (200 ng/well) as an internal control, pGEM-3Zf (+) (trade name, Promega, Catalogue No. p2271) (287.5 ng/well) as a carrier DNA and Lipofect AMINE (trade name, GIBCOBRL, Catalogue No. 26300-61) (2.6 μl/well), followed by thorough mixing. Then, Opti-MEM (trade name) (170.2 μl/well) was added. The resulting mixture was added to the cells washed with PBS (phosphate buffered saline) and Opti-MEM (trade name). Cultivation was conducted at 37°C for 16 hours. Then, the medium was substituted with DMEM-10% active carbon-dextran-treated fetal bovine serum (trade name, HyClone, Catalogue No. SH30068.03) containing a present compound; cultivation was conducted at 37°C for 24 hours to fuse the cells; and luciferase activity was measured according to an ordinary method.

Regarding the PPARα agonist activity, a luciferase activity was taken as 100 which appeared when there was added 10 μM of a positive control compound Wy-14,643 capable of significantly activating the transcription of luciferase gene against PPARα [see Cell, Vol. 83, p813 (1995); J. Biol. Chem., Vol. 270, p12953 (1995); Proc. Natl. Acad. Sci. USA, Vol. 94, p4312 (1997); J. Biol. Chem., Vol. 272, p3406 (1997)]; and a luciferase activity which appeared when there was added a present compound in an amount of 0.1, 1.0 or 10 μM, was shown in Table 1, as a relative activity to the above.

Regarding the PPARγ agonist activity, a luciferase activity was taken as 100 which appeared when there was added 1 μM of a positive control compound Pioglitazone capable of significantly activating the transcription of luciferase gene against PPARγ [see Cell, Vol. 83, p803 (1995) and J. Biol. Chem., Vol. 270, p12953 (1995)]; and a luciferase activity which appeared when there was added a present compound in an amount of 0.1, 1.0 or 10 μM, was shown in Table 2, as a relative activity to the above.

Table 1

| PPARα agonist activity | | | |
|---|---|---|---|
| Compound No. | Relative activity | | |
| | 0.1 μM | 1 μM | 10 μM |
| Example 1 (101) | 0.2 | 0.9 | 28.7 |

Table 1 (continued)

| PPARα agonist activity | | | |
|---|---|---|---|
| Compound No. | Relative activity | | |
| | 0.1 μM | 1 μM | 10 μM |
| Example 2 (61) | NT | 10.4 | 24.6 |
| Example 3 (102) | 0.4 | 0.9 | 8.5 |
| Example 4 (104) | 0.3 | 0.3 | 0.3 |
| Example 5 (71) | 0.3 | 0.5 | 28.4 |
| Example 6 (67) | 5.7 | 47.1 | 74.0 |
| Example 7 (63) | 0.4 | 4.5 | 64.4 |
| Example 8 (65) | 0.3 | 0.3 | 1.5 |
| Example 9 (97) | 0.3 | 0.4 | 23.0 |
| Example 10 (32) | 0.2 | 0.3 | 50.1 |
| Example 11 (99) | 25.2 | 64.3 | 68.6 |
| Example 12 (85) | 0.2 | 0.3 | 6.4 |
| Example 13 (76) | 0.3 | 0.9 | 41.1 |
| Example 14 (86) | 0.3 | 0.3 | 2.1 |
| Example 15 (82) | 0.3 | 0.3 | 5.0 |
| Example 16 (32) | 0.3 | 0.4 | 45.2 |
| Example 18 (112) | 0.3 | 0.3 | 0.3 |
| Example 19 (108) | 0.3 | 0.3 | 0.6 |
| NT = Not tested. | | | |

Table 2

| PPARγ agonist activity | | | |
|---|---|---|---|
| Compound No. | Relative activity | | |
| | 0.1 μM | 1 μM | 10 μM |
| Example 1 (101) | 98.6 | 127.1 | 102.3 |
| Example 2 (61) | 85.0 | 114.2 | NT |
| Example 3 (102) | 74.1 | 114.5 | 108.3 |
| Example 4 (104) | 0.4 | 1.1 | 14.9 |
| Example 5 (71) | 0.3 | 0.4 | 11.6 |
| Example 6 (67) | 122.1 | 140.3 | 124.9 |
| Example 7 (63) | 13.5 | 63.3 | 114.1 |
| Example 8 (65) | 1.3 | 13.0 | 107.2 |
| Example 9 (97) | 0.6 | 16.8 | 110.1 |
| Example 10 (32) | 1.0 | 20.8 | 123.9 |
| Example 11 (99) | 75.2 | 114.7 | 105.6 |
| Example 12 (85) | 1.2 | 60.4 | 111.4 |
| Example 13 (76) | 103.9 | 139.6 | 130.5 |

Table 2 (continued)

| PPARγ agonist activity | | | |
|---|---|---|---|
| Compound No. | Relative activity | | |
| | 0.1 μM | 1 μM | 10 μM |
| Example 14 (86) | 19.6 | 76.0 | 132.3 |
| Example 15 (82) | 52.3 | 132.8 | 124.6 |
| Example 16 (32) | 1.6 | 71.8 | 127.4 |
| Example 18 (112) | 0.5 | 22.2 | 89.9 |
| Example 19 (108) | 25.2 | 83.5 | 97.9 |
| Example 20 (114) | 9.3 | 91.0 | 109.5 |
| Example 21 (118) | 1.4 | 1.9 | 22.4 |
| Example 22 (120) | 3.5 | 62.3 | 107.9 |
| Example 23 (137) | 2.8 | 30.5 | 63.1 |
| Example 24 (141) | 0:8 | 1.2 | 9.4 |
| Example 25 (144) | 0.7 | 1.9 | 6.2 |
| Example 26 (147) | 0.1 | 3.7 | 12.8 |
| Example 27 (128) | 1.2 | 2.1 | 42.5 |
| Example 28 (129) | 1.3 | 2.1 | 14.7 |

[Test Example 2] Test for in-vitro evaluation of the compound's action of removing the hTNFα's inhibition for sugar intake induced by insulin stimulus

It was proven by the following experiment that the compound represented by the formula (1) according to the present invention has an action of removing the hTNFα's inhibition for glucose intake induced by insulin stimulus.

The present compound's action of removing the hTNF α's inhibition for glucose intake induced by insulin stimulus in 3T3-L1 lipocytes was investigated by measurement of glucose concentration in medium.

Specifically, mouse 3T3-L1 fibroblasts (produced by Dainippon Pharmaceutical Co., Ltd.) were suspended in a Dulbecco's modified Eagle's medium (DMEM) containing 10% of a bovine serum and planted in a 24-well collagen plate; and cultivation was conducted until the cells became confluent. Cultivation was continued for a further 2 days (the day of cultivation completion was taken as the 0 (zero) day of differentiation induction). Then, the medium was substituted with a differentiation induction medium (a DMEM containing 10% of a fetal bovine serum, 0.5 mM of 3-isobutyl-1-methyl-xanthine, 0.25 μM of dexamethasone and 1 μg/ml of insulin), followed by cultivation for 40 hours. On the second day of differentiation induction, the medium was substituted with a DMEM containing 10% of a fetal bovine serum and 1 μg/ml of insulin and cultivation was conducted. On the 4th day of differentiation induction, the medium was substituted with a DMEM containing 10% of a fetal bovine serum and 50 ng/ml of insulin and cultivation was conducted. On the 7th day of differentiation induction when the cells differentiated sufficiently into lipocytes, a compound of the present invention was added to a DMEM containing 10% of a fetal bovine serum, 50 ng/ml of insulin and 5 ng/ml of hTNFα and cultivation was conducted therein. The present compound was beforehand dissolved in DMSO and then added to the medium so that the final concentration of DMSO became 0.1%. On the 9th day of differentiation induction, the medium was substituted with a medium containing the present compound, having the same composition as in the 7th day of differentiation induction. In the above cultivations, 50 U/ml of penicillin and 50 μg/ml of streptomycin sulfate were added to each medium in order to avoid contamination and each cultivation was conducted in a 5% carbon dioxide gas at 37°C.

On the 11th day of differentiation induction, the medium was substituted with a DMEM containing 2% of a bovine serum albumin (BSA), in order to remove the influence of serum, and cultivation was conducted for 4 hours. The medium was removed and the cells were washed with a Krebs-Ringer buffer (1.2 mM $KH_2PO_4$, 4.7 mM KCl, 118 mM NaCl, 25 mM $NaHCO_3$, 2.5 mM $CaCl_2$, pH 7.4) containing 0.1% of the BSA and 180 mg/l of glucose. Then, to each well was added 300 μl of Krebs-Ringer buffer containing 0.1% of the BSA, 180 mg/l of glucose and 0.5 ng/ml of insulin, and cultivation was conducted in a 5% carbon dioxide gas at 37°C for 3 hours.

The glucose concentration in supernatant liquid of medium, which is an indication of sugar intake, was measured by using Glucose CII Test Wako (produced by Wako Pure Chemicals Industries, Ltd.).

The activity (the action of removing the hTNFα's inhibition of sugar intake) of the present compound (10 μM) was shown as a relative value when the activity of a positive control compound (Pioglitazone 10 μM) was taken as 100. The results are shown in Table 3.

Table 3

| Compound's removal of hTNFα's inhibition for glucose metabolism | |
|---|---|
| Compound No. | Relative activity |
| Example 1 (101) | NT |
| Example 2 (61) | 83 |
| Example 3 (102) | NT |
| Example 4 (104) | NT |
| Example 5 (71) | 67 |
| Example 6 (67) | 68 |
| Example 7 (63) | 93 |
| Example 8 (65) | 91 |
| Example 9 (97) | NT |
| Example 10 (32) | NT |
| Example 11 (99) | NT |
| Example 12 (85) | NT |
| Example 13 (76) | 81 |
| Example 14 (86) | 69 |
| Example 15 (82) | 82 |
| Example 16 (32) | 70 |
| Example 17 (106) | NT |
| Example 18 (112) | 107 |
| Example 19 (108) | 77 |
| Example 20 (114) | 85 |
| Example 21 (118) | 51 |
| Example 22 (120) | 89 |
| Example 23 (137) | 16 |
| Example 24 (141) | 48 |
| Example 25 (144) | 97 |
| Example 26 (147) | 85 |
| Example 27 (128) | 74 |
| Example 28 (129) | 23 |
| NT = Not tested | |

[Test Example 3] Test for in-vitro evaluation of the action of inhibiting the production of tumor necrosis factor α (TNFα) using lipocytes (derived from 3T3-L1)

It was proved by the following experiment that a compound represented by the formula (1) according to the present invention has an action for inhibiting the production of TNFα.

A luciferase gene was bonded to the downstream side of human TNF transcription-controlling region . The

resulting material was stably inserted into the chromosomes of 3T3-L1 cells. The thus-obtained cell strain No. 46 was used for evaluation.

The cell strain No. 46 was cultivated according to an ordinary method. Specifically, a bovine serum (Cell Culture Technology, Catalogue No. CC017-502) was added to a Dulbecco's modified Eagle's medium (DMEM) so that the final concentration became 10%. Further, 50 U/ml of penicillin G, 50 ug/ml of streptomycin sulfate and 0.4 mg/ml of G418 were added. In the resulting medium was conducted cultivation in a 5% carbon dioxide gas at 37°C.

The cell strain No. 46 was planted into a PLL (poly L-lysine)-coated 48-well plate. When the cell strain reached confluence and then at least two days passed, the medium was substituted with the above medium containing 9 ng/m of hTNF (Genzyme) and an activity for TNFα transcription was induced. hTNF was added and, simultaneously therewith, a compound of the present invention was added so that the final concentration became 10 μM. 24 hours later, the cells were observed by a microscope. Then, the cells were fused and the luciferase activity was measured according to an ordinary method.

Regarding the activity for TNFα transcription, the luciferase activity when DMSO was added in place of the compound, was taken as 100; and the relative activity when 10 uM of the present compound was added, was shown in Table 1. Incidentally, the relative luciferase activity when no hTNF was added, was 40%. The results of evaluation are shown in Table 4.

Table 4

| Activity for TNFα transcription | |
|---|---|
| Compound No. | Relative activity |
| | 10 μM |
| Example 3 (102) | 71.7 |
| Example 4 (104) | 64.0 |
| Example 24 (141) | 71.6 |
| Example 26 (147) | 59.0 |

[Test Example 4] Test for in-vivo evaluation of the activities for blood sugar reduction and lipid reduction using diabetes model mice (KKAy mice)

Type 2 diabetes mice "KK-Ay/Ta Jci" (male, Clea Japan, Inc., 10-week old) put in cages were used (each cage contained 5 mice). In the morning of the 1st day of test start, a blood was collected from the orbital vein of each mouse. The blood sugar value in the blood was measured by subjecting the blood to deproteinization using perchloric acid, subjecting the resulting material to centrifugation, and subjecting the resulting supernatant liquid to a new blood sugar test (Boehringer Mannheim). Further, the blood was subjected to centrifugation to obtain a plasma and the triglyceride concentration and free fatty acid concentration in the plasma were measured using Triglyceride E-Test Wako and NEFA-C Test Wako (Wako Pure Chemical Industries, Ltd.), respectively. Grouping was made so that the blood sugar values in individual groups became the same. Then, an Example 2 compound (exemplary compound No. 61) was suspended in a 0.5% aqueous CMC solution and administered orally one time per day for 4 days. In the 5th day of test, blood collection was made to measure blood sugar value, triglyceride concentration and free fatty acid concentration. Incidentally, a group to which only the 0.5% aqueous CMC solution was administered, was used as a control group; and Pioglitazone was used in a positive control group. The reduction in parameter in each group was calculated using the following formula. The results are shown in Table 5.

Reduction (%) =

{1 - (parameter of 5th day in each group)/

(parameter of 1st day in each group)} x 100

Table 5

| Results of in-vivo evaluation of reduction in blood sugar and reduction in lipid, using KKAy mice | | | | |
|---|---|---|---|---|
| Compound group | Dose (mg/kg) | Reduction in blood sugar (%) | Reduction in triglyceride (%) | Reduction in free fatty acid (%) |
| Control group | - | 9 | -8 | 13 |
| Example 2 | 30 | 50 | 57 | 46 |
| Pioglita-zone | 30 | 28 | 60 | 49 |

Industrial Applicability

The compound of the present invention is a novel substance and, as shown in Examples and Test Examples, allows PPARα or γ (which is an intranuclear transcription factor) to function strongly. Further, the present compound is low in toxicity and is therefore expected to be used as a preventive or therapeutic agent against various diseases related to PPARα or γ.

## Claims

**1.** A quinoline derivative represented by the following formula (1) or a pharmacologically acceptable salt thereof:

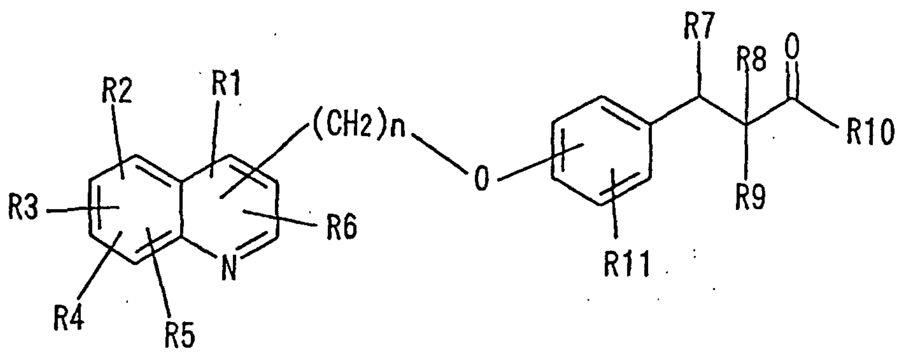

(1)

(wherein R1, R2, R3, R4, R5, R6 and R11 are each independently a hydrogen atom, a lower alkyl group of 1 to 4 carbon atoms, a halogen atom, a hydroxyl group, an alkyloxy group of 1 to 10 carbon atoms, a nitro group, an optionally substituted phenyl group, an amino group which may be substituted with a lower alkyl group of 1 to 4 carbon atoms, a cyano group, a carboxyl group, a lower alkyloxycarbonyl group of 1 to 4 carbon atoms, an aminocarbonyl group which may be substituted with a lower alkyl group of 1 to 4 carbon atoms, or a trifluoromethyl group; R7 and R8 are a hydrogen atom or a lower alkyl group of 1 to 4 carbon atoms and may directly bond to each other to form a carbon-to-carbon double bond; R9 is a hydroxyl group, an alkyloxy group of 1 to 10 carbon atoms, an optionally substituted phenyloxy group, a thiol group, an alkylthiol group of 1 to 10 carbon atoms, an optionally substituted phenylthiol group, a lower alkylcarbonyl group of 1 to 4 carbon atoms, an optionally substituted benzoyl group, a lower alkyloxycarbonyl group of 1 to 4 carbon atoms, an aminocarbonyl group which may be substituted with a lower alkyl group of 1 to 4 carbon atoms, or a carboxyl group; R10 is a hydroxyl group, a lower alkyl group of 1 to 4 carbon atoms, a lower alkyloxy group of 1 to 4 carbon atoms, an optionally substituted phenyloxy group or an amino group which may be substituted with a lower alkyl group of 1 to 4 carbon atoms; and n is an integer of 1 to 4).

**2.** A quinoline derivative represented by the following formula (2) or a pharmacologically acceptable salt thereof:

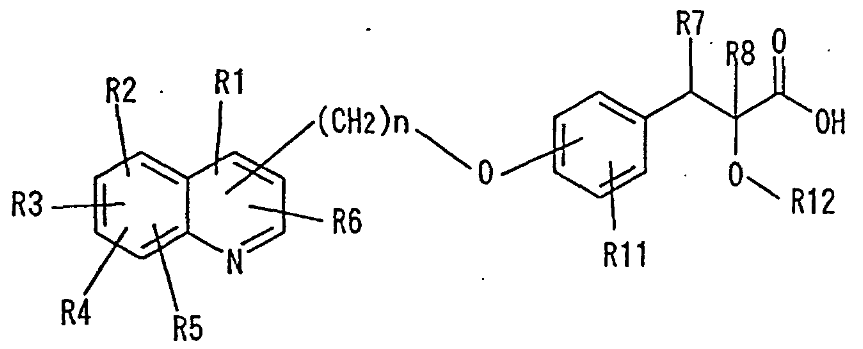

(2)

(wherein R1, R2, R3, R4, R5, R6, R7, R8 and R11 have the same definitions as in Claim 1; and R12 is a lower alkyl group of 1 to 4 carbon atoms or an optionally substituted phenyl group).

**3.** A quinoline derivative represented by the following formula (3) or a pharmacologically acceptable salt thereof:

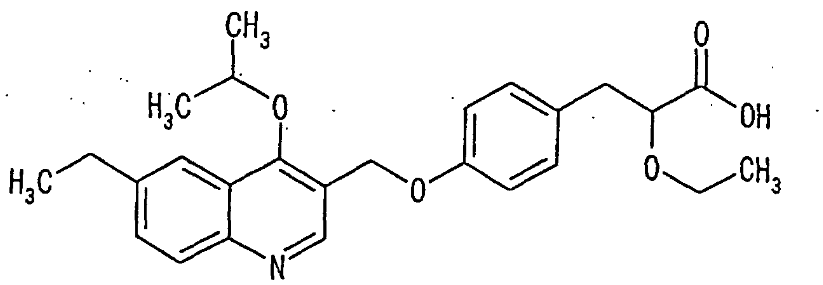

(3)

**4.** An antagonist for peroxisome proliferator-activated receptor α or γ (which is an intranuclear transcription factor), comprising a quinoline derivative set forth in Claim 1, 2 or 3 as an active ingredient.

**5.** An inhibitor for production of tumor necrosis factor α, containing, as an active ingredient, a quinoline derivative set forth in Claim 1, 2 or 3.

**6.** A preventive or therapeutic agent for diabetes comprising a quinoline derivative set forth in Claim 1, 2 or 3 as an active ingredient.

**7.** A preventive or therapeutic agent for hyperlipemia comprising a quinoline derivative set forth in Claim 1, 2 or 3 as an active ingredient.

**8.** A preventive or therapeutic agent for arterioscrelosis comprising a quinoline derivative set forth in Claim 1, 2 or 3 as an active ingredient.

## INTERNATIONAL SEARCH REPORT

International application No.
PCT/JP01/02168

**A. CLASSIFICATION OF SUBJECT MATTER**
Int.Cl$^7$ C07D215/22, 215/14, A61K31/47, A61P43/00, 3/10, 3/06, 9/10

According to International Patent Classification (IPC) or to both national classification and IPC

**B. FIELDS SEARCHED**
Minimum documentation searched (classification system followed by classification symbols)
Int.Cl$^7$ C07D215/22, 215/14, A61K31/47, A61P43/00, 3/10, 3/06, 9/10

Documentation searched other than minimum documentation to the extent that such documents are included in the fields searched

Electronic data base consulted during the international search (name of data base and, where practicable, search terms used)
REGISTRY(STN), CA(STN), CAOLD(STN), CAPLUS(STN)

**C. DOCUMENTS CONSIDERED TO BE RELEVANT**

| Category* | Citation of document, with indication, where appropriate, of the relevant passages | Relevant to claim No. |
|---|---|---|
| X | WO, 99/11255, A1 (ONO PHARMACEUTICAL CO., LTD.), 11 March, 1999 (11.03.99), Full text & AU, 9887502, A | 1-8 |
| X | WO, 99/20275, A1 (RHONE-POULENC RORER PHARMACEUTICALS INC.), 29 April, 1999 (29.04.99), Full text & EP, 1030665, A1 & AU, 9896961, A & BR, 9814087, A & NO, 2000001962, A | 1-8 |
| PX | WO, 00/64888, A1 (AVENTIS PHARMACEUTICALS PRODUCTS INC.), 02 November, 2000 (02.11.00), Full text (Family: none) | 1-8 |
| PX | WO, 00/64876, A1 (AVENTIS PHARMACEUTICALS PRODUCTS INC.), 02 November, 2000 (02.11.00), Full text (Family: none) | 1-8 |

☒ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 June, 2001 (04.06.01) | 19 June, 2001 (19.06.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/02168

C (Continuation). DOCUMENTS CONSIDERED TO BE RELEVANT

| Category* | Citation of document, with indication, where appropriate, of the relevant passages | Relevant to claim No. |
|---|---|---|
| A | COLLINS, Jon L. et al., "N-(2-Benzoylphenyl)-L-tyrosine PPAR γ Agonists. 2. Structure-Activity Relationship and Optimization of the Phenyl Alkyl Ether Moiety", J. Med. Chem. (1998), Vol.41, No.25, pp.5037-5054 | 1-8 |

Form PCT/ISA/210 (continuation of second sheet) (July 1992)